# EUROPEAN PATENT APPLICATION

(11) **EP 1 302 550 A1**
(43) Date of publication of application: **16.04.2003**
(21) Application number: 01123379.8
(22) Date of filing: 10.10.2001
(51) Int. Cl.: C12Q 1/70

(54) **Method and detector for identifying subtypes of human papilloma viruses**

(71) Applicant: King Car Food Industrial Co., Ltd., Taipei (TW)
(72) Inventor: Lin, Ching-Yu, Ilan City, Ilan (TW); Lin, Ruey-Wen, Sanshia Jen, Taipei (TW); You, Chiou-Mien, Ilan City, Ilan (TW); Huang, Hsing-Hsuan, Luodung jen, Ilan (TW); Lee, Bor-Heng, Chiai (TW); Lee, Hsien-Hsiung, Danshuei Jen, Taipei (TW); Lin, Yu-Ju, Ilan City, Ilan (TW); Fan, Chih-Chun, Toufen Jen, Miaoli (TW); Hsu, Han-Chuan, Luodung Jen, Ilan (TW); Shih, Chia-Wen, Ilan City, Ilan (TW); Yeh, Chih-Hsing, Dounan Jen, Yunlin (TW); Kao, Yi-Feng, Sanchung City, Taipei (TW); Pan, Chih-Long, Ilan City, Ilan (TW); Chan, Peter, Taipei (TW)
(74) Representative: Reinhard - Skuhra - Weise & Partner

(57) **Abstract**

A detector (10, 20, 30) for simultaneously detecting and identifying subtypes of human papilloma viruses (HPV) contained in a sample is provided. The detector (10, 20, 30) includes a carrier (11, 21, 31) having a first part and a second part for carrying the sample thereon, a first oligonucleotide carried on the first part of the carrier, and a second oligonucleotide carried on the second part of the carrier, characterized in that the first and second oligonucleotides respectively hybridized with deoxyribonucleic acids contained in a first subtype of human papilloma virus and a second subtype of human papilloma virus for simultaneously detecting and identifying subtypes of human papilloma viruses.

## Description

The present invention relates to a method and a detector for detecting human papilloma viruses, and more particularly to a method and a detector for simultaneously detecting and identifying subtype of human papilloma viruses.

Cervical cancer is the most common cancer in women. The consorts are often men with penile warts. Sexual activity appears to be an important predisposing factor of the epidemic disease and precancerous lesions. In early 5 to 10 years during the development of cervical cancer, cervical cells form cervical intraepithelial neoplasm.

Recently, in order to decrease the incidence of cervical cancer, Pap smear is used for the cervical cancer screening. However, the Pap smear has a false negative rate of about 30%∼40%. In addition, it is known that more that 95% of cervical carcinoma tissue contain detectable DNA sequences for known varieties of the human papilloma virus (HPV). Hence, the combination of Pap smear and HPV detection for the cervical cancer screening is considered.

The applicant cooperates with the hospital to did the epidemiolory research in women cervical cancer by using Pap smear and HPV detection, wherein the HPV detection is proceeded by using polymerase chain reaction and nucleotide sequencing. There are 2424 women aged from 16 to 84 for the epidemiology research, wherein 1963 women provide the effective specimen. The research results are shown as follows.
1) 1.9% (37/1963) of the women have abnormal cytological smears.
2) 12.7% (244/1926) of the women with normal cytological smears but have HPV infection.
3) The HPV prevalence in the women with abnormal cytological smears is 51.4% (19/37) and positively relative to the degree of the abnormal cytological smears, wherein the incidence of abnormal non-typical squamous cells is 23.1%, the incidence of low abnormal epithelial cells is 41.7%, and the incidence of high abnormal epithelial cells is 75%.
4) The subtypes of human papilloma viruses detected in the specimens are HPV 52, HPV 58, HPV 70, HPV 16, HPV 18, HPV 68, HPV 33, HPV 66, HPV 35, HPV 37, HPV 54, HPV 59, HPV 67, HPV 72, HPV 69, HPV 82, HPV 39, HPV 31, HPV 32, HPV HLT7474-S, HPV 6, HPV CP8061, HPV 62, HPV CP8304, HPV 44, HPV 11, HPV 61, HPV 74, HPV 42 and HPV 43.

The conventional HPV detecting kits are only used for detecting 18 subtypes of human papilloma viruses including high risk HPV 16, HPV 18, HPV 31, HPV 33, HPV 35, HPV 39, HPV 45, HPV 51, HPV 52, HPV 56, HPV 58, HPV 59 and HPV 68, and detecting low risk HPV 6, HPV 11, HPV 42, HPV 43 and HPV 44.

According to the comparison of the epidemiology research and the conventional HPV detecting kits, various subtypes of human papilloma viruses contained in a specimen would not be identified by the conventional HPV detecting kits. In addition, the conventional HPV detecting kits are only used for detecting the high risk HPV and the low risk HPV, but not for identifying the HPV subtypes. Furthermore, the conventional HPV detecting kits lack the system control for checking the house-keep genes contained in a specimen.

In order to overcome the foresaid drawbacks of the conventional HPV detecting kits, the present invention provides a method and a detector for simultaneously detecting and identifying subtypes of human papilloma viruses contained in a sample.

It is therefore an object of the present invention to provide a detector for simultaneously detecting and identifying subtypes of human papilloma viruses (HPV) contained in a sample.

In accordance with the present invention, the detector includes a carrier having a first part and a second part for carrying the sample thereon, a first oligonucleotide carried on said first part of the carrier, and a second oligonucleotide carried on the second part of the carrier, wherein the first and second oligonucleotides respectively hybridized with deoxyribonucleic acids contained in a first subtype of human papilloma virus and a second subtype of human papilloma virus for simultaneously detecting and identifying subtypes of human papilloma viruses.

Preferably, the carrier is made of nylon.

Preferably, the carrier is a glass plate.
Preferably, the first and second subtypes of human papilloma viruses are respectively selected from 38 subtypes of human papilloma viruses, wherein the sequence of the first oligonucleotide is selected from one of the sequence group corresponding to the first subtype of human papilloma virus and complementary sequences thereof, and the sequence of the second oligonucleotide is selected from one of the sequence group corresponding to the second subtype of human papilloma virus and complementary sequences thereof.
Preferably, the detector could be an oligonucleotide chip.

In another aspect of the present invention to provide a method for simultaneously detecting and identifying subtypes of human papilloma viruses contained in a sample.

In accordance with the present invention, the method includes steps of providing a first oligonucleotide and a second oligonucleotide for respectively hybridizing with a first and a second subtypes of human papilloma viruses, hybridizing deoxyribonucleic acid (DNA) contained in the sample with the first and second oligonucleotides, and removing nonhybridized DNA, thereby the subtypes of human papilloma viruses contained in the sample are detected and identified.

Preferably, the DNA contained in the sample is the product of polymerase chain reaction (PCR).

Preferably, the DNA contained in the sample has signaling substances labeled thereon.

Preferably, the signaling substance is biotin.

In addition, the method further includes a step of performing a reaction of biotin and avidin-alkalinephosphatase.

On the other hand, the signaling substances could be fluorescent substances. Preferably, the fluorescent substance is Cyanine 5.

It is another aspect of the present invention to provide a method for detecting a subtype of human papilloma viruses contained in a sample.

In accordance with present invention, the method includes steps of providing an oligonucleotide complementary to a sequence specific to the subtype of human papilloma viruses, hybridizing said oligonucleotide with deoxyribonucleic acid (DNA) contained in the sample, removing non-hybridized DNA contained in the sample, and detecting hybridized DNA to show whether the subtype of human papilloma viruses contained in the sample.

The present invention may best be understood through the following descriptions with reference to the accompanying drawings, in which:
Fig. 1 is a schematic view showing the detector according to the first preferred embodiment of the present invention;
Fig. 2 is a schematic view showing the detector according to the second preferred embodiment of the present invention;
Fig. 3(a) is a schematic view showing the detector according to the third preferred embodiment of the present invention;
Fig. 3(b) is a schematic view illustrating the subtype of human papilloma viruses identified by each dot shown in Fig. 3(a);
Fig. 4(a) is the electrophoresis result showing the analyzed products of the first polymerase chain reaction;
Fig. 4(b) is the electrophoresis result showing the analyzed products of the second polymerase chain reaction;
Fig. 4(c) is detecting result on the detectors of detecting the HPV positive clones according to the third preferred embodiment of the present invention;
Fig. 5 is a view showing the detecting result on the detectors of detecting samples according to the third preferred embodiment of the present invention;
Fig. 6(a) is a schematic view showing the detector according to the fourth preferred embodiment of the present invention;
Fig. 6(b) is a schematic view illustrating the subtype of human papilloma viruses identified by each dot shown in Fig. 6(a);
Fig. 7(a) is a view showing the detector stained with SYBR Green II according to the fourth embodiment of the present invention; and
Fig. 7(b) is a view showing the detecting result on the detectors of detecting samples according to the fourth preferred embodiment of the present invention.

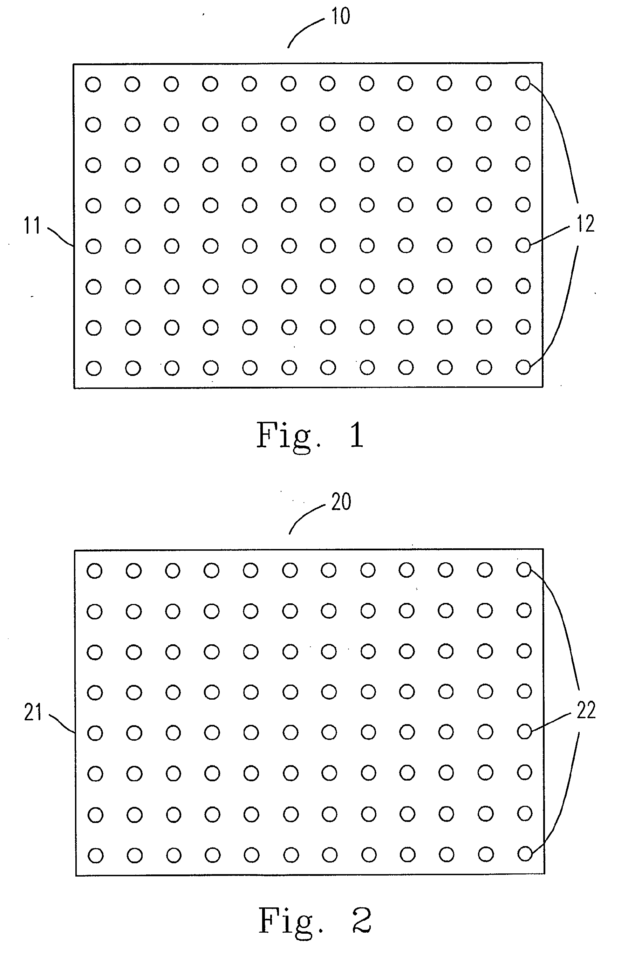

Please refer to Fig. 1. A detector 10 is the first embodiment of the present invention for simultaneously detecting and identifying the subtypes of human papilloma viruses contained in a sample. The detector 10 includes a carrier 11 and a dot array. The carrier 11 is a nylon membrane having the dot array 12 mounted thereon. Each dot in the dot array 12 is an oligonucleotide (15∼30mer) for identifying a specific subtype of human papilloma viruses.

The sequences of the oligonucleotides provided by the present invention are specific to the epidemics of human papilloma viruses. The sequences of the oligonucleotides shown in Tables 1 to 38 are determined by the way of comparing DNA sequences of 97 subtypes of human papilloma viruses. Each table illustrates a plurality of oligonucleotides for identifying a specific subtype of human papilloma viruses.

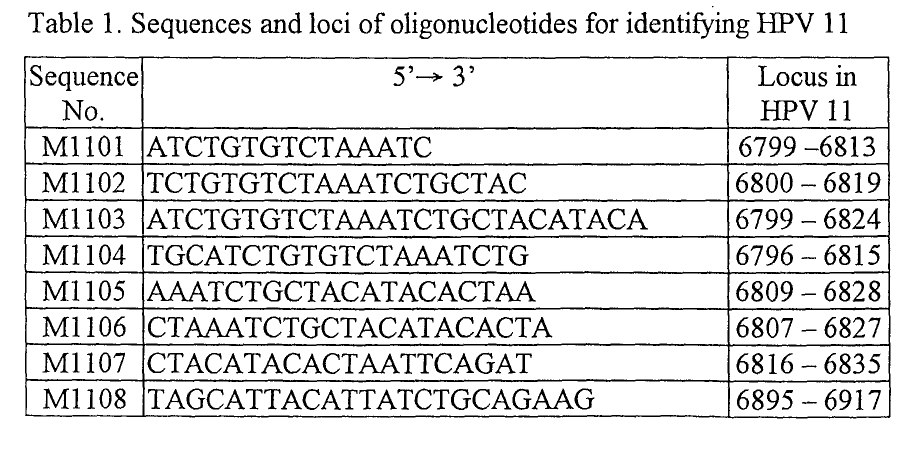

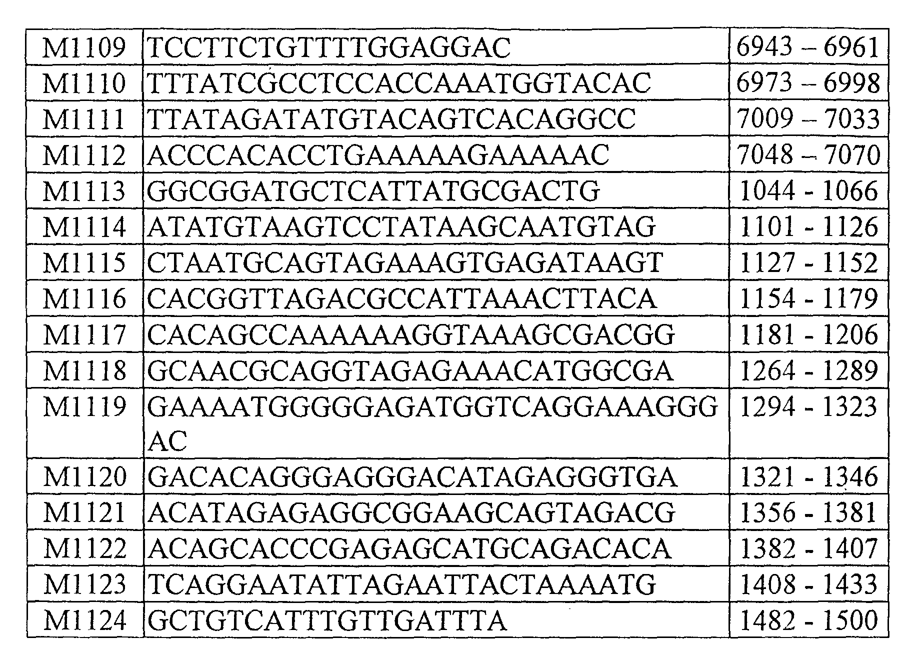

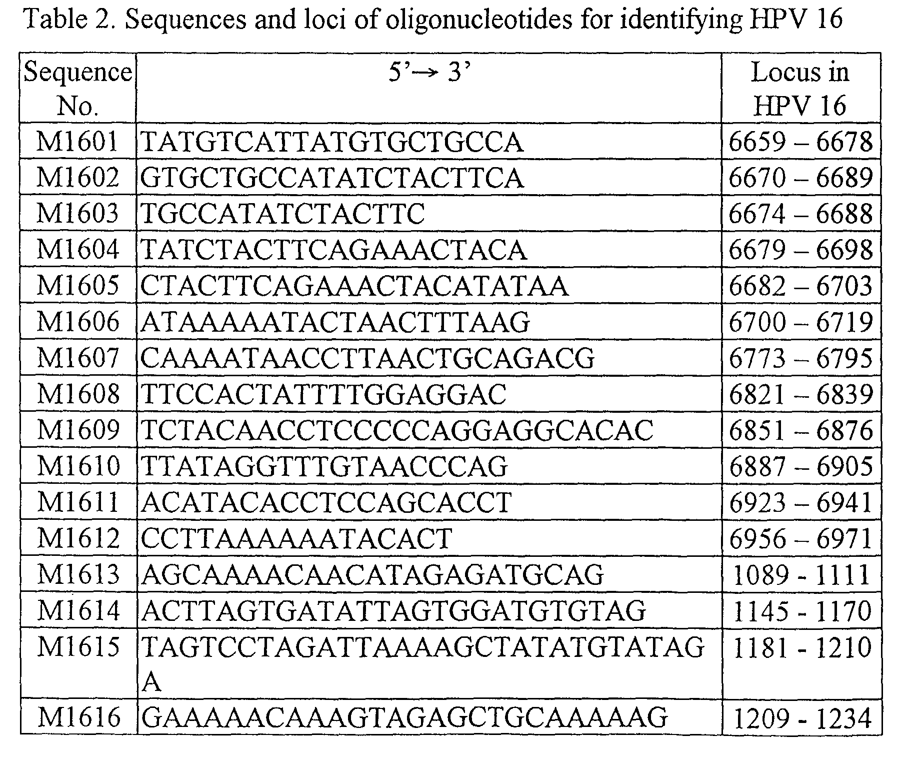

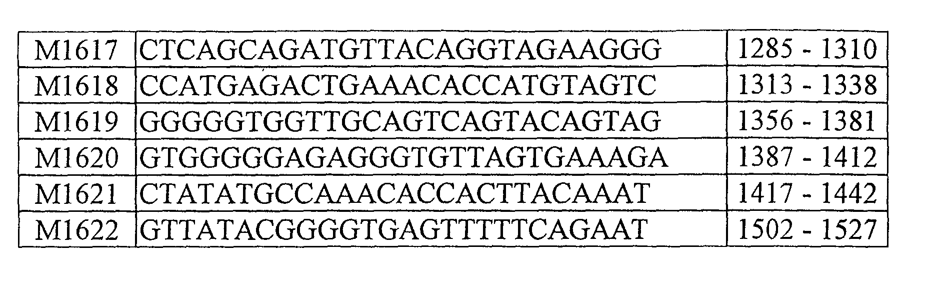

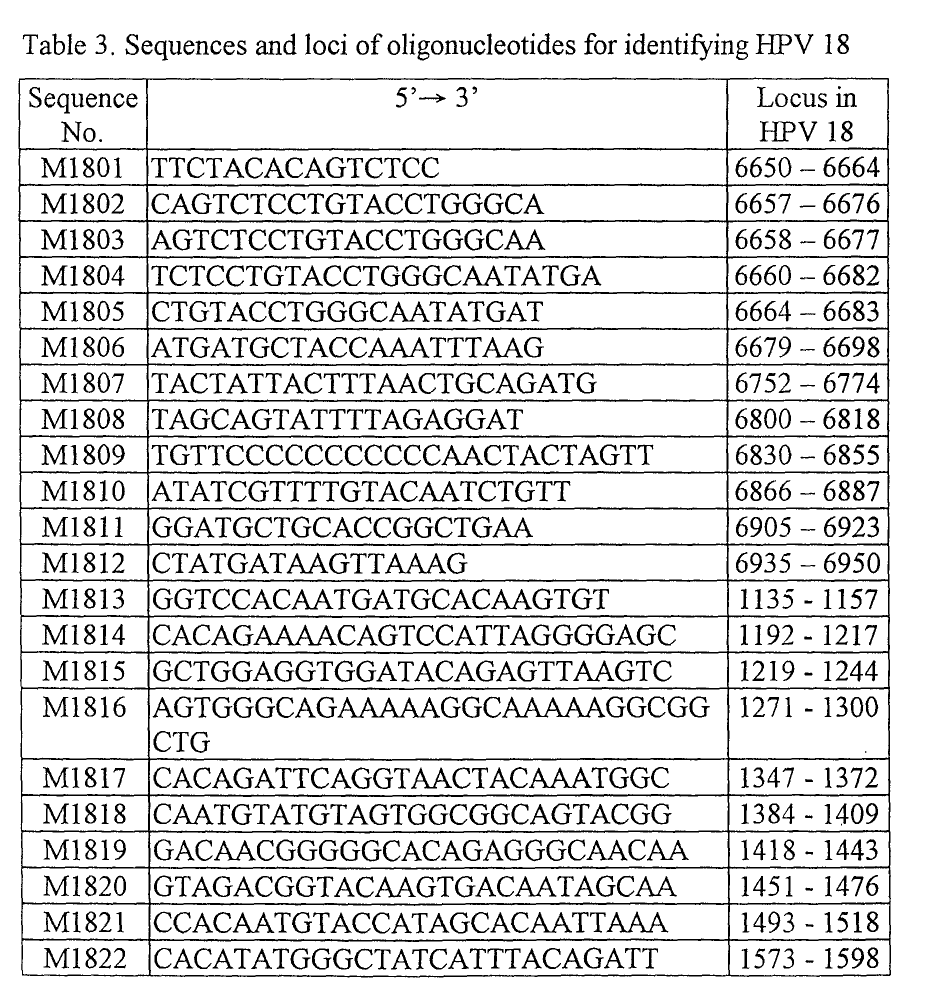

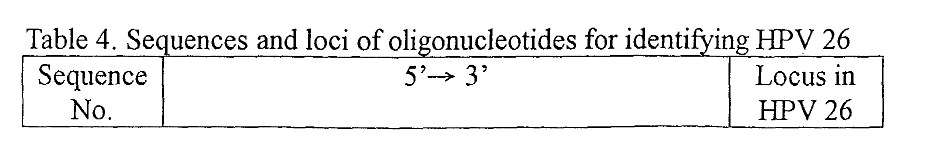

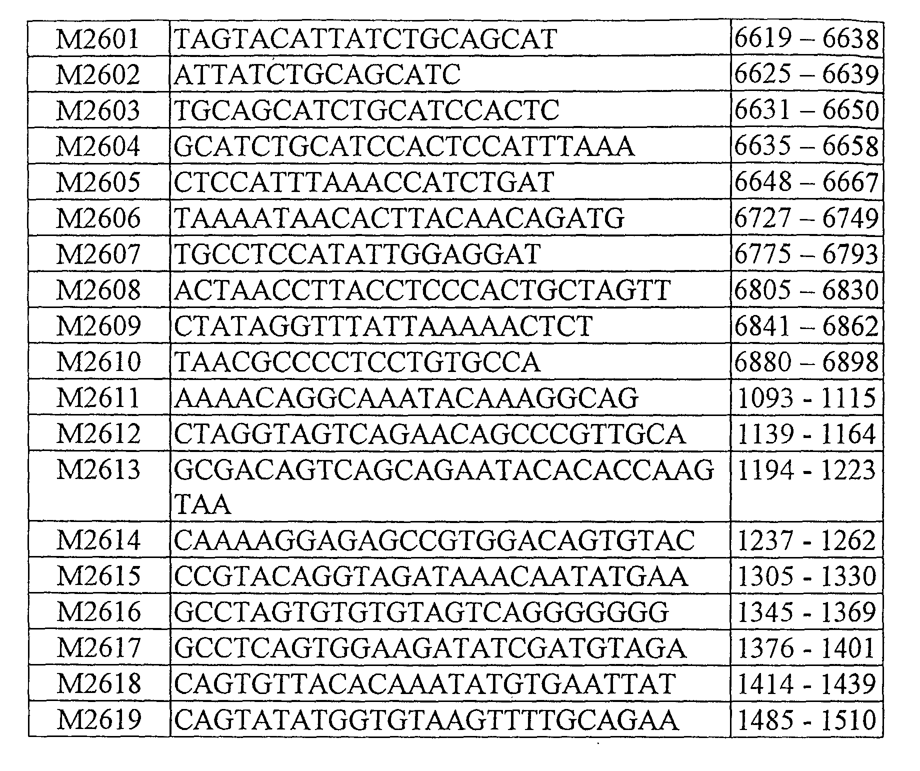

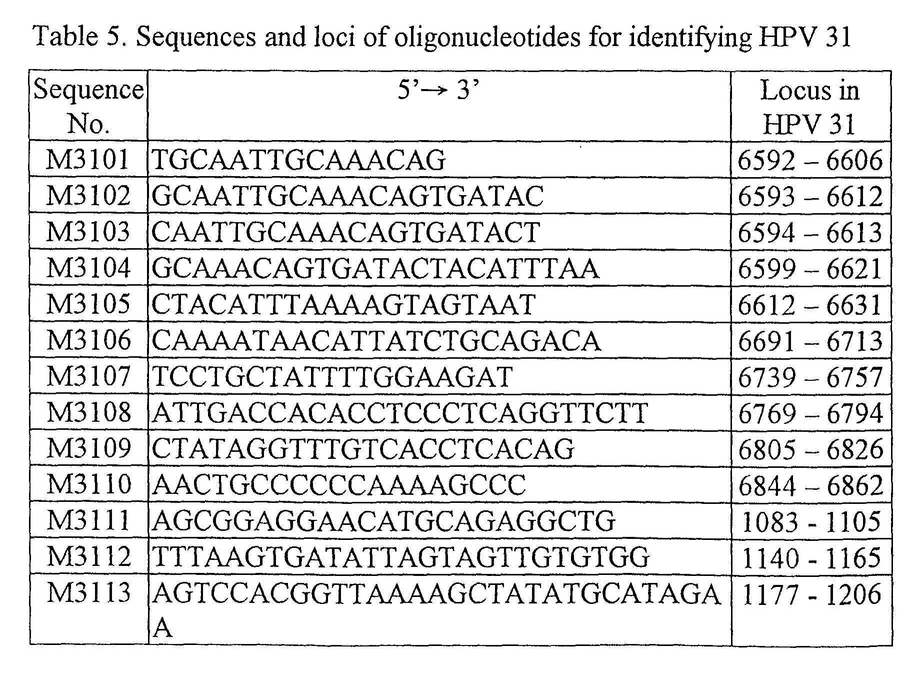

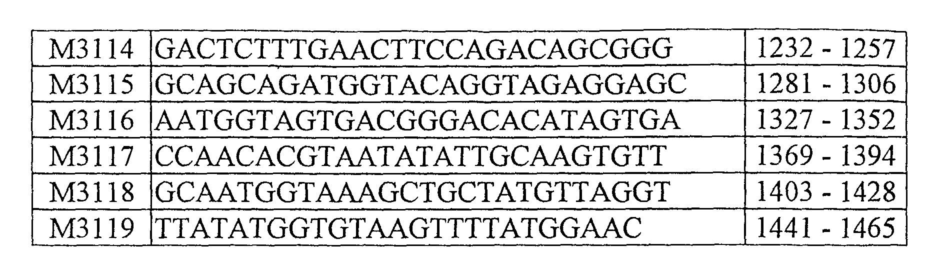

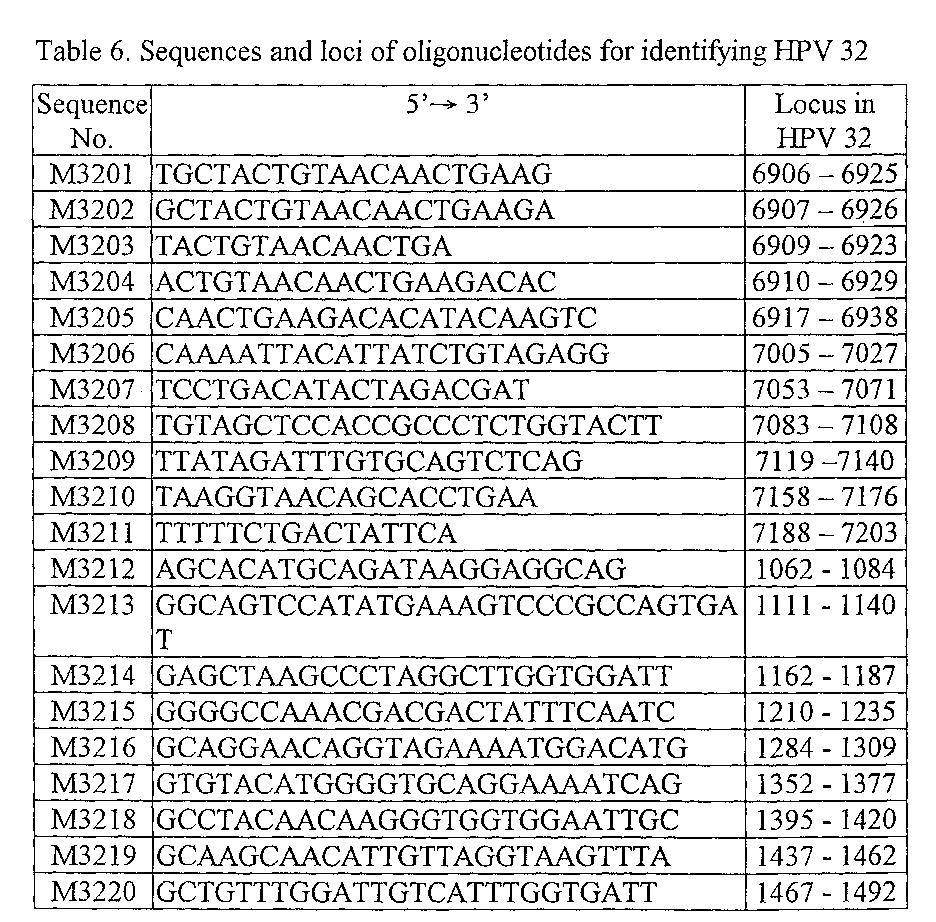

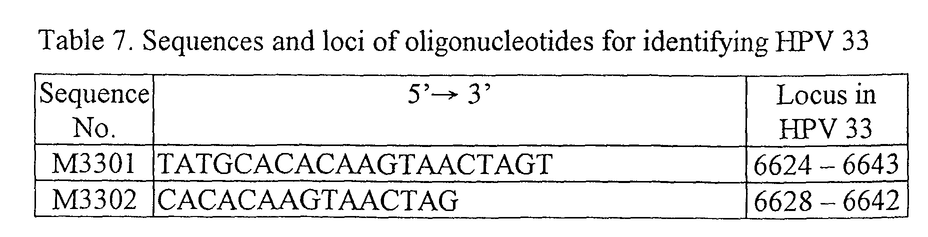

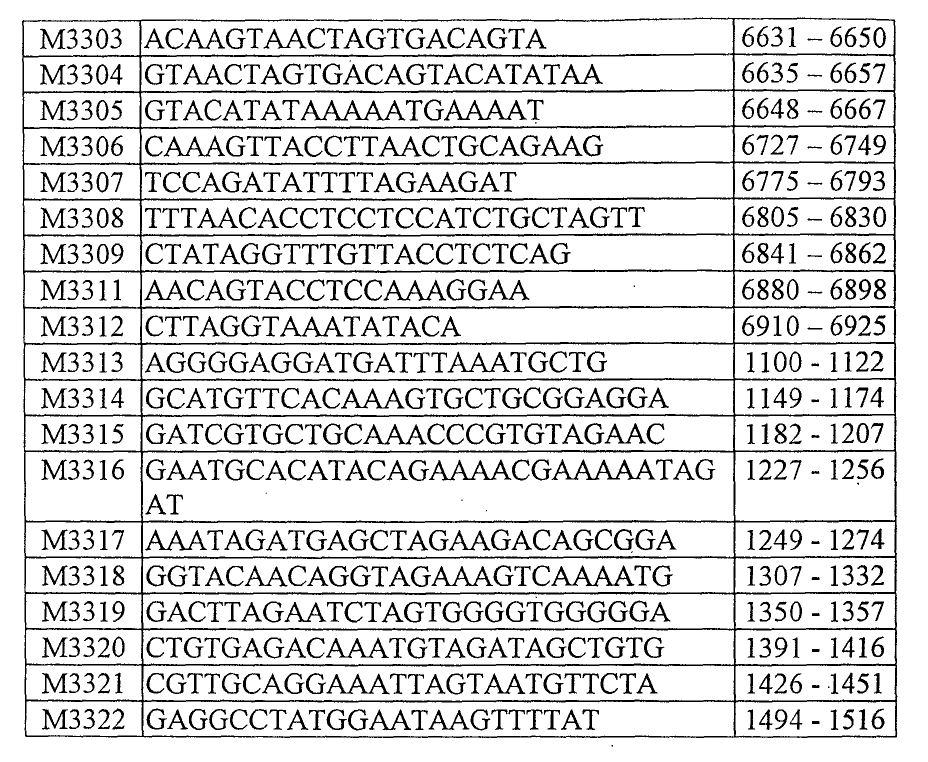

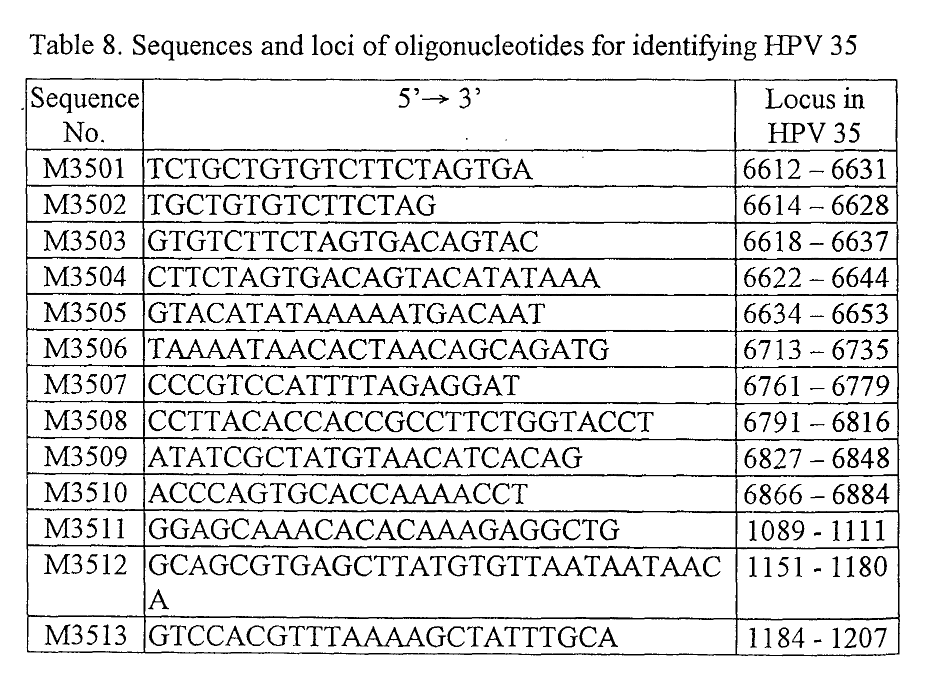

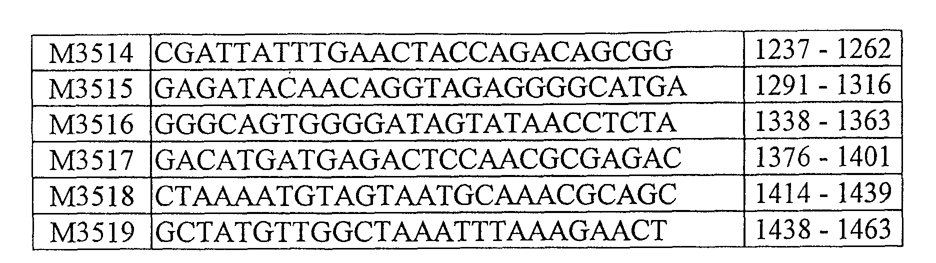

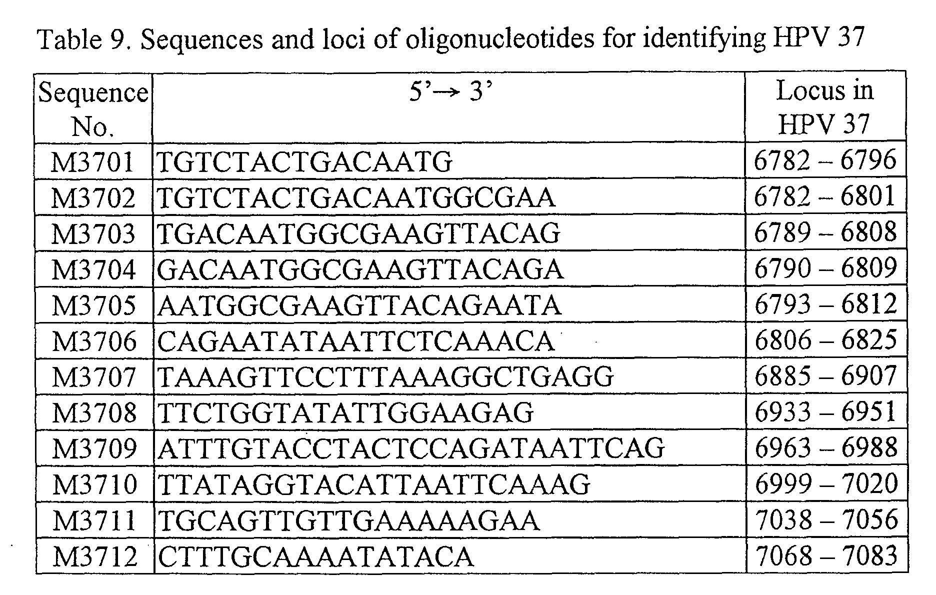

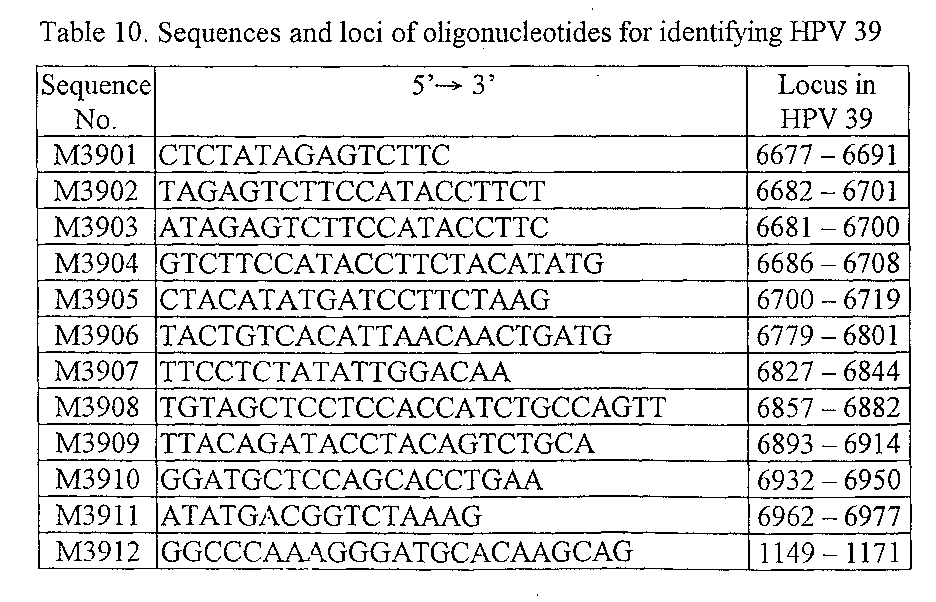

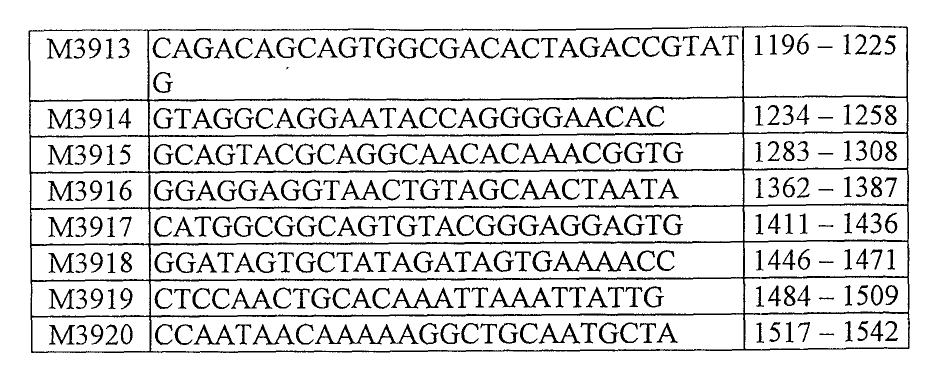

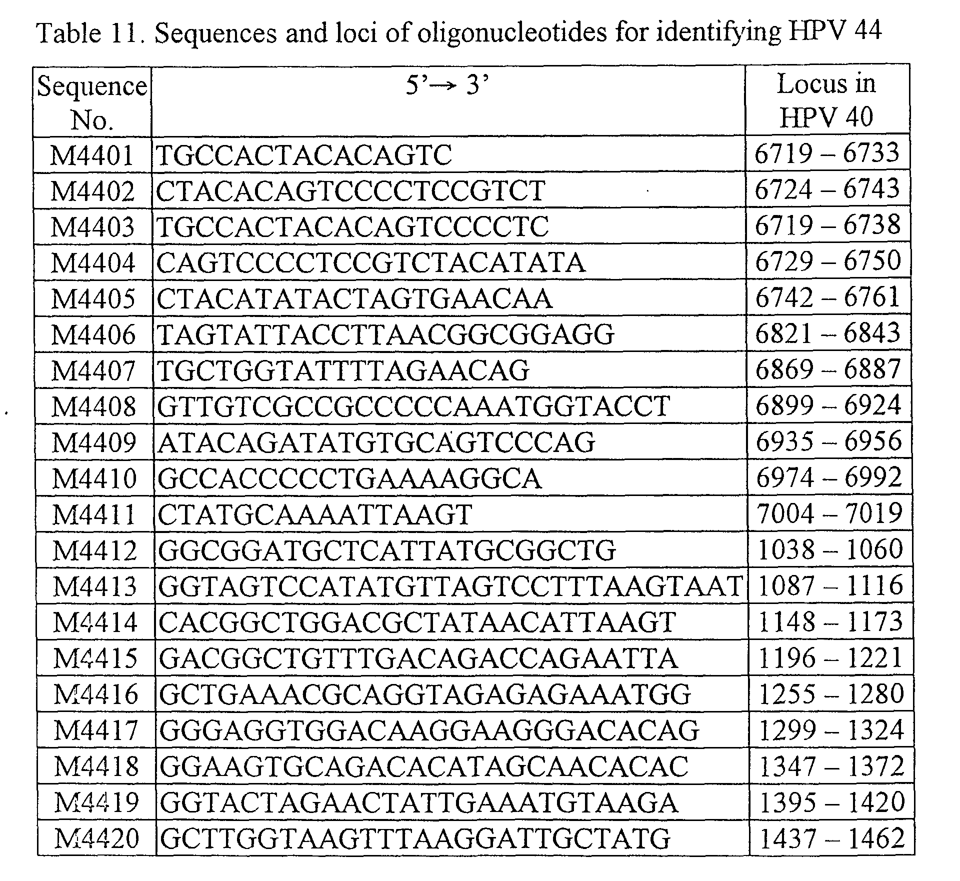

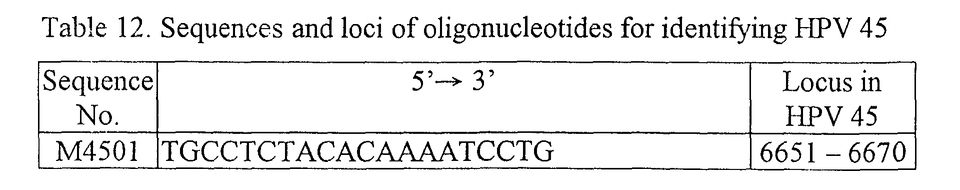

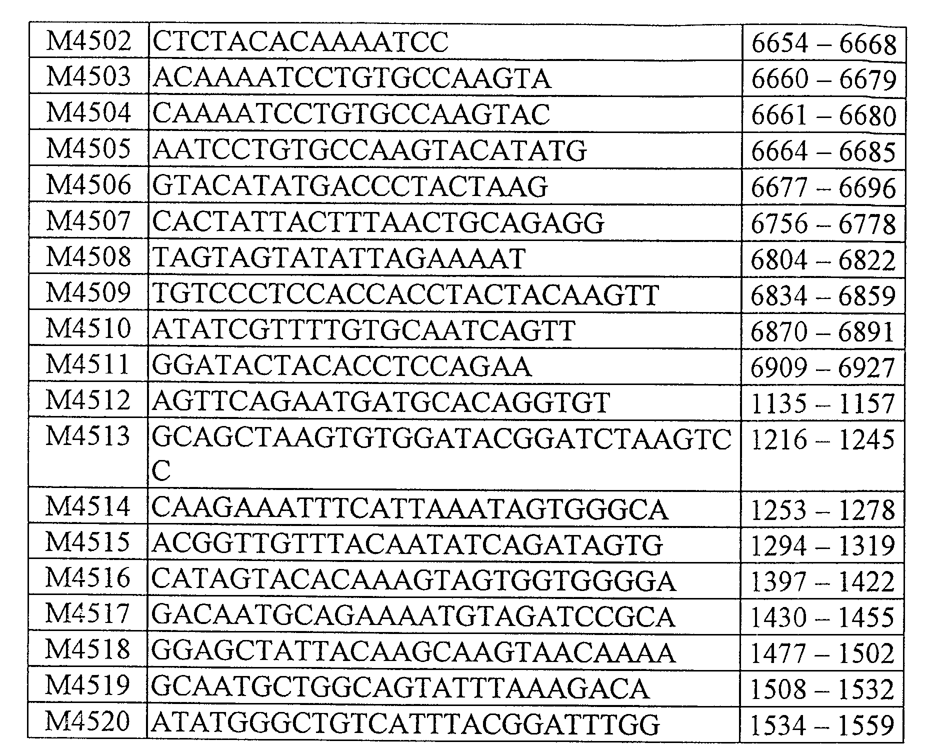

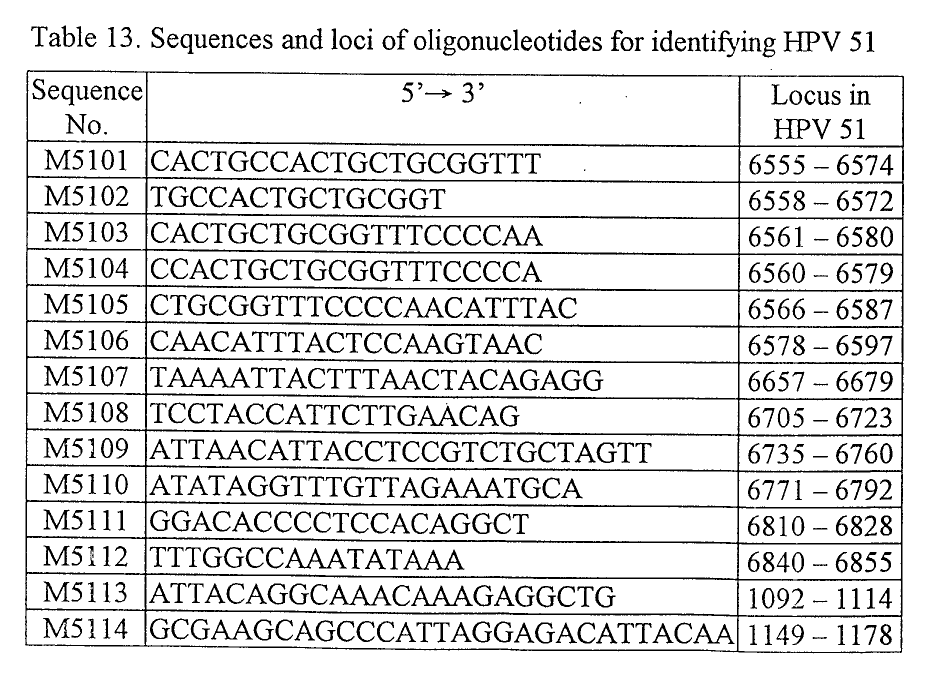

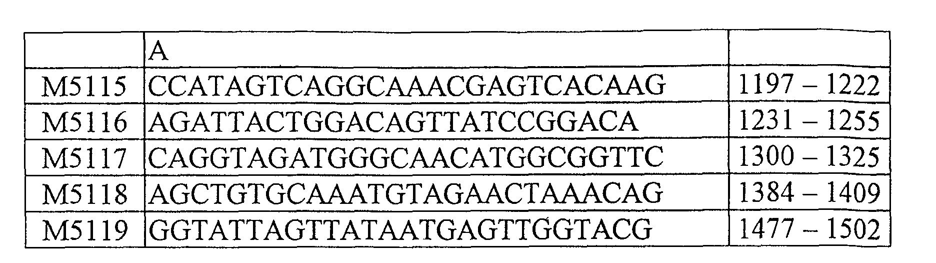

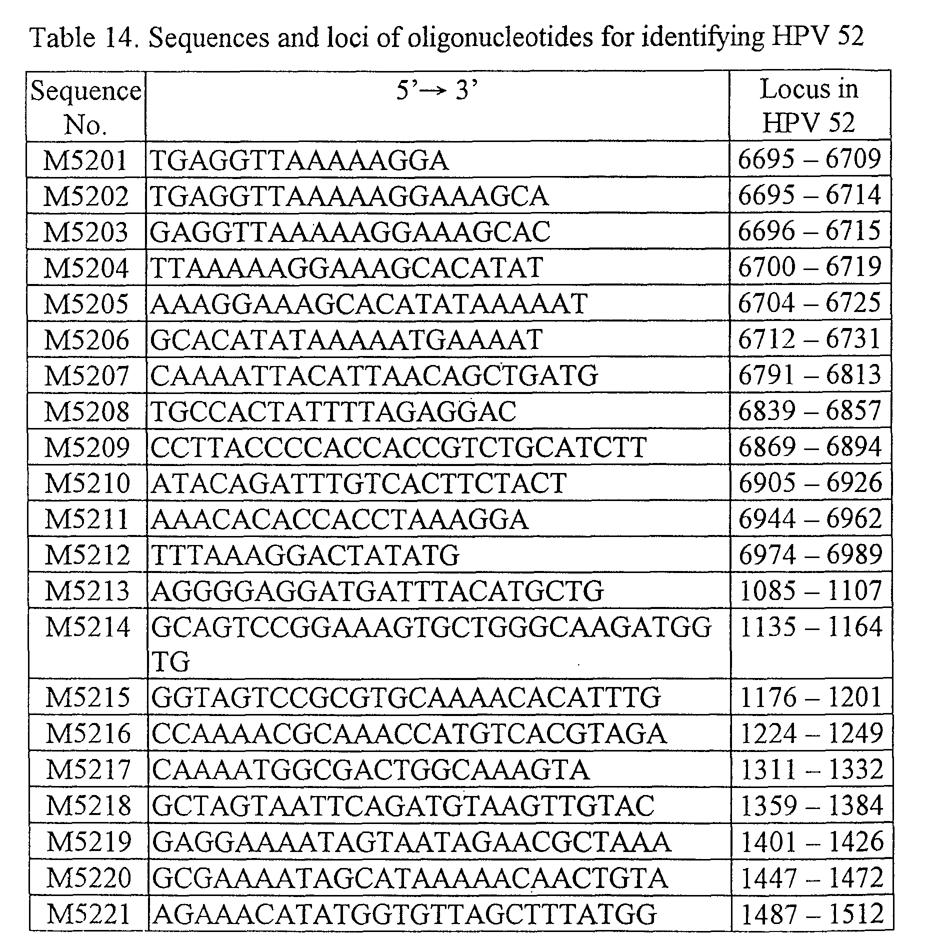

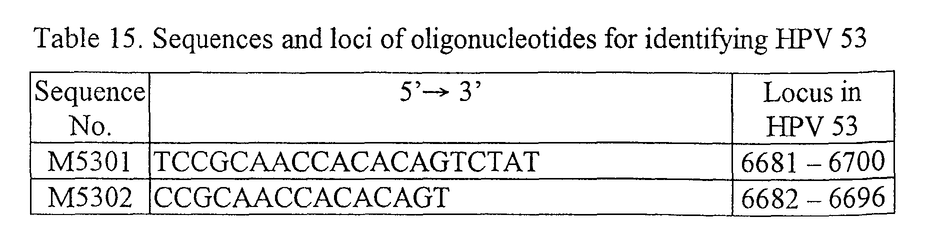

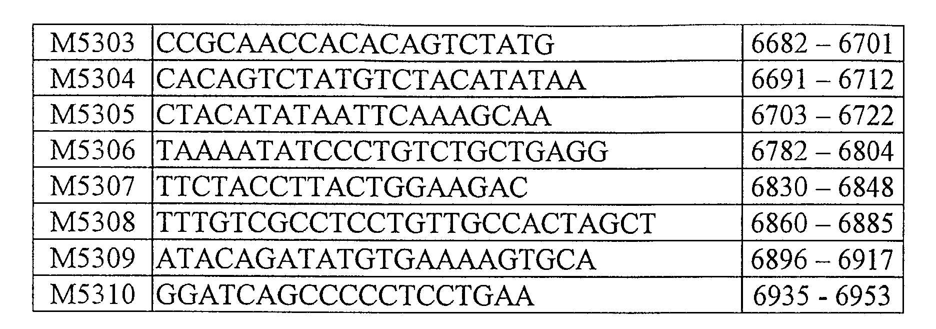

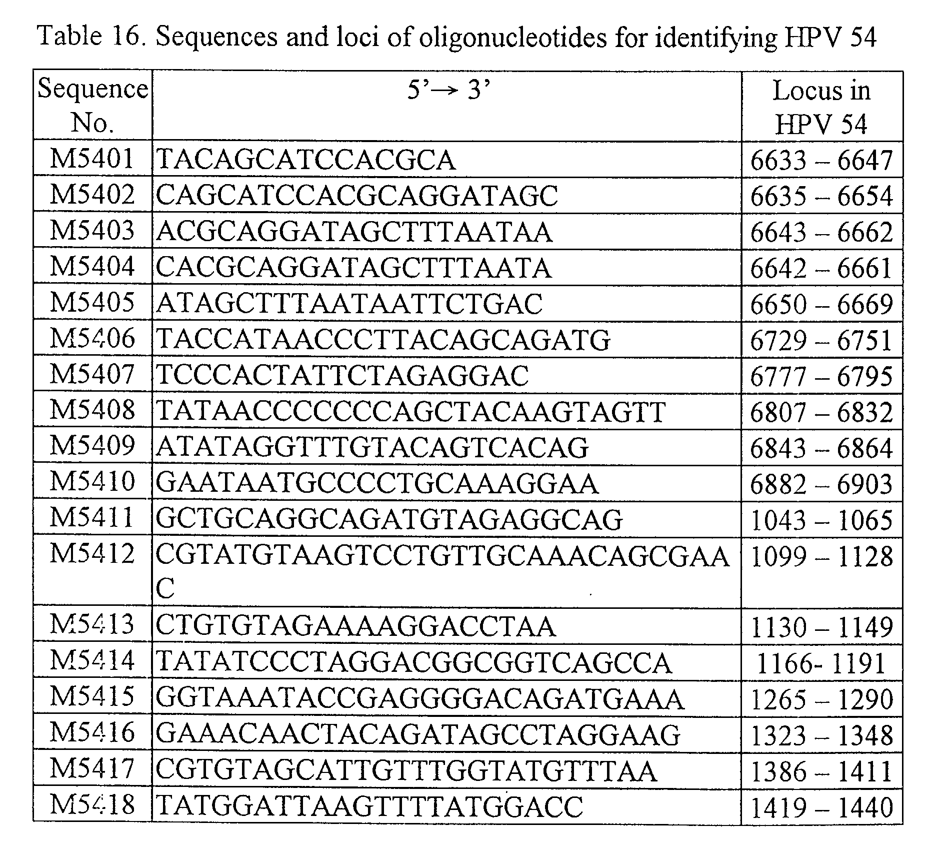

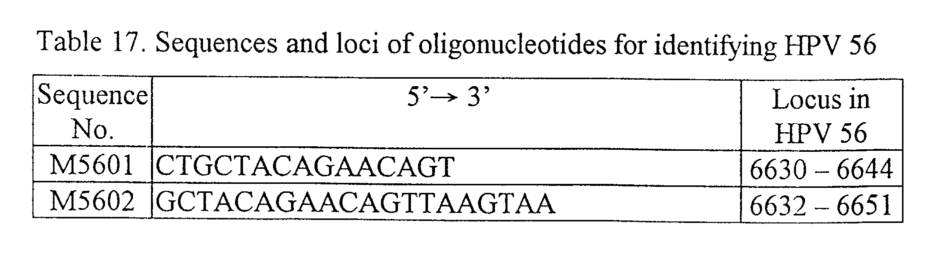

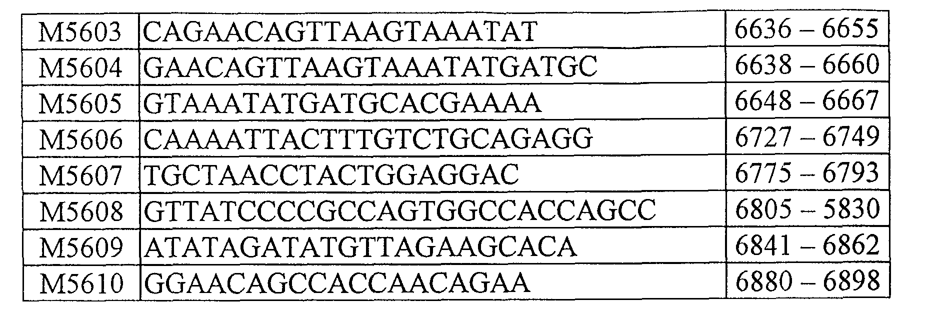

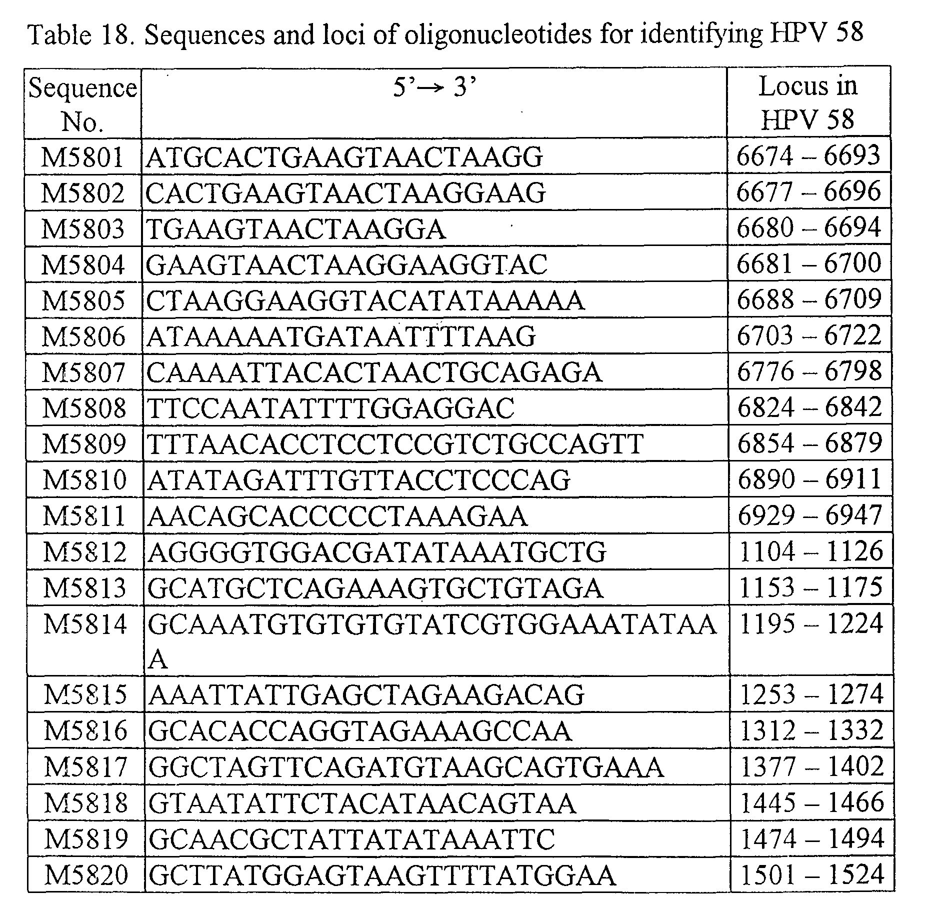

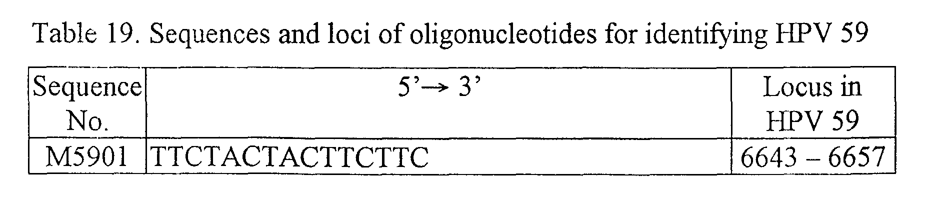

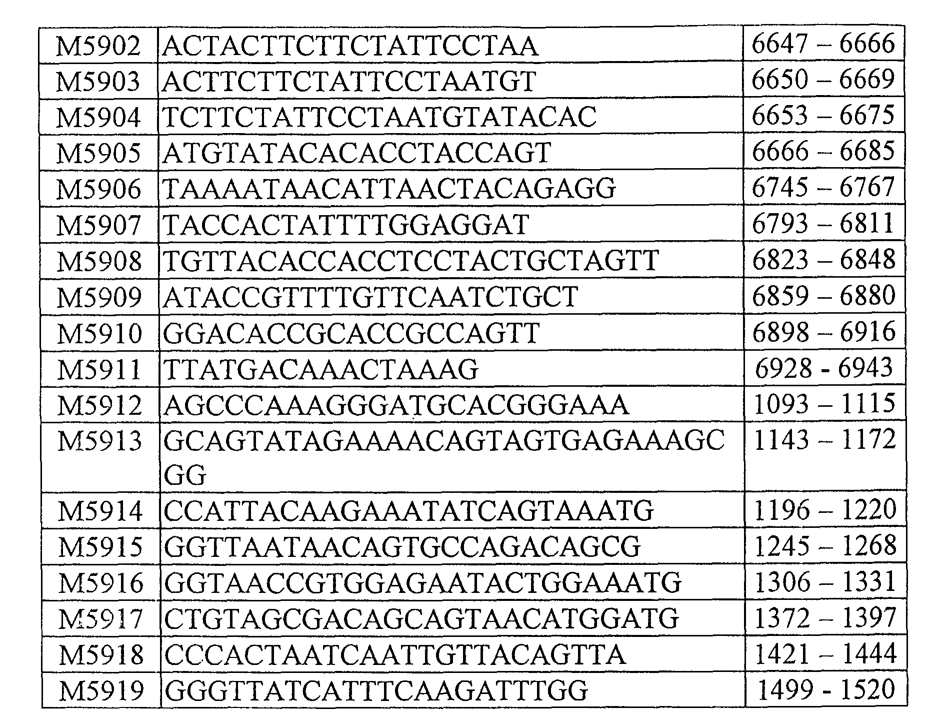

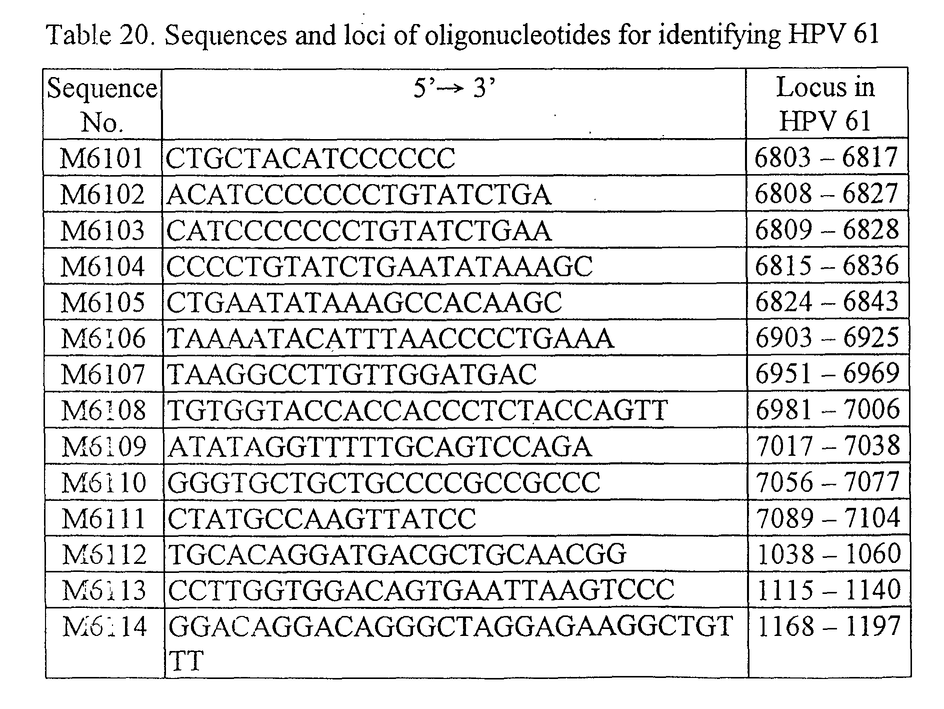

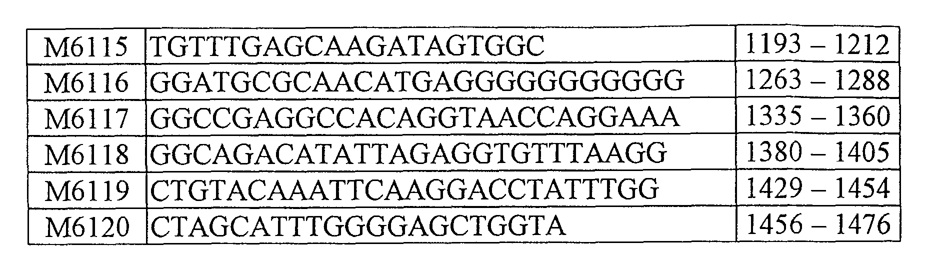

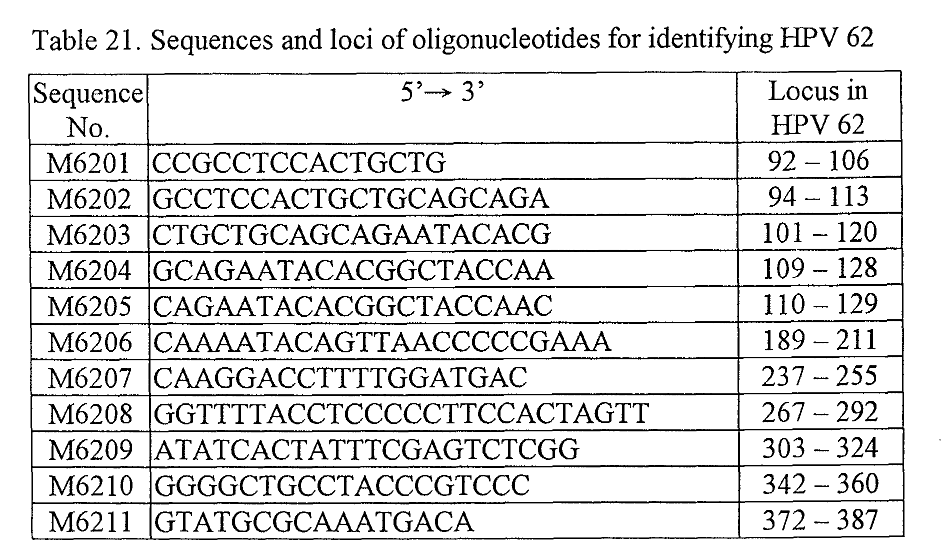

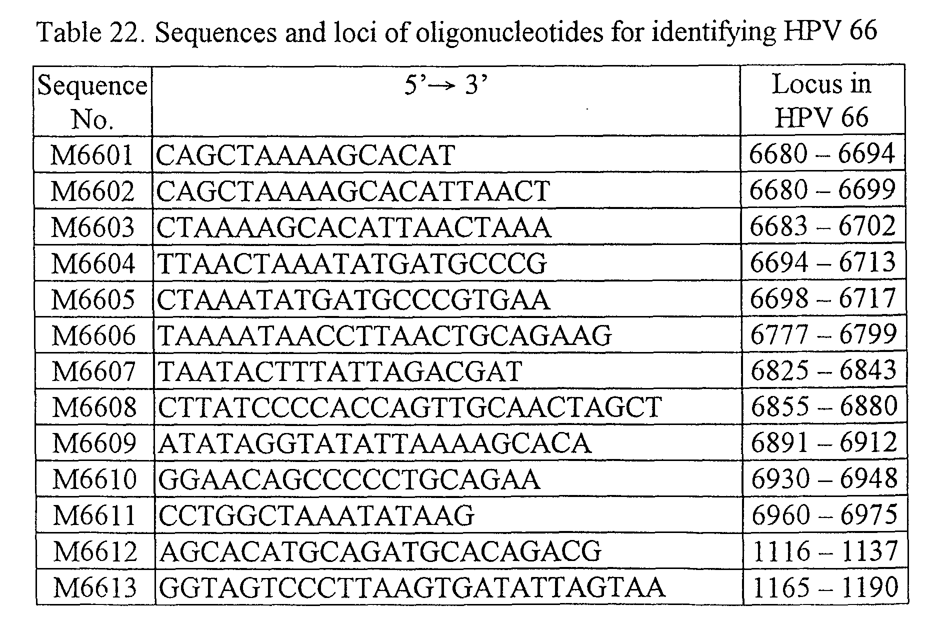

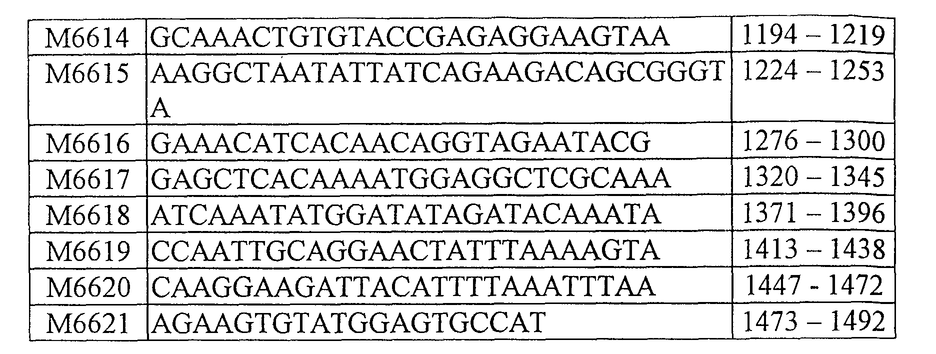

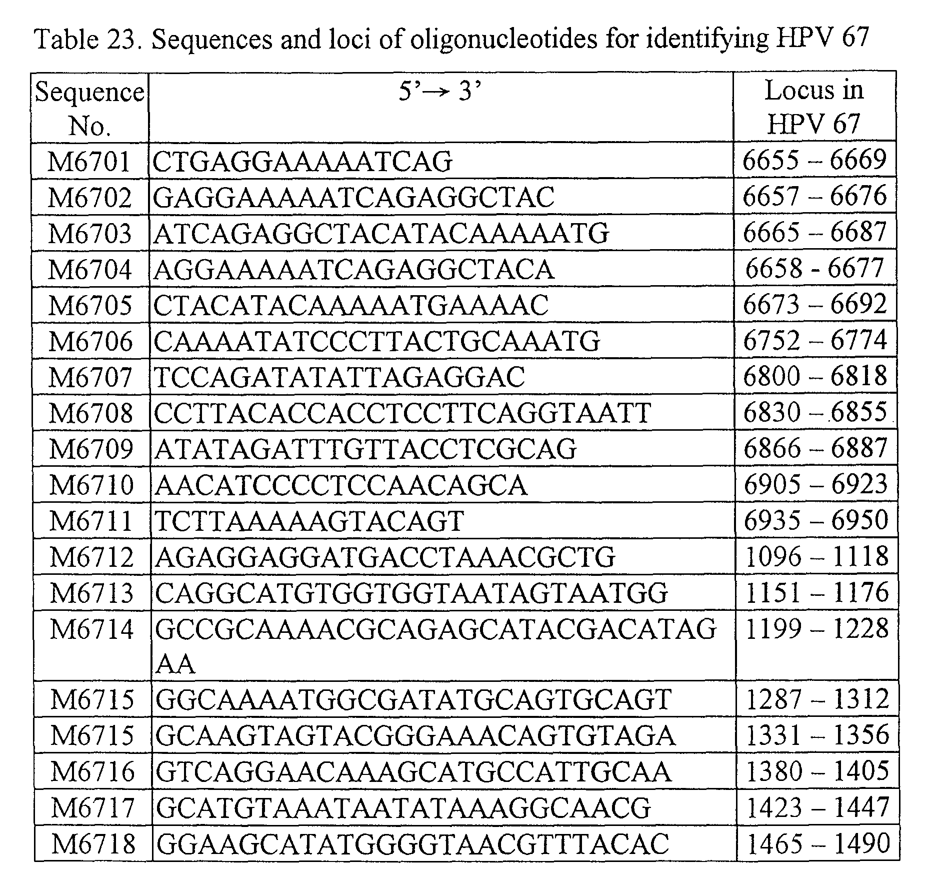

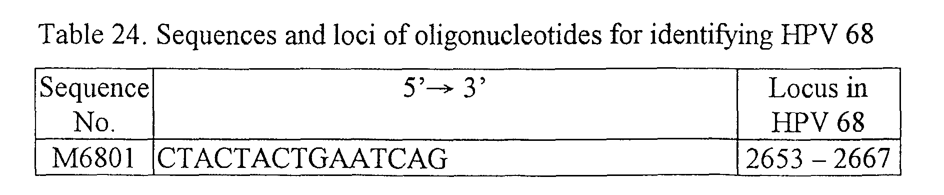

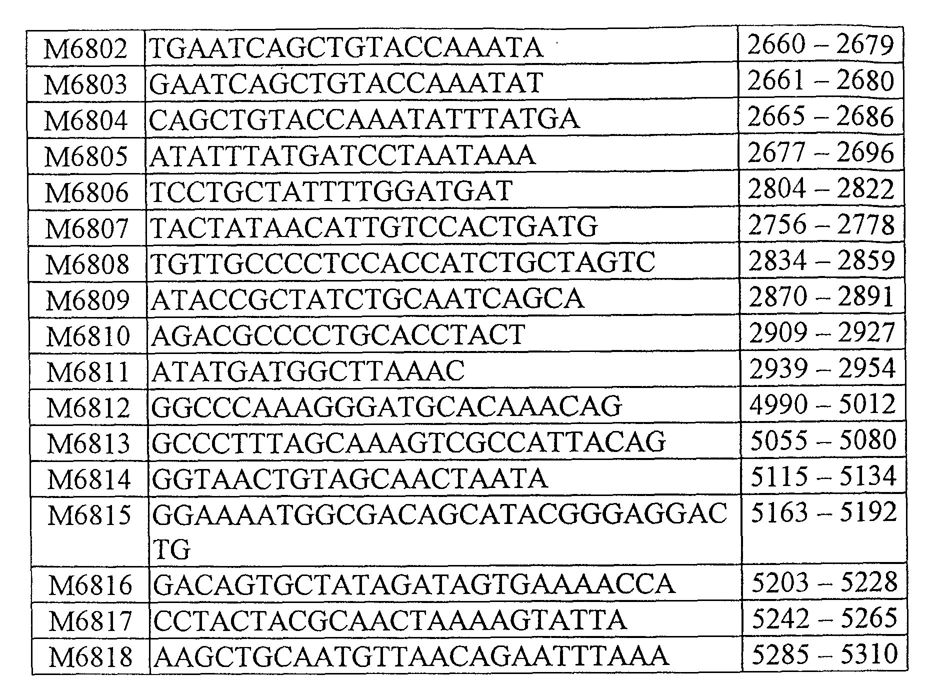

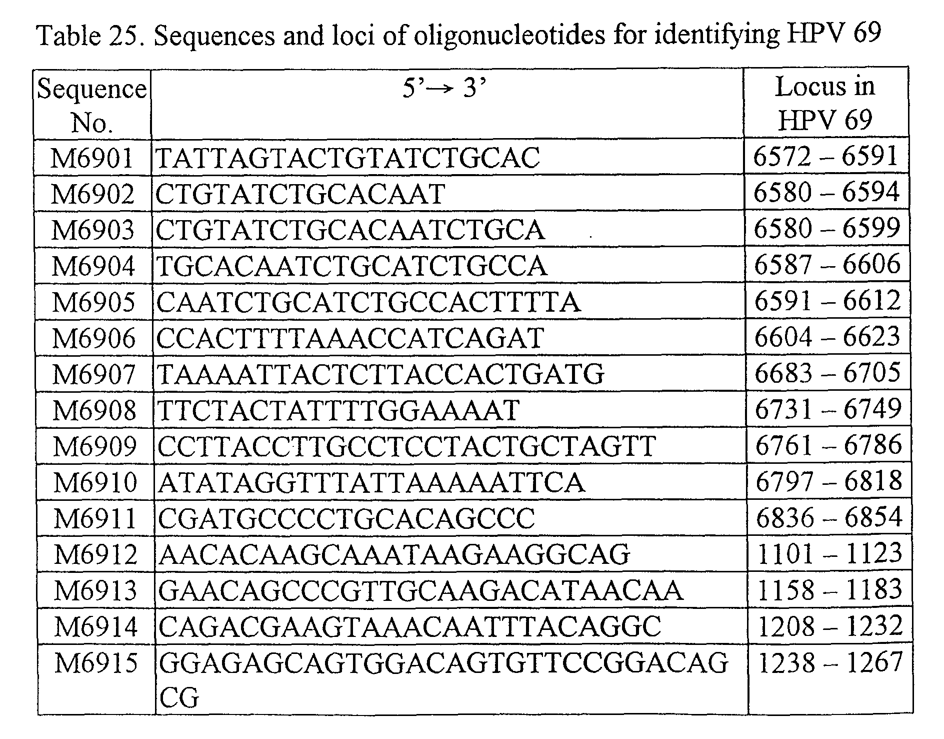

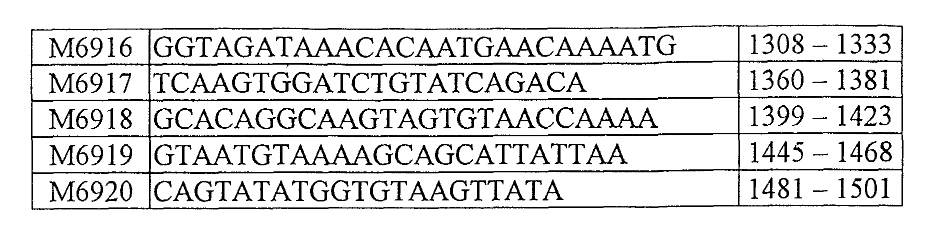

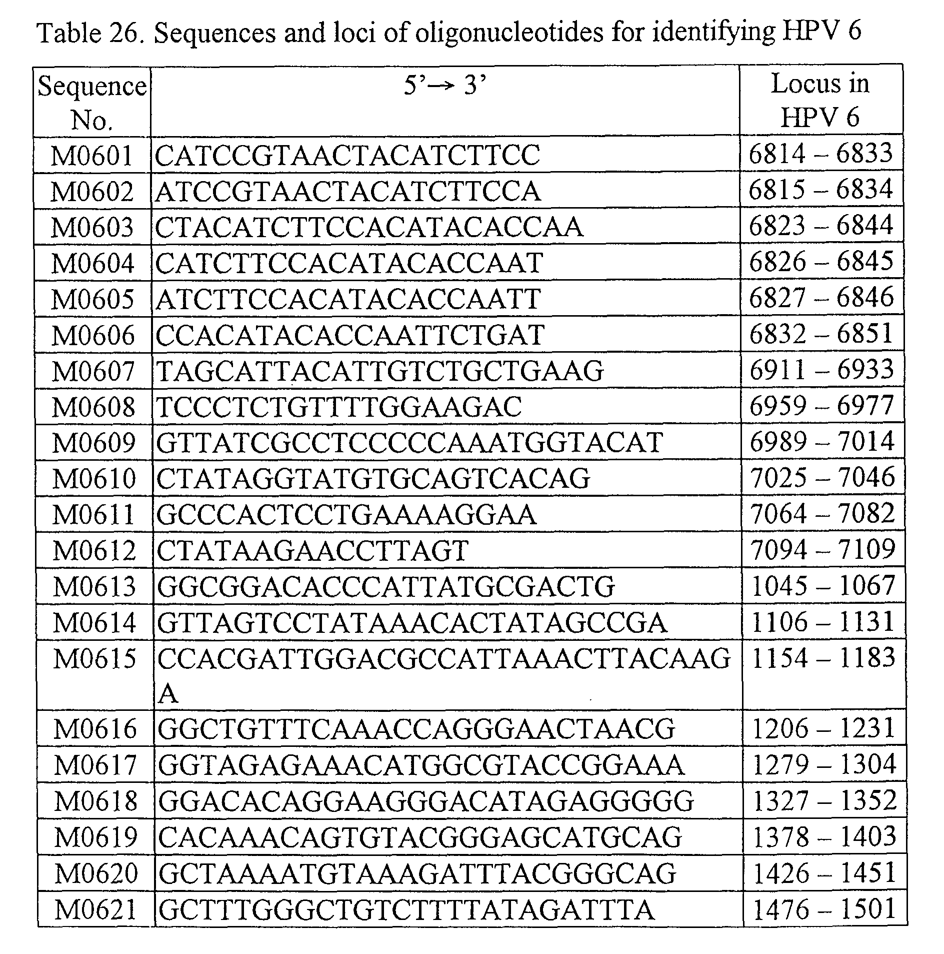

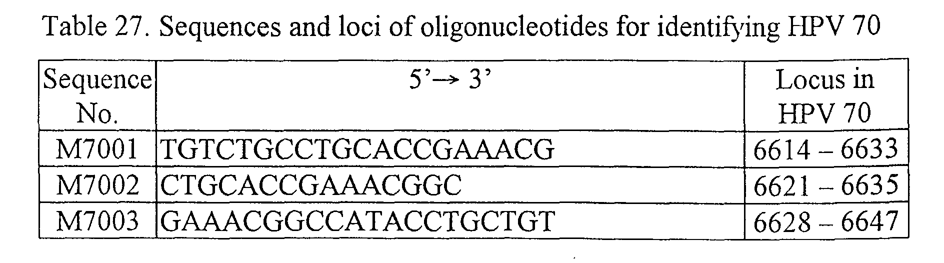

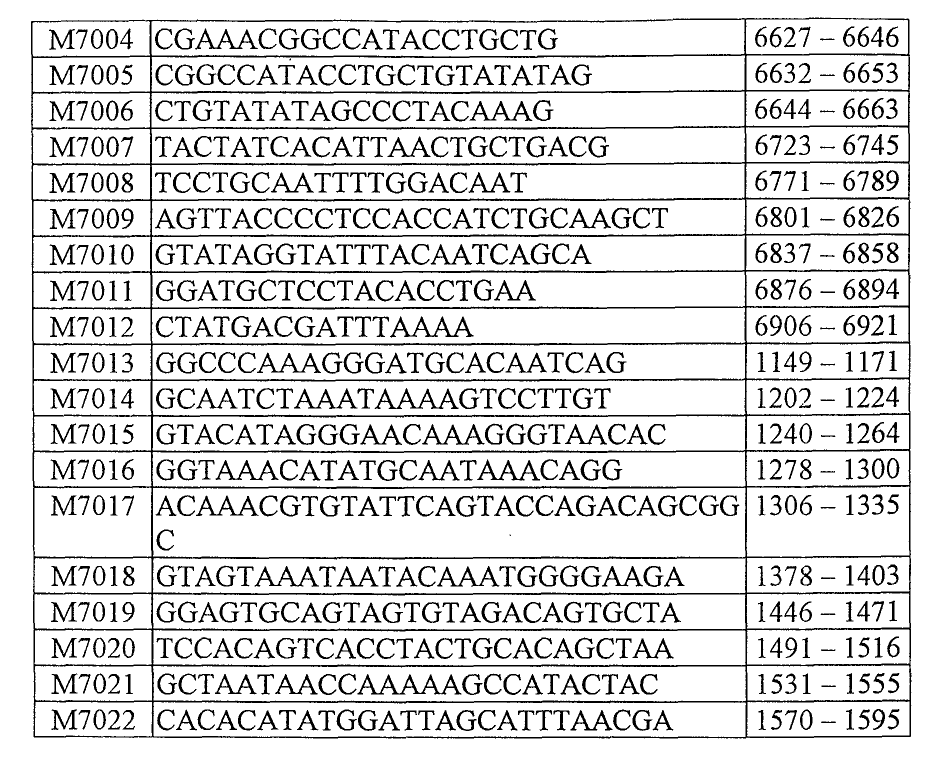

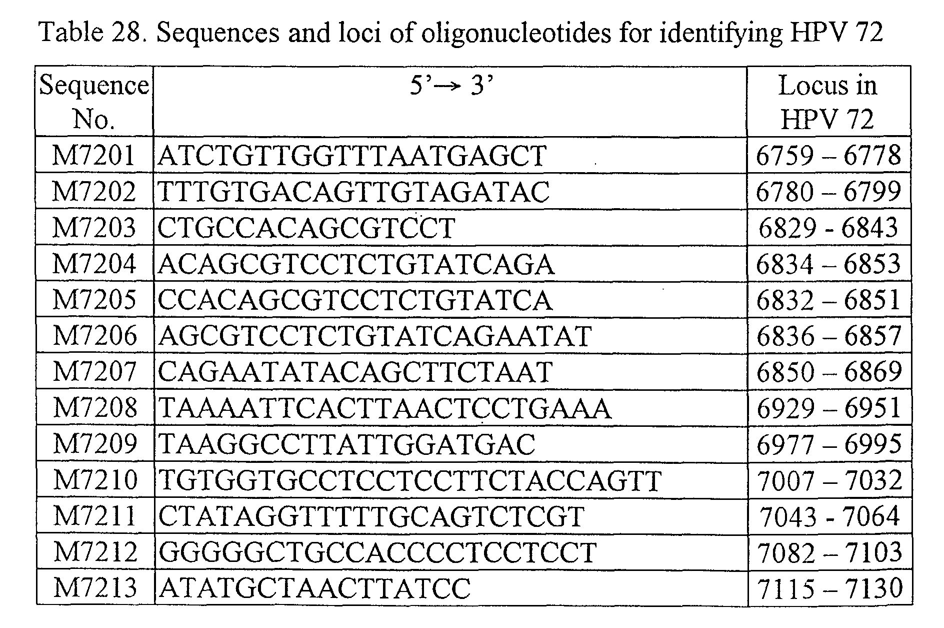

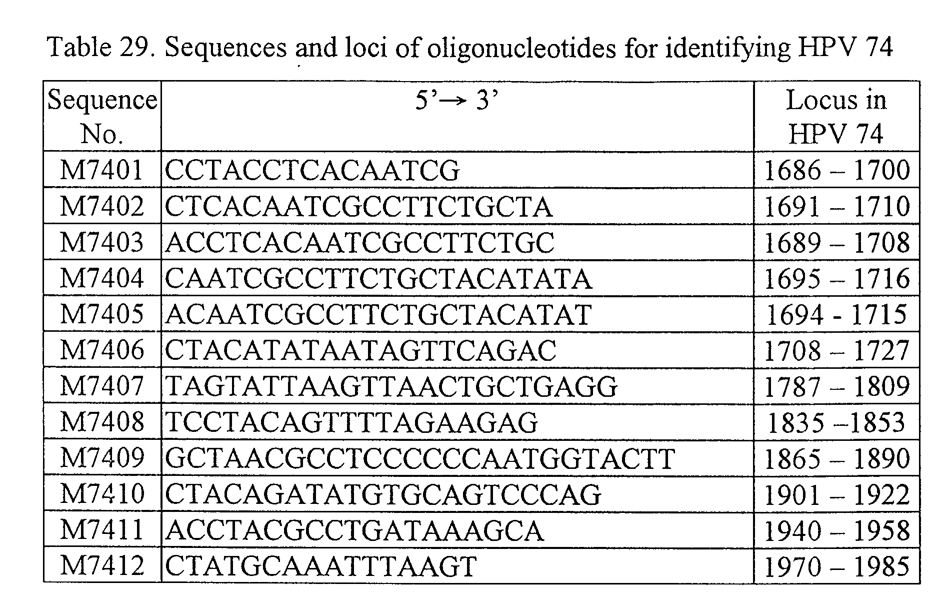

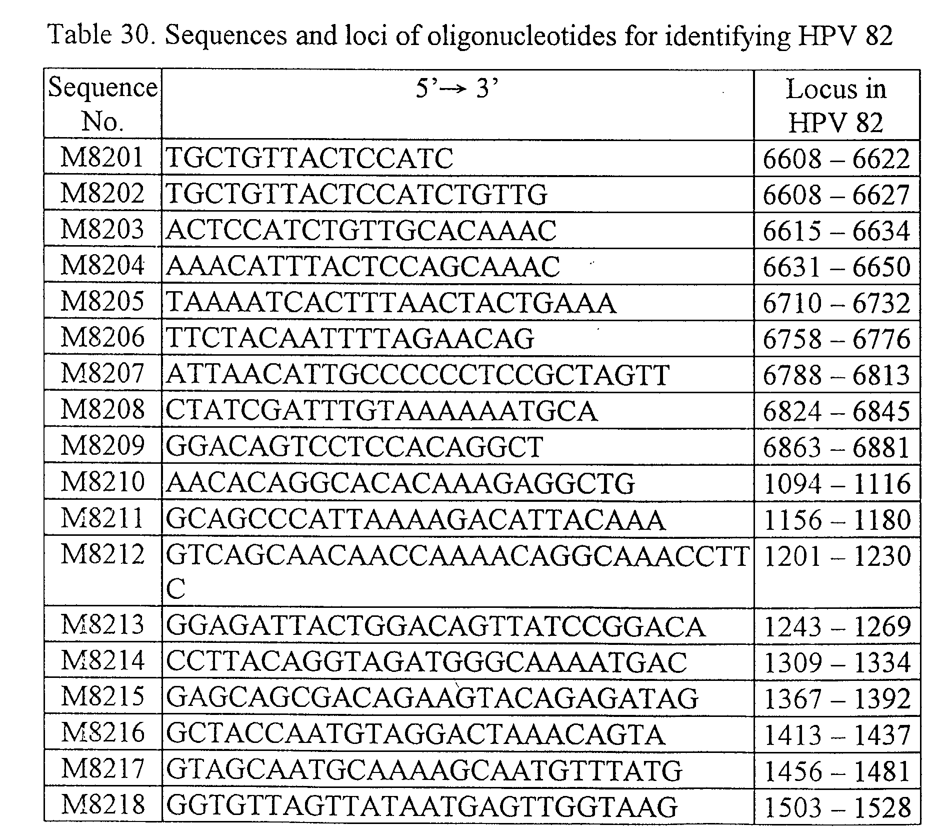

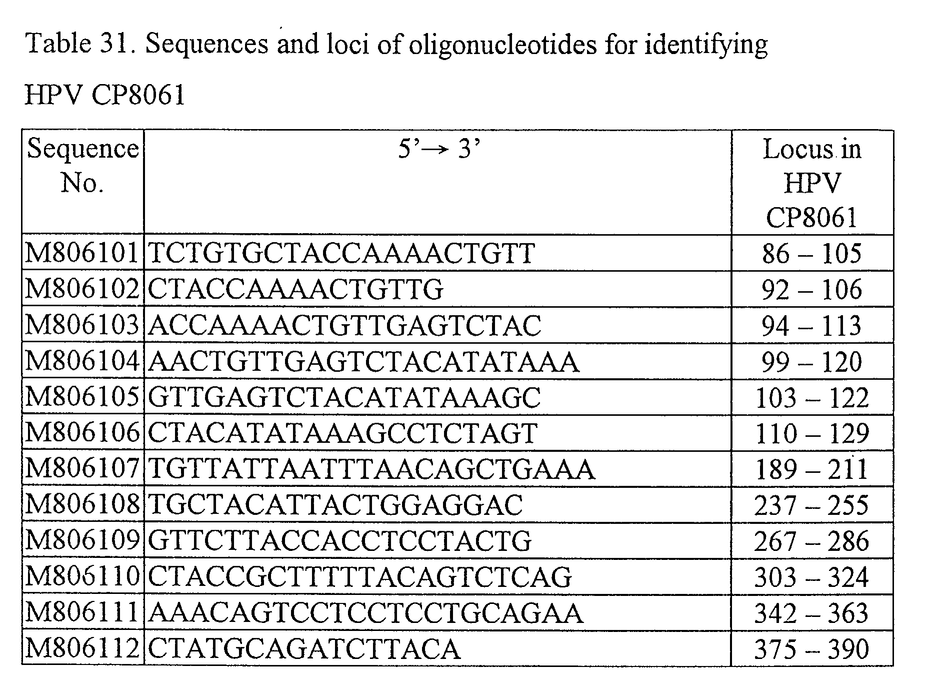

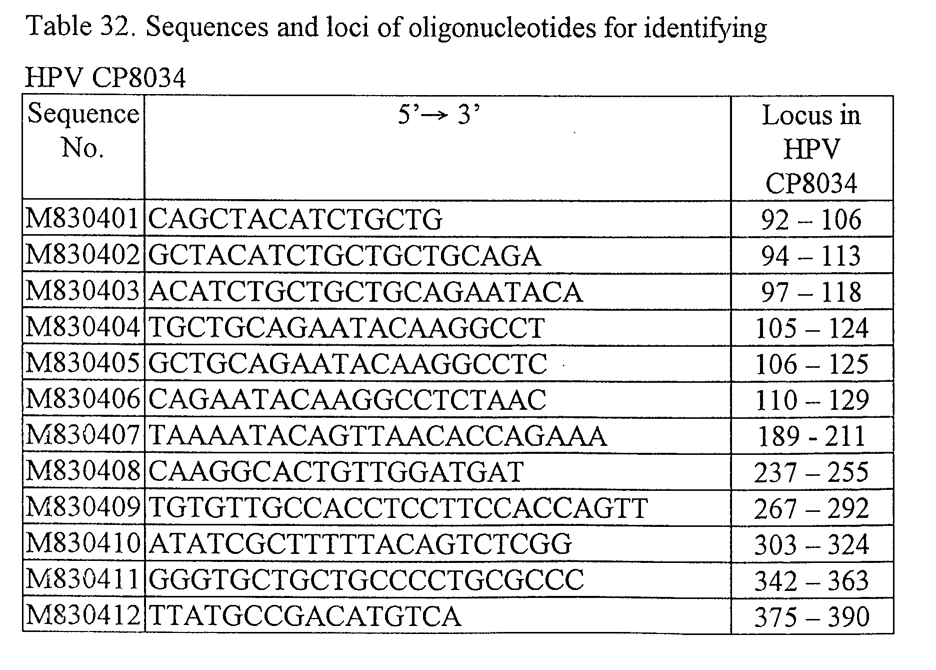

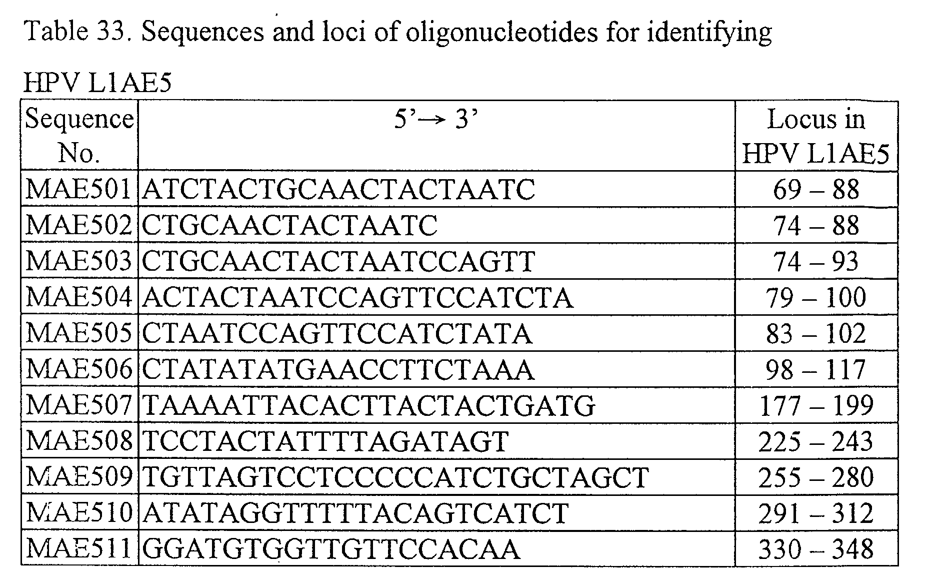

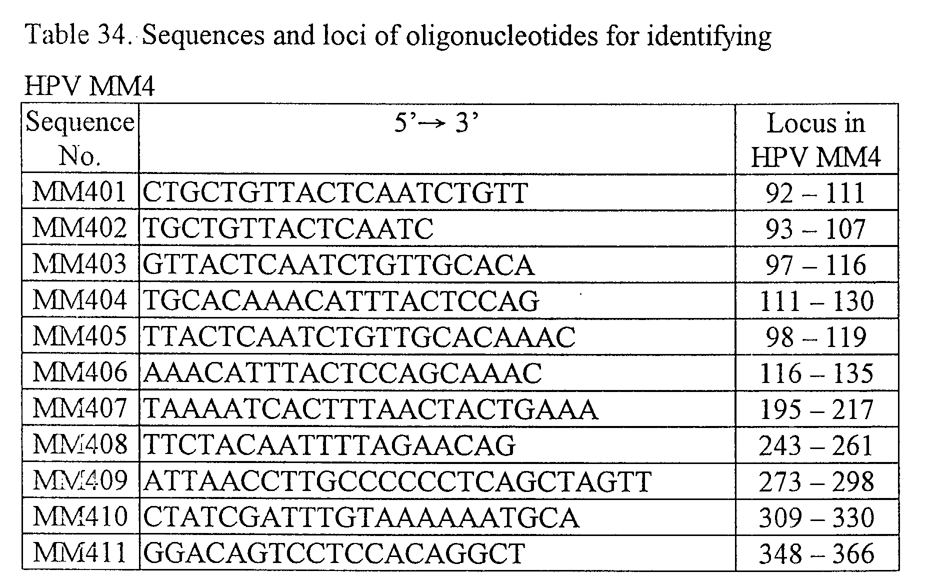

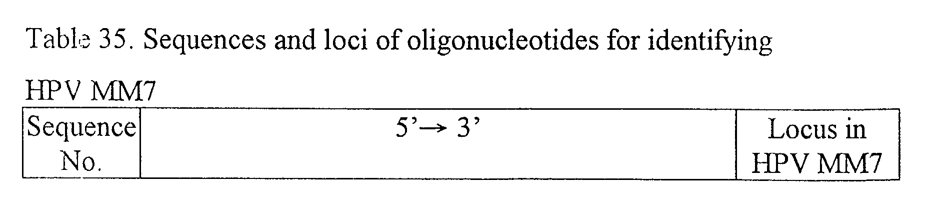

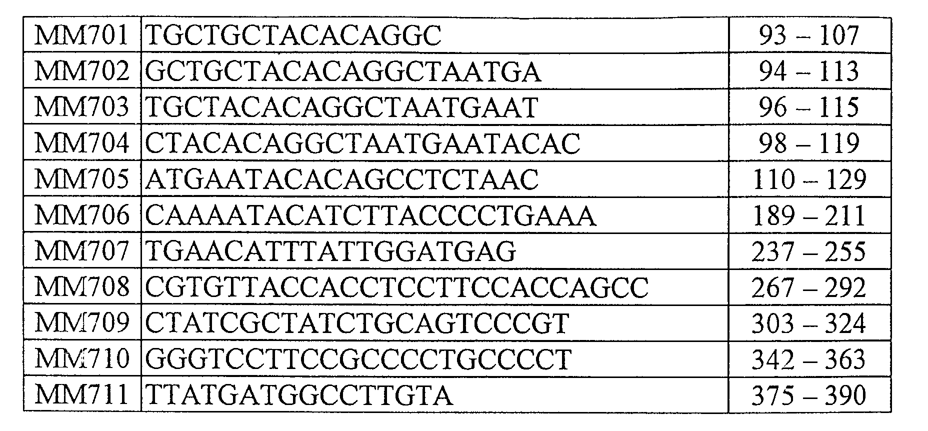

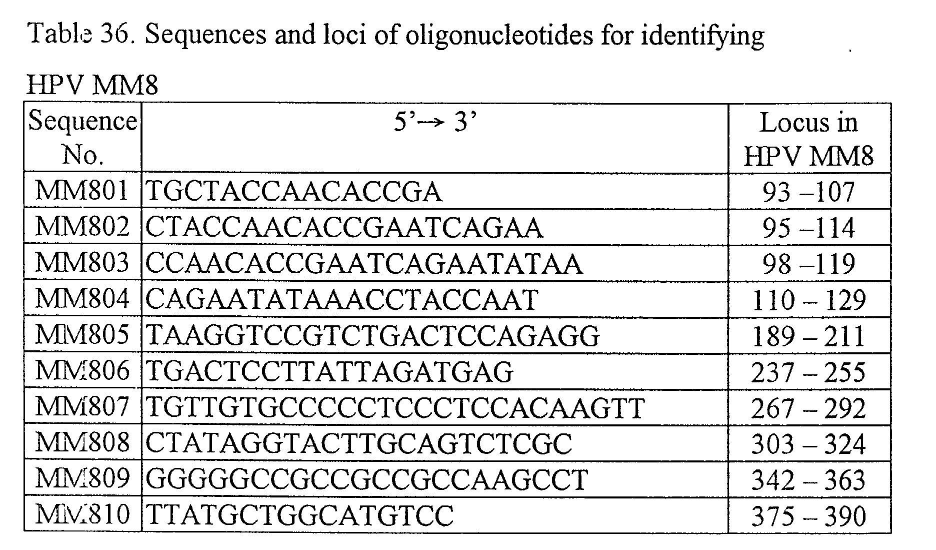

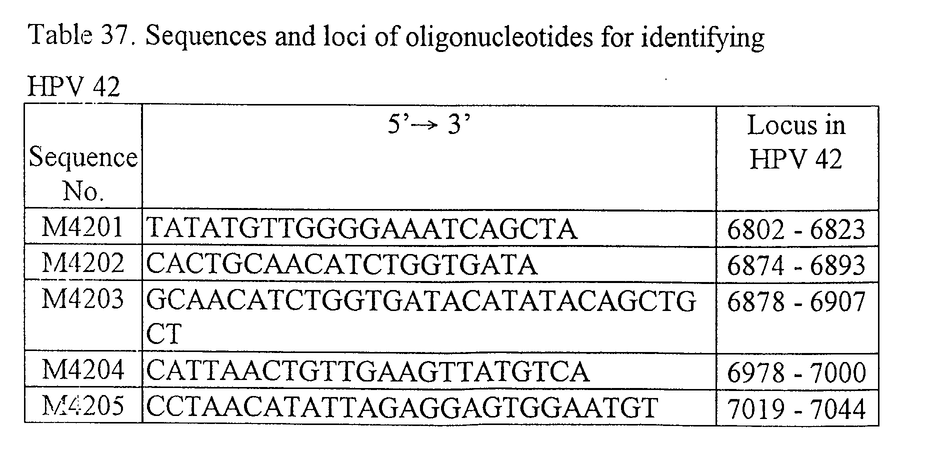

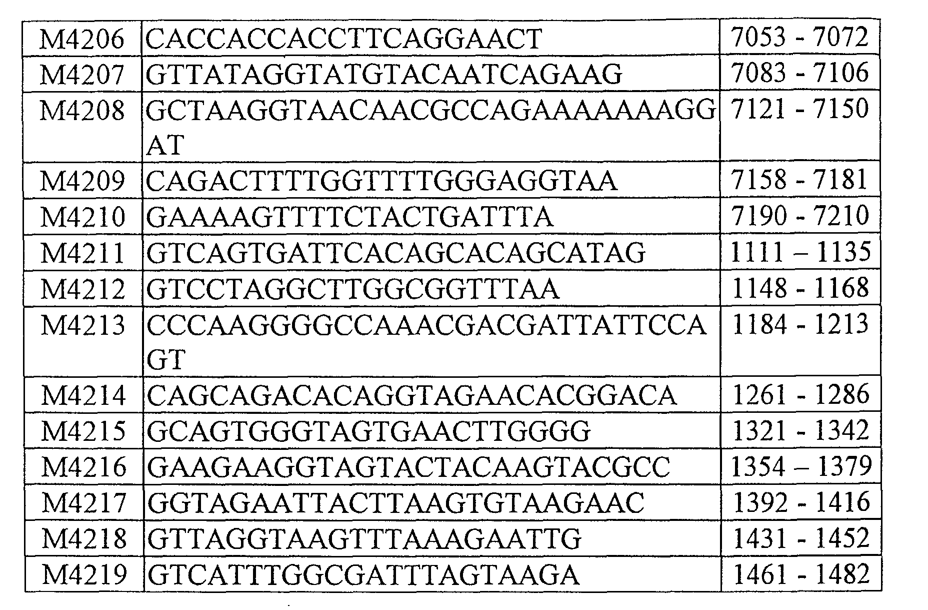

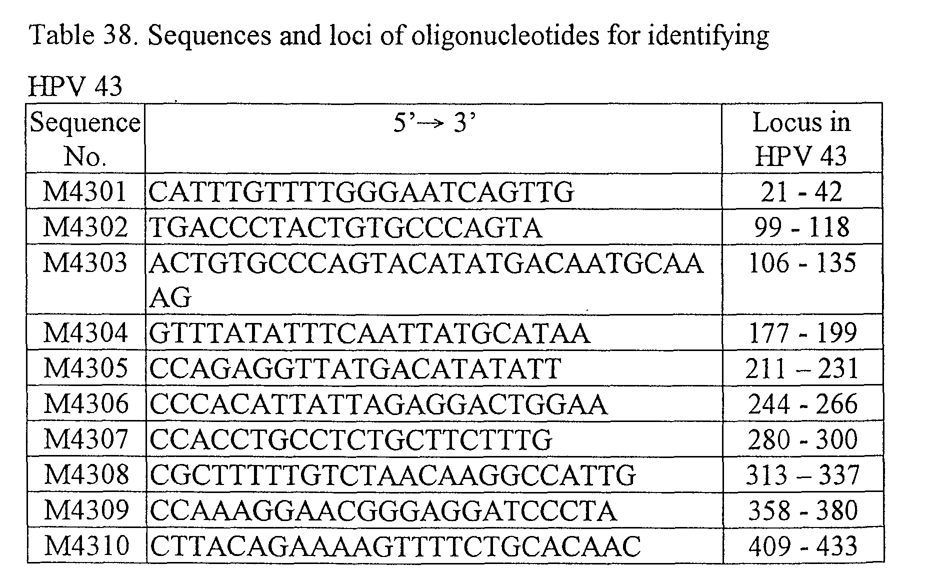

Each dot on the carrier 11 is an oligonucleotide selected from Tables 1 to 36. For example, an oligonucleotide on the carrier 11 could be selected from one of the sequences numbered M1101 to M1124 (shown in Table 1) for indentifying the subtype 11 of human papilloma viruses (HPV 11).

The method for mounting the oligonucleotides on the carrier 11 (the nylon membrane) is described as follows.
1.-TTTTTTTTTTTTTTT is added to the 3' end of the oligonucleotide provided by the present invention by terminal transferase according to the following steps 1.1 to 1.3.

| | |
|---|---|
| 1.1 Mixing the following components: 10X NEBuffer 4 | 5 µl |
| 2.5 mM CoCl₂ | 5 µl |
| oligonucleotide | 5 ∼ 300 pmol |
| 10 ∼ 300 mM dATP 'dCTP' dTTP or dGTP | 1 µl |
| Terminal Transferase (20U/ul) (NEW English BioLabs,M0252S) | 0.5 ∼ 5 µl |
| Add M.Q. H₂O to final volume | 50 µl |

1.2 The components are mixed at 37°C for 15∼60 minutes.
1.3 10 µl of 0.2 M EDTA (pH 8.0) is added to the mixture to stop the reaction.
2. The oligonucleotide having 3' end labeling is mounted on the carrier 11 according to the following steps 2.1 to 2.3.
2.1 The oligonucleotide having 3' end labeling is mounted on the carrier 11 by a needle having a 400 µm wide head. The distance between each dot is 1200 µm.
2.2 The carrier 11 having the dot array 12 thereon is exposed to UV light, and the detector 10 is formed.
2.3 The detector 10 is preserved in a drying box.

Please refer to Fig. 2. A detector 20 is the second embodiment of the present invention for simultaneously detecting and identifying subtypes of human papilloma viruses contained in a sample. The detector 20 includes a carrier 21 and a dot array 22. The carrier 21 is a glass plate having the dot array 22 mounted thereon. Each dot in the dot array 12 is an oligonucleotide (15∼30mer) for identifying a specific subtype of human papilloma viruses.

Each dot on the carrier 21 is an oligonucleotide selected from Tables 1 to 36. For example, an oligonucleotide on the carrier 21 could be selected from one of the sequences numbered M1101 to M1124 (shown in Table 1) for indentifying the subtype 11 of human papilloma viruses (HPV 11).

The method for mounting the oligonucleotides on the carrier 21 (the glass plate) is described as follows.
1. The surface of the carrier 21 is treated according to the following steps 1.1 to 1.8.
1.1 The carrier 21 is cleaned in non-fluorescent and soft cleaner.
1.2 The clean carrier 21 is immersed in 10% NaOH.
1.3 The carrier 21 is oscillated in double-distilled water, 1% HCl solution and methanol in sequence for 2 minutes, and dried in an oven.
1.4 The carrier 21 is immersed in 1% 3-aminopropyltrimethoxysilane (APTMS) in 95% aqueous acetone at room temperature for about 2 minutes.
1.5 The carrier 21 is washed in acetone, and the carrier 21 is dried in the oven at 110°C for 45 minutes.
1.6 The dried carrier 21 is immersed in 0.2% 1,4-phenylene diisothiocyanate, wherein the solvent is 10% pyridine in dimethyl formamide), at room temperature for 2 hours.
1.7 The carrier 21 is washed in methanol and acetone, and then the carrier 21 is dried.
1.8 The dried carrier 21 is preserved in a vacuum and dry box.
2. The oligonucleotides provided by the present invention are mounted on the carrier 21 (the glass plate) according to the following steps 2.1 to 2.3.
2.1 The oligonucleotide having 3' end labeling is mounted on the carrier 21 by a needle having a 400 µm wide head. The distance between each dot is 1200 µm.
2.2 The carrier 21 is immersed in 1% NH₄OH solution for about 2 minutes, washed in double-distilled water, and then dried at room temperature. Thus, the detector 20 is formed.
2.3 The detector 20 is preserved in a dried box.

The method provided by the present invention for simultaneously detecting and identifying subtypes of human papilloma viruses contained in a sample is described as follows.
1. The sample is treated according to the following steps 1.1 to 1.3.
1.1 The cells are centrifuged at 1,500 rpm at 20°C for 5 minutes.
1.2 The cell pellet is washed in 10 mM Tris (pH 8.5) and dissolved in 8 mM NaOH. Then, the solution is transfer to 1.5 mL micro-tube.
1.3 A proper amount of TreTaq (1U/µl) solution is added to the micro-tube. The reaction is carried out at 95°C for 1 hour. The DNA contained in the sample is obtained after centrifugation at 13,500 rpm, 20°C for 5 minutes. The otained DNA is preserved at -20°C.
2 Polymerase chain reactions are performed according to the following steps.
2.1 Glutaldehyde-3-phosphodehydrogenase gene is used as the internal control of the polymerase chain reactions according to the following steps 2.1.1 to 2.1.3.
2.1.1 Mixing the following components: 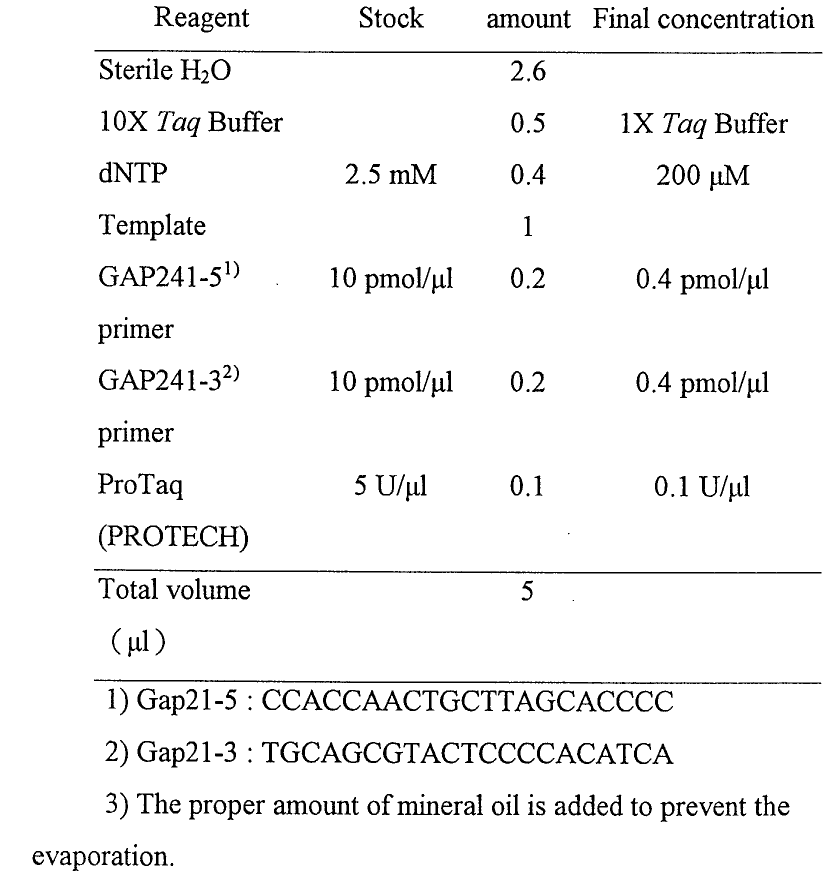
2.1.2 The polymerase chain reaction is performed according to the following programs. 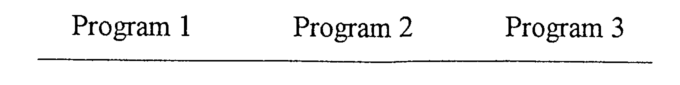 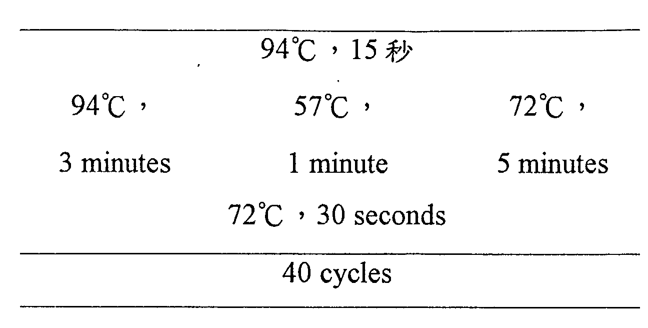
2.1.3 The product of the polymerase chain reaction is analyzed in 2.5% agarose/EtBr (0.5×TBE).
2.2 The DNA contained in the sample is proceeded the first round of polymerase chain reaction according to the following steps.
2.2.1 Mixing the following components:

| Reagent | Stock | Amount | Final concentration |
|---|---|---|---|
| Sterile H₂O | | 4.7-5.7 | |
| 10X *Taq* Buffer | | 1 | 1X *Taq* Buffer |
| dNTP | 2.5 mM | 0.8 | 200 µM |
| Template | | 1-2 | |
| BSA | 10 mg/ml | 0.1 | 0.1 µg/µl |
| Primer^{1,2)} | 10 pmol/µl | 0.6 | 0.6 pmol/µl |
| Primer^{1,2)} | 10 pmol/µl | 0.6 | 0.6 pmol/µl |
| ProTaq (PRO_{TECH}) | 5 U/µl | 0.2 | 0.1 U/µl |
| Total volume (µl) | | 10 | |

| | | | |
|---|---|---|---|
| 1) MY09/MY11 : Manos *et al*., *Cancer Cells* 1989, 7, 209-214 ; 2) E1 301L/E1 847R : *Ylitalo et al*., *J. Clin. Microbiol.* 1995, 33, 1822-1828 ; 3) The proper amount of mineral oil is added to prevent the evaporation. | | | |

2.2.2 The polymerase chain reaction is performed according to the following programs. 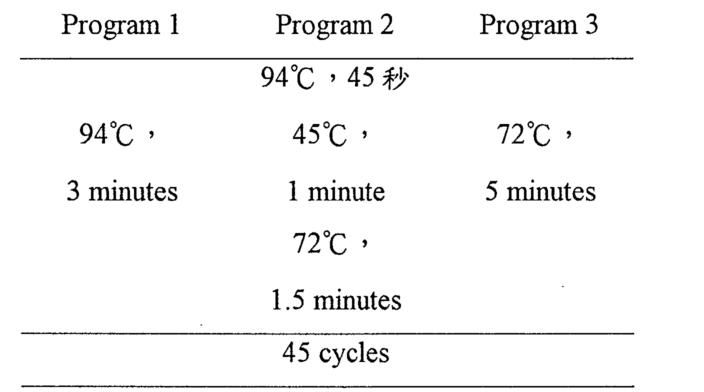
2.2.3 The product of the polymerase chain reaction is analyzed in 2.5% agarose/EtBr (0.5×TBE).
2.3 The DNA contained in the sample is proceeded the second round of polymerase chain reaction via internal primers according to the following steps.
2.3.1 Mixing the following components:

| Reagent | Stock | Amount | Final concentration |
|---|---|---|---|
| Sterile H₂O | | 11.75 | |
| 10X *Taq* Buffer | | 2.5 | 1X *Taq* Buffer |
| dNTP | 2.5 mM | 2 | 200 µM |
| First round PCR product | | 5 | |
| BSA | 10 mg/ml | 0.25 | 0.1 µg/µl |
| Primer¹⁾ | 10 pmol/µl | 1.5 | 0.6 pmol/µl |
| Primer^{1,2,3)} | 10 pmol/µl | 1.5 | 0.6 pmol/µl |
| ProTaq (PRO_{TECH}) | 5 U/µl | 0.5 | 0.1 U/µl |
| Final volumn (µl) | | 25 | |

| | | | |
|---|---|---|---|
| 1) GP5+/GP6+ : De Roda Husman *et al*., *J. Gen. Virol.* 1995, 76, 1057-1062 ; | | | |
| 2) E1 350L/E1 547R : Ylitalo *et al. J. Clin. Microbiol.* 1995, 33, 1822-1828 ; 3) The 5' end of the GP6+ and E1 547R primers could be labeled with biotin or Cy5 fluorescent substances. 4) The proper amount of mineral oil is added to present the evaporation. | | | |

2.3.2 The polymerase chain reaction is performed according to the following programs.

| Program 1 | Program 2 | Program 3 |
|---|---|---|
| | 94°C ^{,} | |
| | 45 seconds | |
| 94°C ^{,} | 45°C ^{,} | 72°C ^{,} |
| 3 minutes | 1 minute | 5 minutes |
| | 72°C ^{,} | |
| | 1.5 minutes | |
| | 45 cycles | |

2.3.3 The product of the polymerase chain reaction is analyzed in 2.5% agarose/EtBr (0.5×TBE).
3. The detector 10 provided by the present invention is used for identifying the subtypes of human papilloma viruses according to the following steps.
3.1 The detector 10 is immersed in 2x SSC solution for 5 minutes.
3.2 The detector 10 is immersed in a buffer containing salmon sperm DNA (50 µg/µl), and the oligonucleotides mounted on the detector 10 are pre-hybridized with the salmon sperm DNA at 35°C for 30 minutes.
3.3 The PCR product having biotin labeled thereon is added into and mixed with a buffer containing salmon sperm DNA (50 µg/µl) at 95°C for about 5 minutes. The denatured DNA is placed on ice.
3.4 The denature DNA is added to the detector 10 and hybridized with the oligonucleotides at 35°C for 4 hours or overnight.
3.5 The detector 10 is washed in 2x SSC/1% SDS solution at 35°C for 15 minutes.
3.6 The detector 10 is washed in 0.2x SSC/0.1% SDS solution at 35 °C for 15 minutes.
3.7 The detector 10 is treated in 0.5% isolation reagent for 1 hour.
3.8 The detector 10 is treated with avidin-alkalinephosphatase for about 1 hour.
3.9 The detector 10 is washed in 1x PBST solution.
3.10 The detector 10 is washed in Tris/NaCl solution.
3.11 The detector 10 is treated with NBT/BCIP at room temperature to show the reacting dot in blue.
3.12 The blue dot having the specific oligonucleotide sequence presents the specific subtype of human papilloma viruses contained in the sample.
4. The detector 20 provided by the present invention is used for identifying the subtypes of human papilloma viruses according to the following steps.
4.1 The PCR product having Cy5 labeled thereon is purified by PCR Clean Up-M System (Viogene, USA), and the PCR product is precipitated in ethanol. Then, the PCR product is dried.
4.2 The precipitated DNA is dissolved in 12 µl of the buffer (2x SSC/0.1% SDS), and centrifugated for 1 minute, and then placed on boiled water for 2 minutes. Then, the mixture is placed on ice for 5 minutes.
4.3 The mixture is centrifugated for 30 seconds, and 10 µl of the mixture is added to the left side of the dot array 22. A cover slice is carefully covered on the dot array from the left side of the dot array to prevent the bubble formation. Then, the detector 20 is place in Humid Chamber (Sigma, USA), and the dot array is faces downward at 35°C for 4 hours or overnight.
4.4 The detector 20 is vertically placed in the solution A (2x SSC/1% SDS), and the detector is slightly oscillated apart from the cover slice. Then, the detector 20 is washed in a shaker at 160 rpm for 12 minutes.
4.5 The detector 20 is washed in the solution B (0.2x SSC/0.1% SDS) and oscillated at 35°C for 12 minutes. The detector 20 is washed in water. Then the detector 20 is dried.
4.6 The dried detector 20 is scanned by GenePix™4000 (Axon, USA), excited by the light having 635 nm of wavelength, and analyzed by GenePixPro 3.0 (Axon, USA).

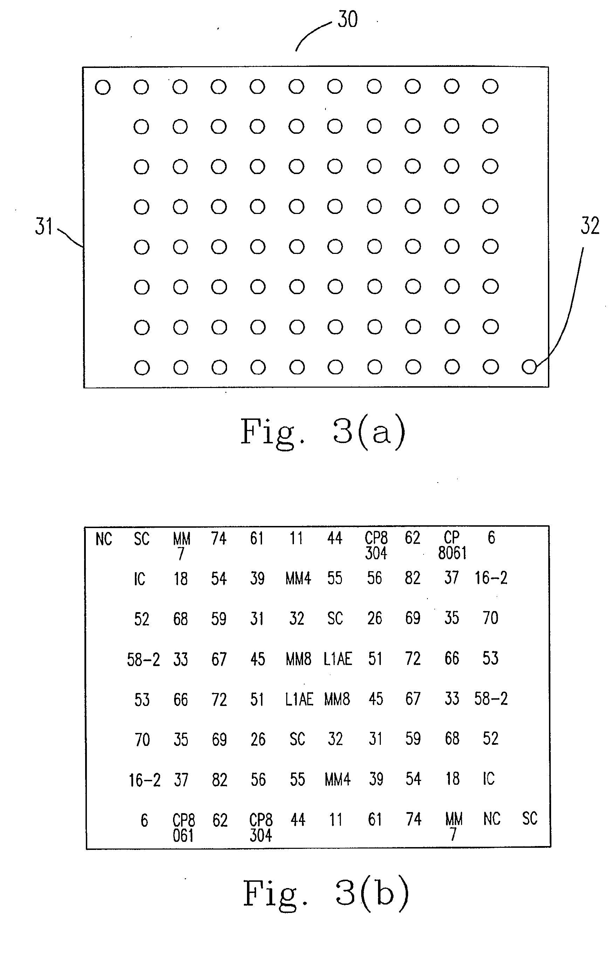

Please refer to Figs. 3(a) and (b). Fig. 3(a) is a schematic view showing the third embodiment of the present invention. The detector 30 provided by the present invention is used for simultaneously detecting and identifying subtypes of human papilloma viruses contained in a sample. The detector 30 includes a carrier 31 and a dot array 32.

The carrier 31 is a nylon membrane. The actual length of the nylon membrane is about 1.44 cm and the actual width of the nylon membrane is about 0.96 cm. The dot array is mounted on the carrier 31 according to the foresaid method, wherein the distance between each dot is about 1.2 mm and the diameter of each dot is about 0.4 mm. Each dot is an oligonucleotide (15∼30mer), and each oligonucleotide is used for specifically identifying a subtype of human papilloma viruses. The sequence of the oligonucleotide is selected from the Tables 1 to 36.

The subtype of human papilloma viruses identified by each dot of the dot array 32 is illustrated in Fig. 3(b). SC (system control) presents the PCR product amplified from any subtype of human papilloma viruses and biotin-contained primer. NC (negative control) presents the plants DNA fragment irrelevant to HPV. IN (internal control) presents the sequence 5'-gcccagactgtgggtggcag-3' of the housekeeping gene, Glyceraldehyde-3 -Phosphate Dehydrogenase (GAP-DH).

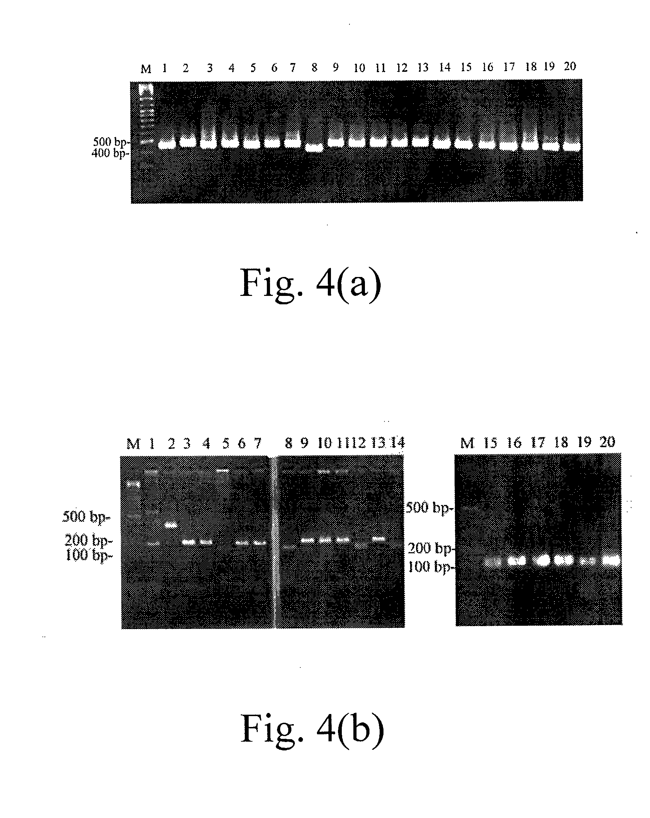

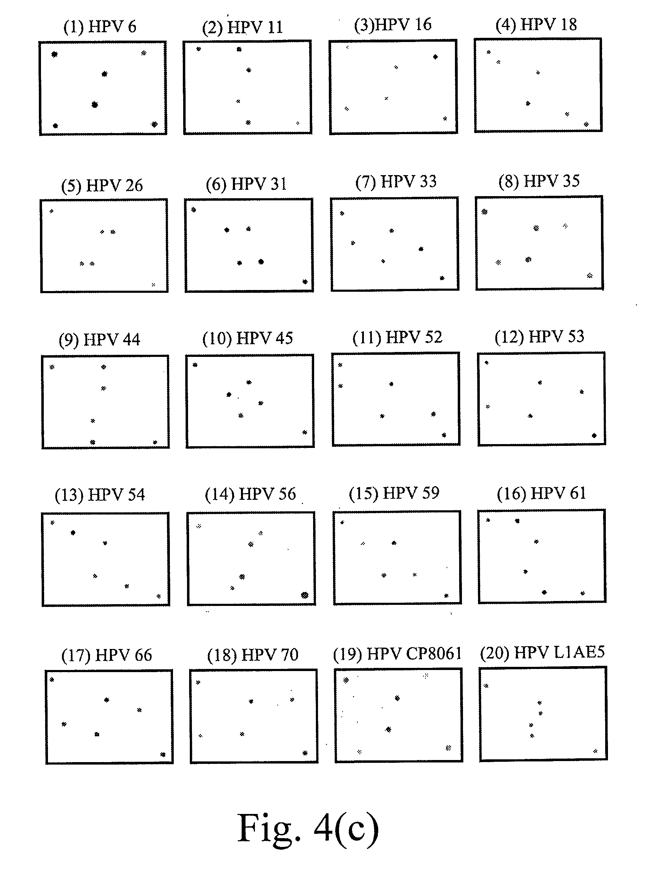

The plural detector 30 are used for identifying positive clones of human papilloma viruses according to the foresaid method, and the results are shown in Figs. 4(a) to (c). The positive clones are respective amplified by using MY11/MY09 primers and the first round of polymerase chain reaction. The products of the first polymerase chain reaction are analyzed in 2.5% agarose/EtBr, and the electrophoresis results are shown in Fig. 4(a). The products of the first round polymerase chain reaction are respective amplified by using GP5/GP6 primers and the second round of polymerase chain reaction. The products of the second polymerase chain reaction are analyzed in 2.5% agarose/EtBr, and the electrophoresis results are shown in Fig. 4(b). The numbers labeled in Figs. 4(a) and (b) and the corresponding HPV clones are illustrated in Table 39.

**Table 39**

| No. | HPV clone | No. | HPV clone | No. | HPV clone |
|---|---|---|---|---|---|
| M | DNA marker | 7 | HPV 33 | 14 | HPV 56 |
| 1 | HPV 6 | 8 | HPV 35 | 15 | HPV 59 |
| 2 | HPV 11 | 9 | HPV 44 | 16 | HPV 61 |
| 3 | HPV 16 | 10 | HPV 45 | 17 | HPV 66 |
| 4 | HPV 18 | 11 | HPV 52 | 18 | HPV 70 |
| 5 | HPV 26 | 12 | HPV 53 | 19 | HPV CP8061 |
| 6 | HPV 31 | 13 | HPV 54 | 20 | HPV L1AE5 |

The foresaid products numbered 1 to 20 of the second polymerase chain reactions are respectively detected by the detectors 30, and the results are shown in Fig. 4(c). According to the comparison between the results shown in Fig. 4 (c) and Fig. 3(b) based on the "SC" dot, the detector 30 provided by the present invention is used for precisely identifying the subtype of human papilloma viruses. Furthermore, no cross reactions occur in the detection.

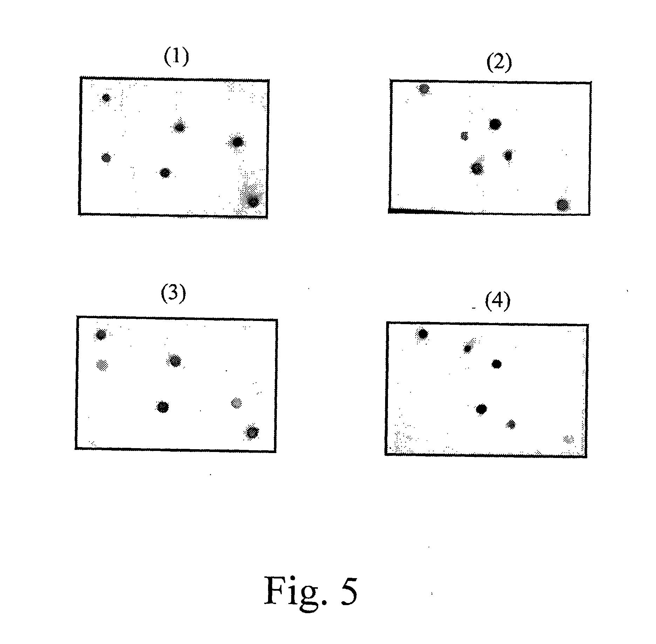

In addition, the detectors 30 are used for detecting and identifying the subtypes of human papilloma viruses contained in a sample, and the results are shown in Fig. 5. According to the comparison between the results shown in Fig. 5 and Fig. 3(b) based on the "SC" dot, HPV 53 is contained in the sample (1), HPV 45 is contained in the sample (2), HPV 52 is contained in the sample (3), and HPV 39 is contained in the sample (4).

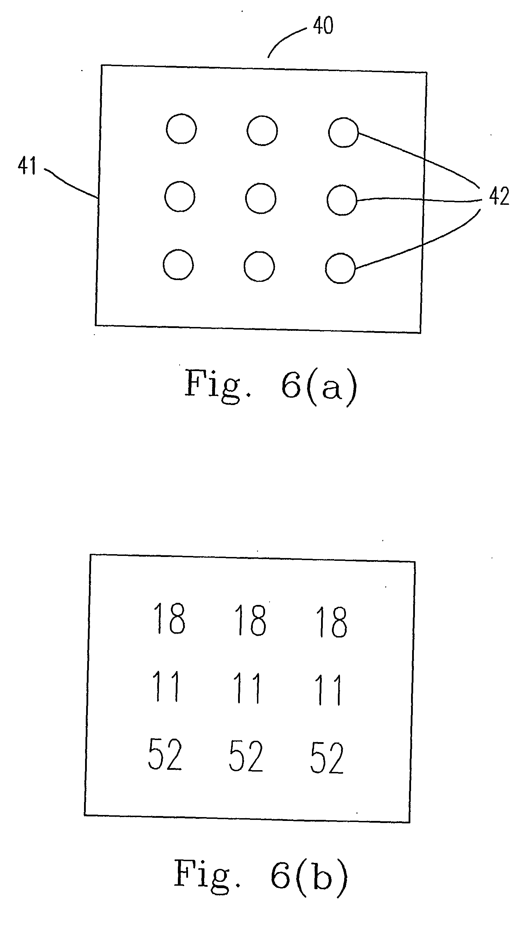

Please refer to Figs. 6(a) and (b). Fig. 6(a) is a schematic view showing the fourth embodiment of the present invention. The detector 40 is used for simultaneously detecting and identifying the subtypes of human papilloma viruses contained in a sample. The detector 40 includes a carrier 41 and a dot array 42. The carrier 41 is a glass plate, and the dot array 42 is mounted on the glass plate according to the foresaid method. Each dot is an oligonucleotide (15∼30mer) for specifically identifying a subtype of human papilloma viruses. The sequences of the oligonucleotides are selected from Tables. 1 to 36. The subtype of human papilloma viruses identified by each dot of the dot array 42 is illustrated in Fig. 6(b).

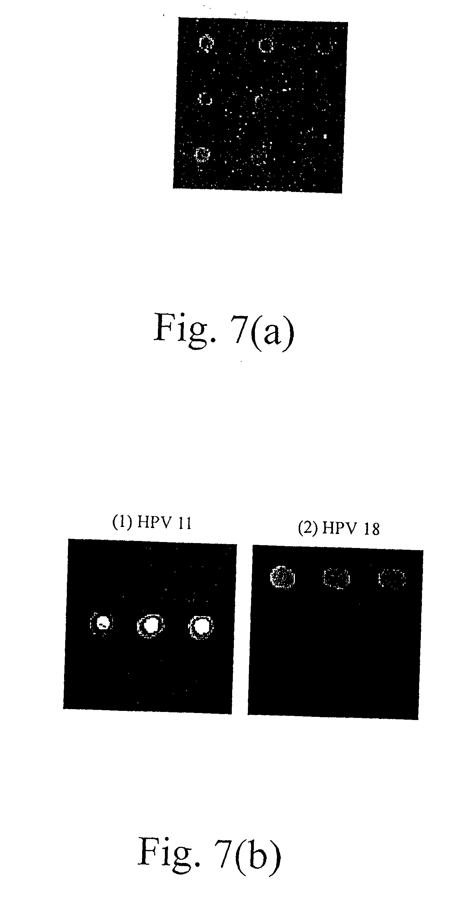

The detector 40 is stained with SYBR Green II, scanned by GenePix™ 4000 (Axon, USA) and excited by the light having 635 nm of wavelength. The result is shown in Fig. 7(a).

In addition, the positive clones of HPV 11 and HPV 18 are respectively treated with two rounds of polymerase chain reactions, and the products of the polymerase chain reactions are respectively analyzed by the detectors 40. The results are shown in Fig. 7(b), wherein the red fluorescent dot presents positive result. According to the comparison between the results shown in Fig. 7(b) and Fig. 6(b), the detector 40 provided by the present invention is used for precisely identifying the subtype of human papilloma viruses. Furthermore, no cross reactions occur in the detection.

## Claims

1. A detector (10, 20, 30) for simultaneously detecting and identifying subtypes of human papilloma viruses (HPV) contained in a sample, comprising:
a carrier (11, 21, 31) having a first part and a second part for carrying the sample thereon;
a first oligonucleotide carried on the first part of the carrier; and
a second oligonucleotide carried on the second part of the carrier,
**characterized in that** the first and second oligonucleotides respectively hybridized with deoxyribonucleic acids contained in a first subtype of human papilloma virus and a second subtype of human papilloma virus for simultaneously detecting and identifying subtypes of human papilloma viruses.

2. The detector (10, 20, 30) according to claim 1, **characterized in that** the carrier (11, 21, 31) is made of nylon.

3. The detector (10, 20, 30) according to claim 1, **characterized in that** the carrier (11, 21, 31) is a glass plate.

4. The detector (10, 20, 30) according to claim 1, **characterized in that** the first and second subtypes of human papilloma viruses are respectively selected from one of following viruses: 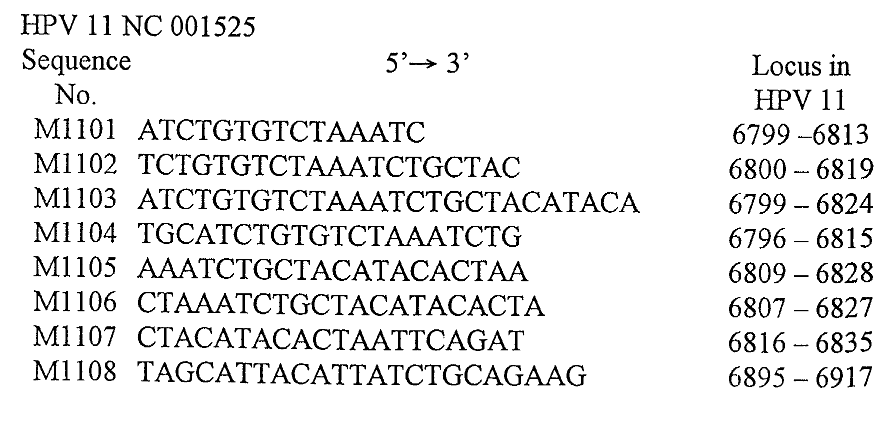 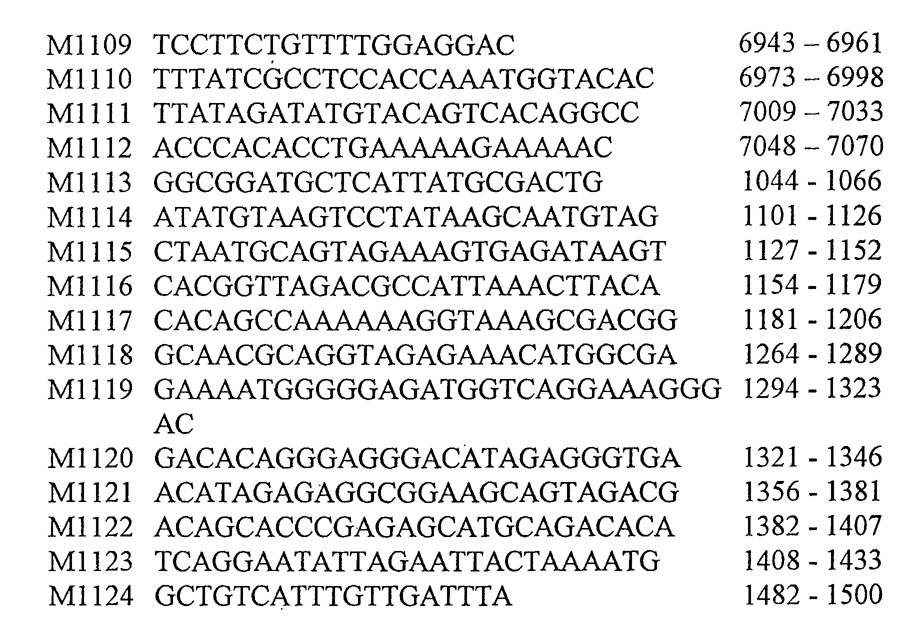 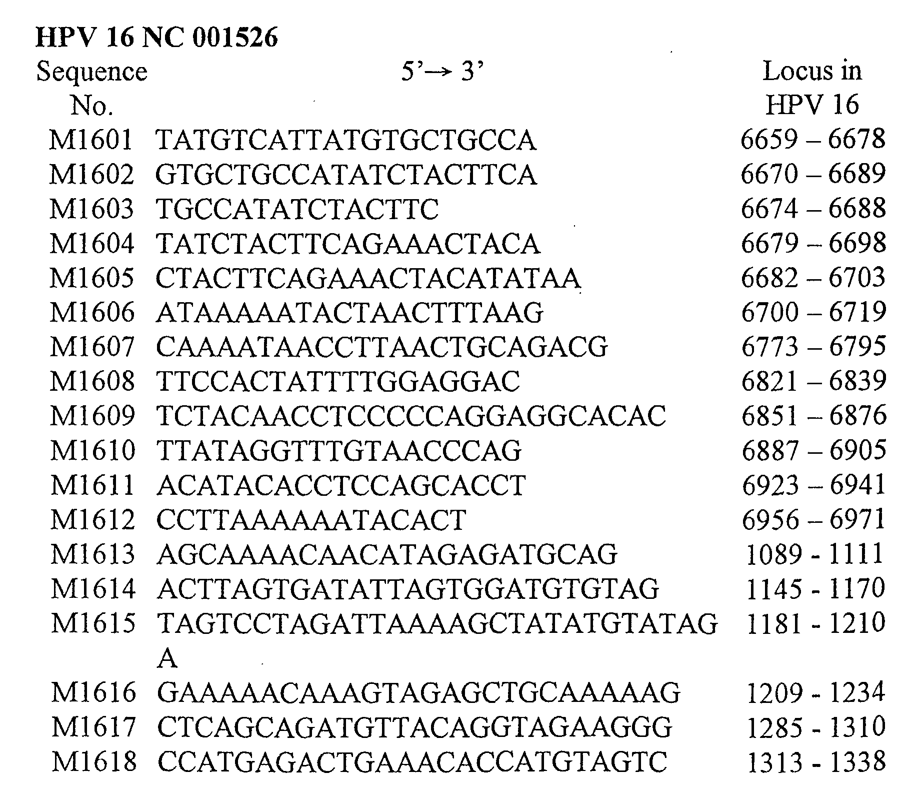 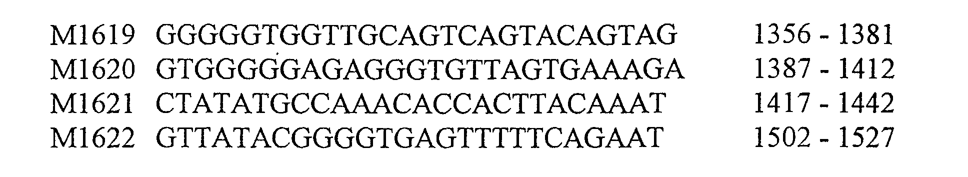 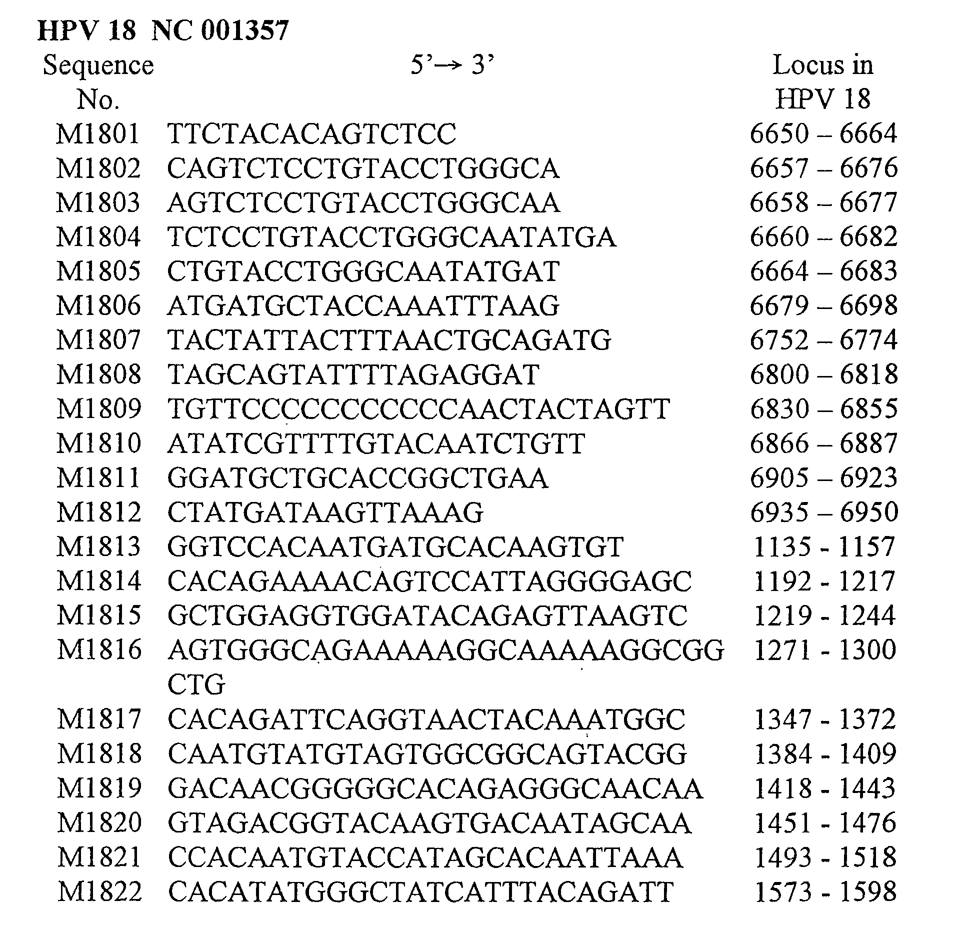 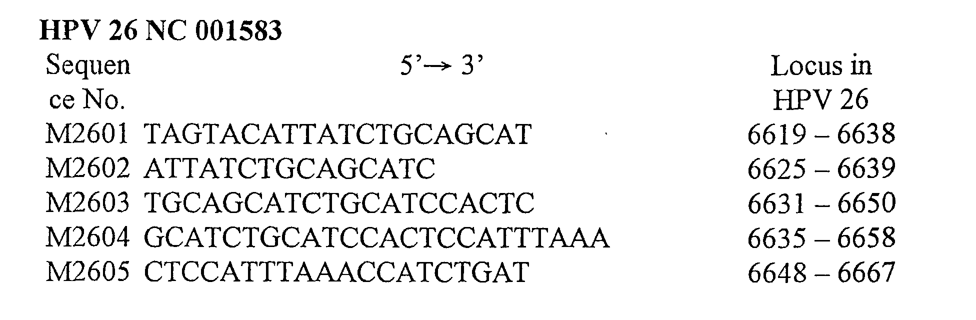 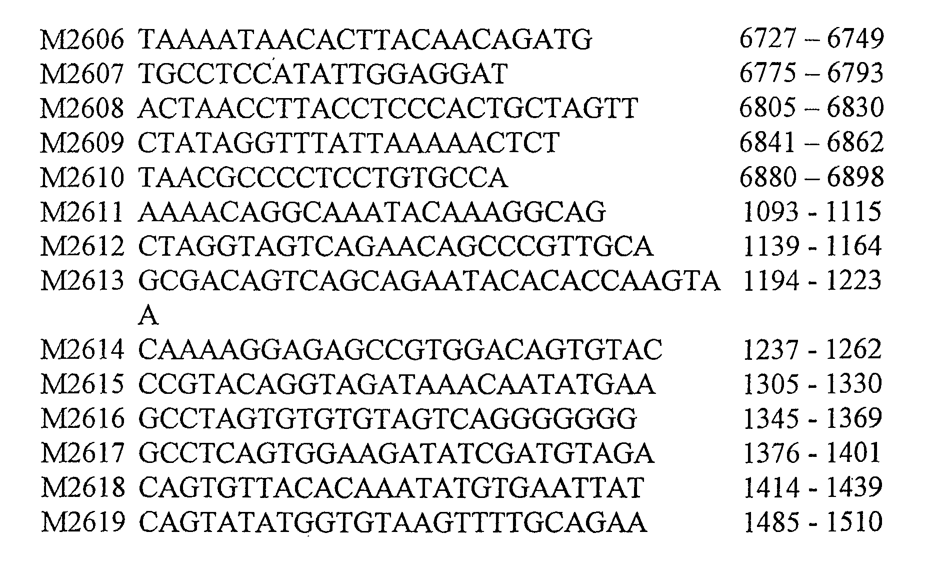 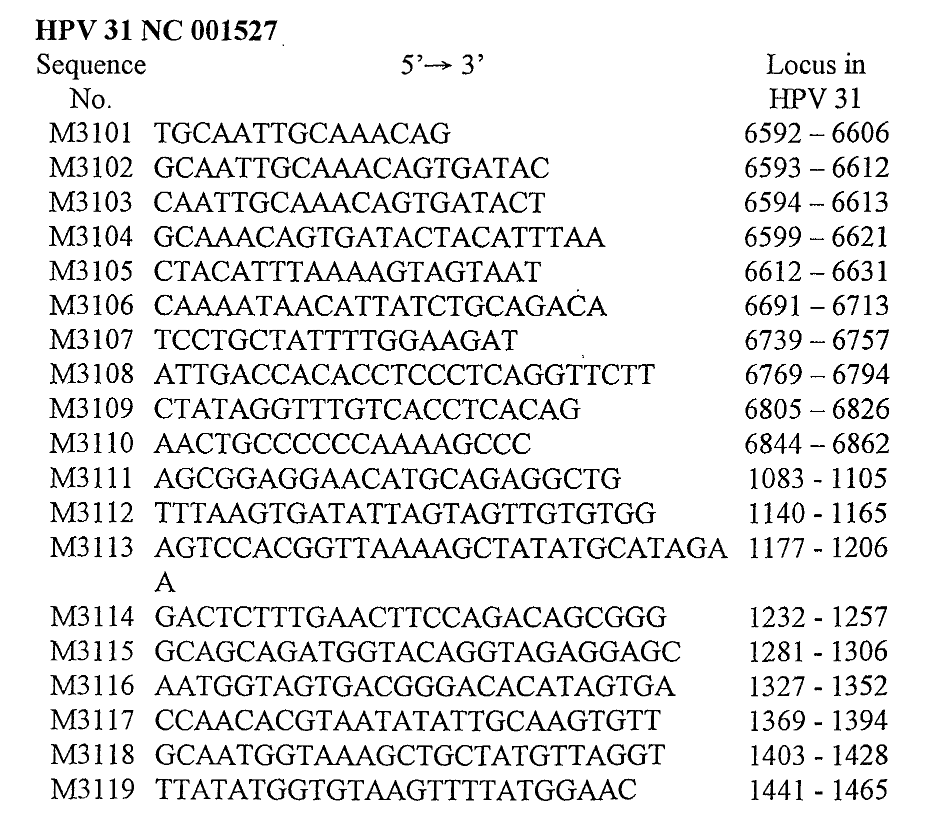 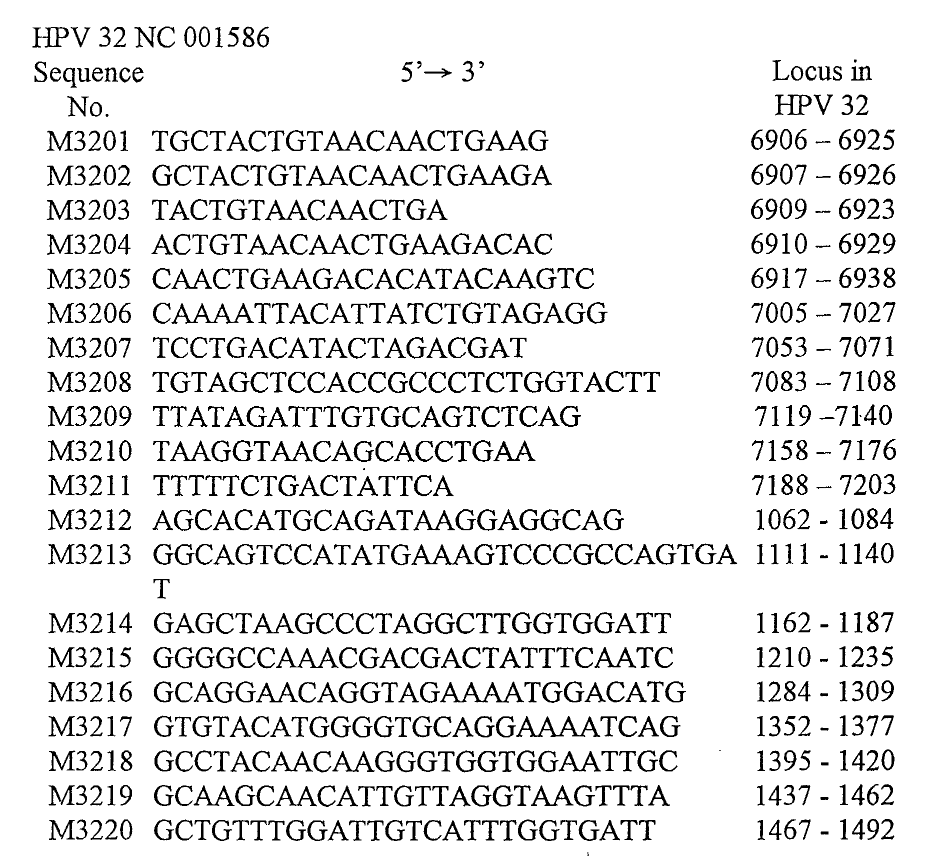 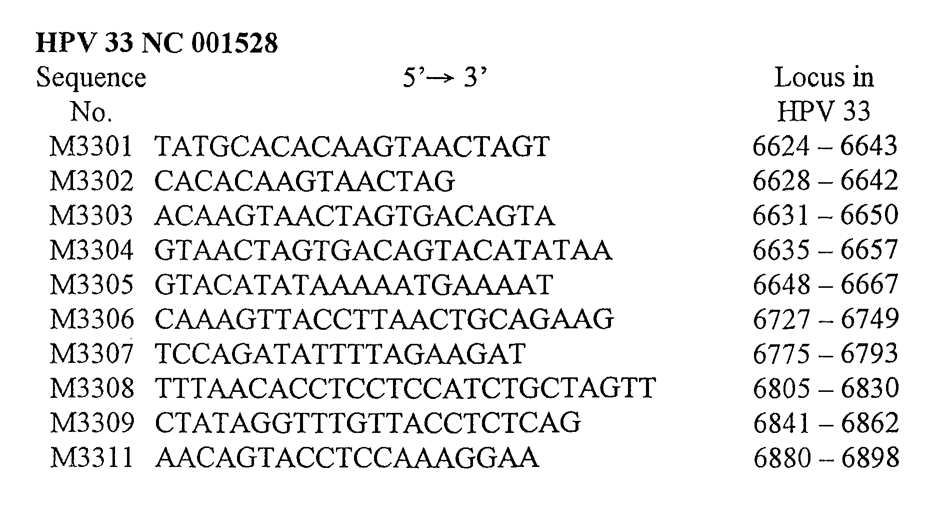 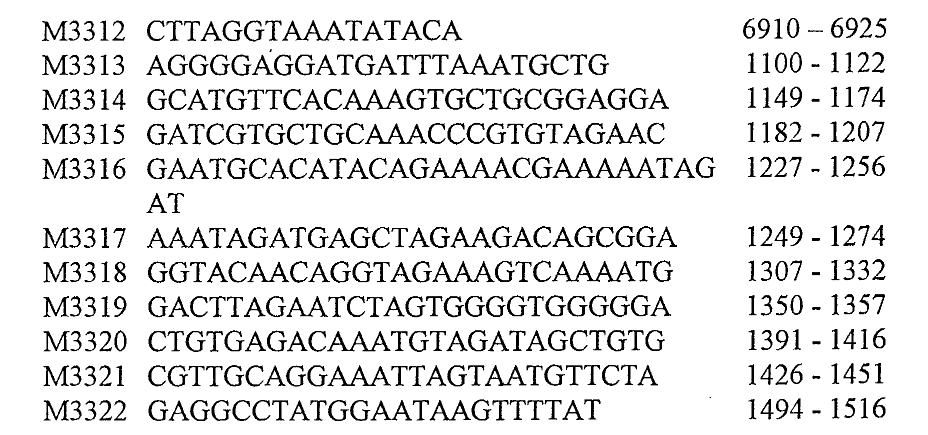 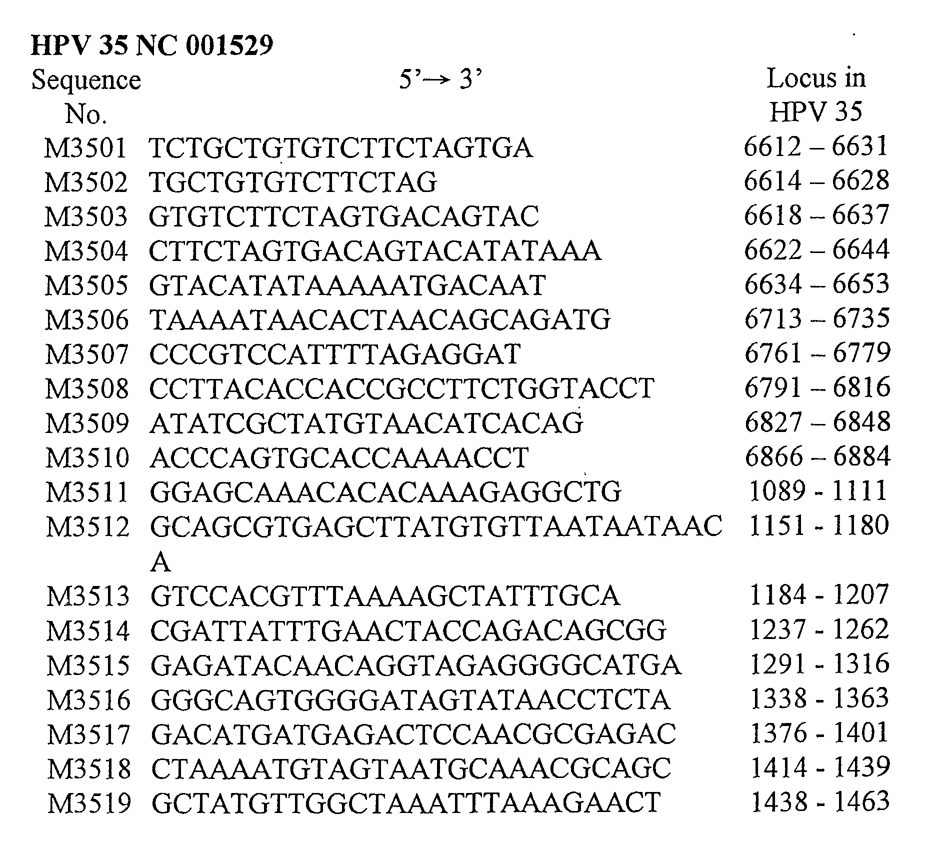 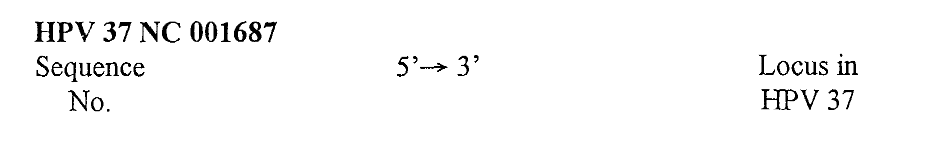 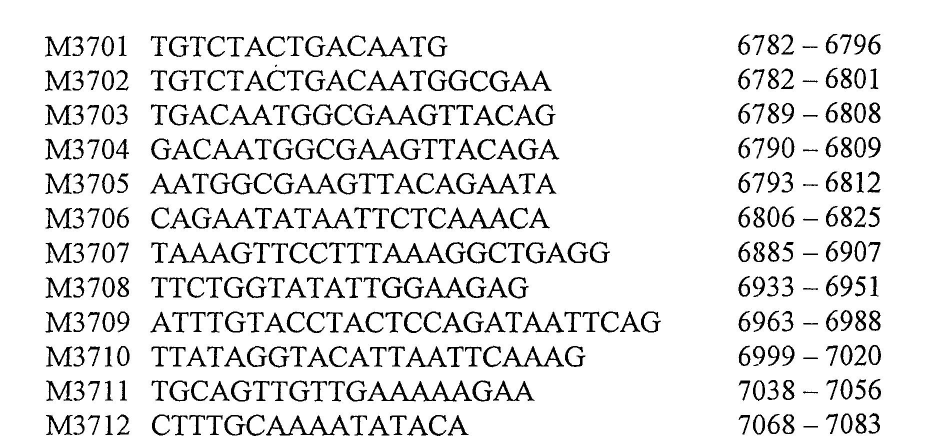 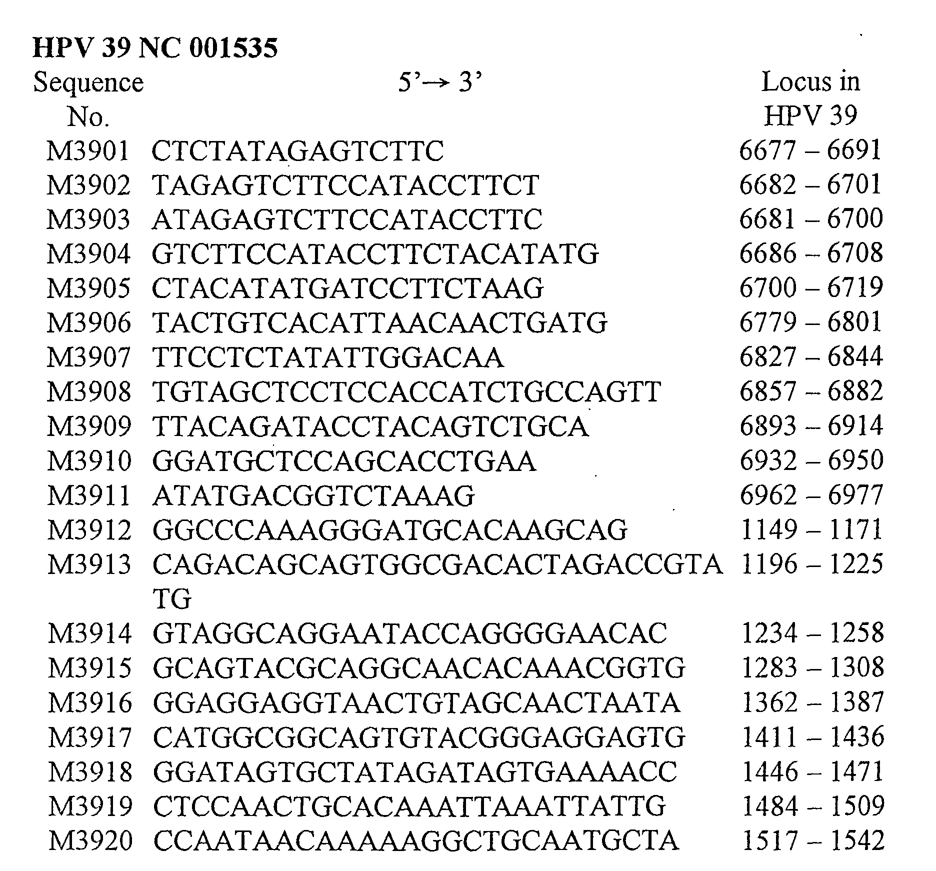 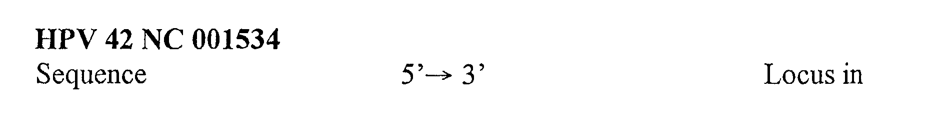 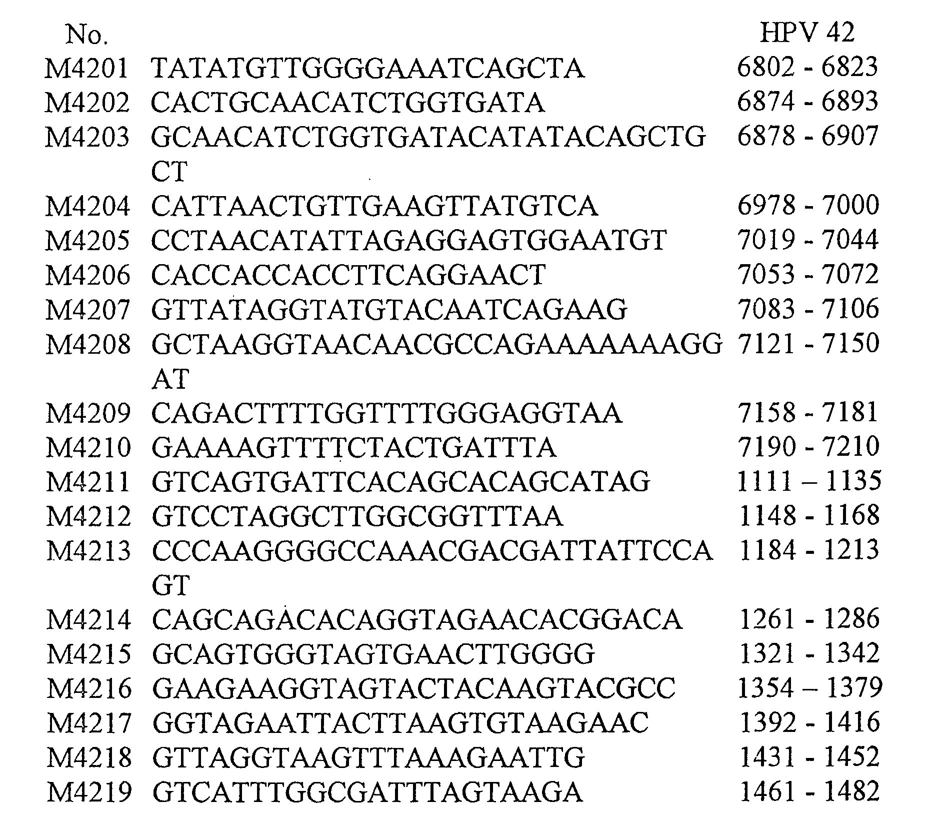 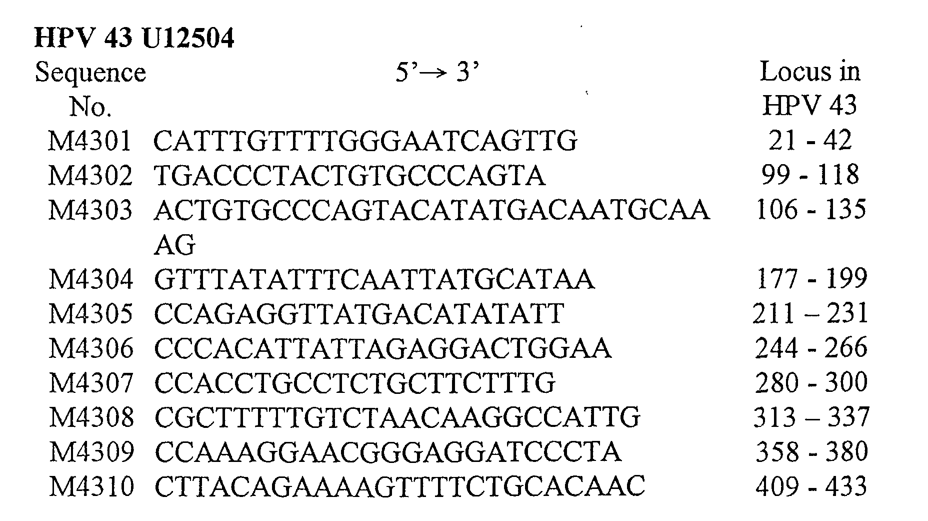 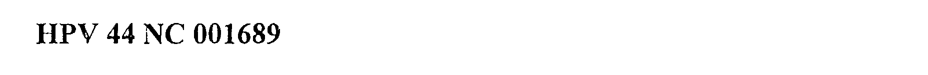 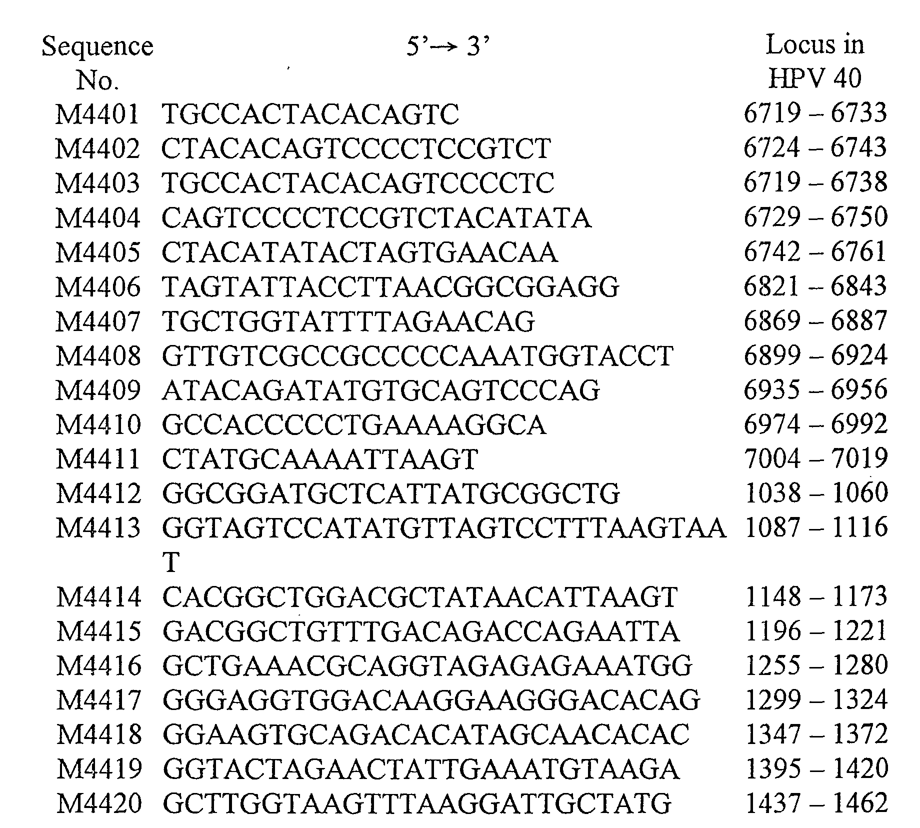 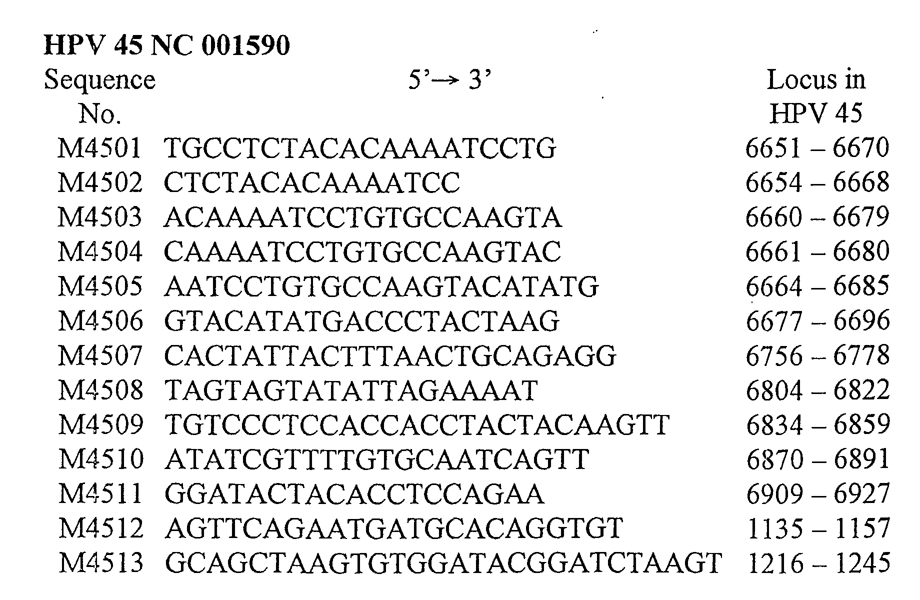 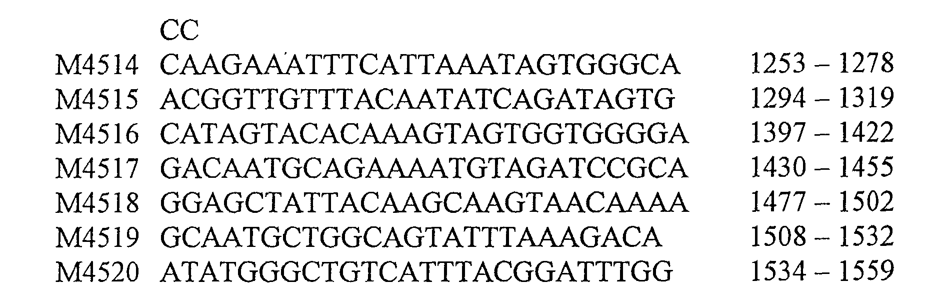 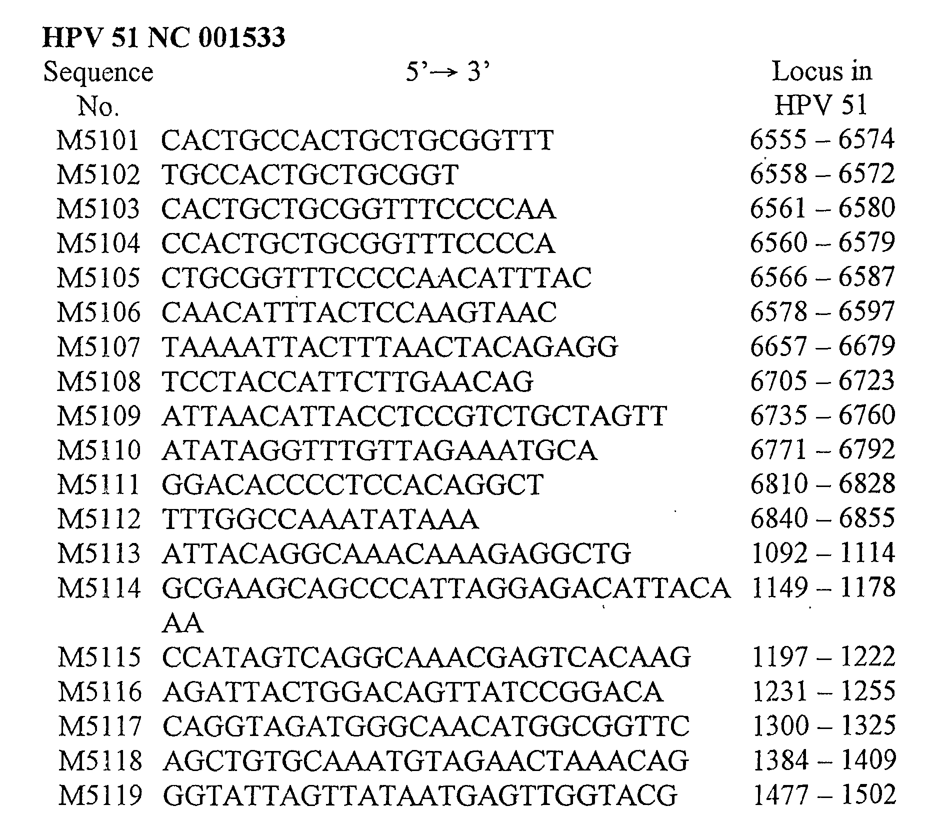 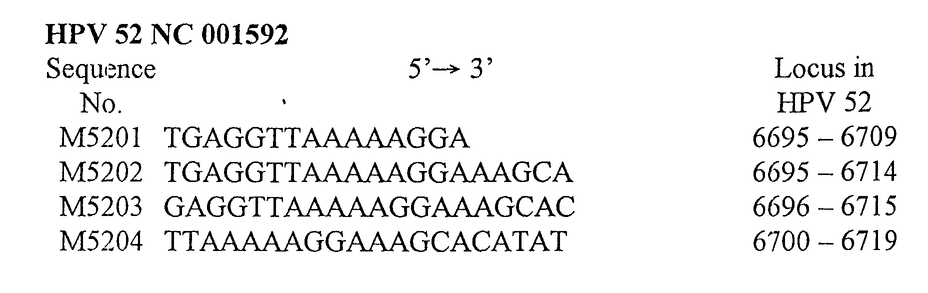 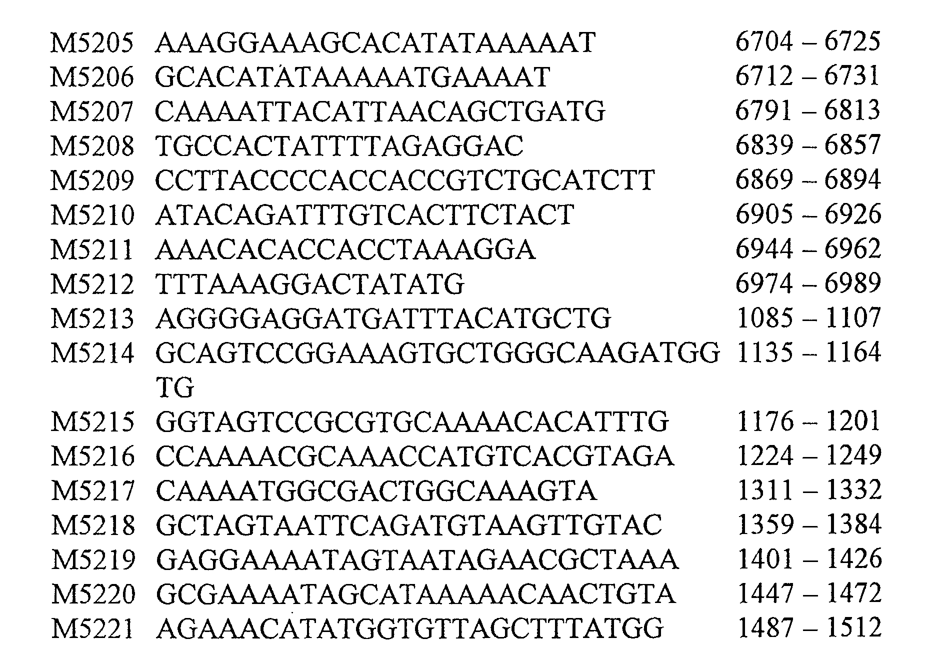 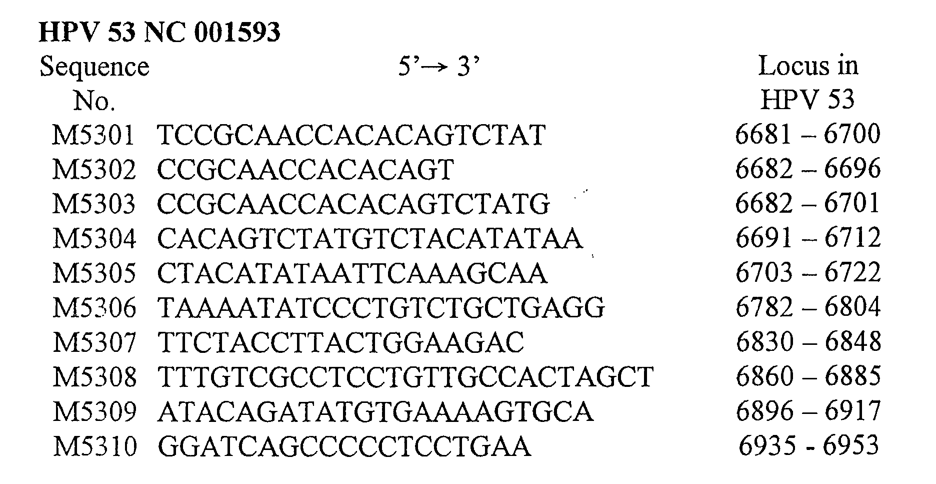 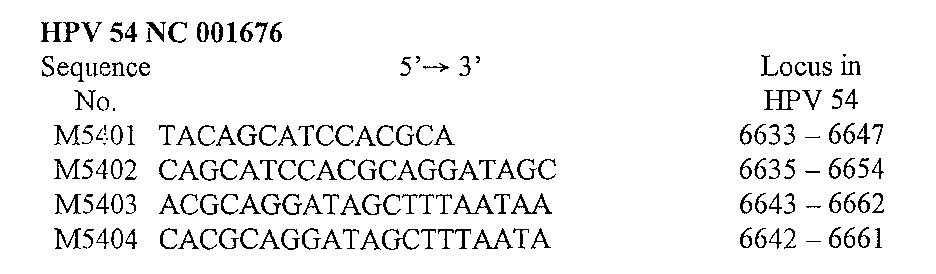 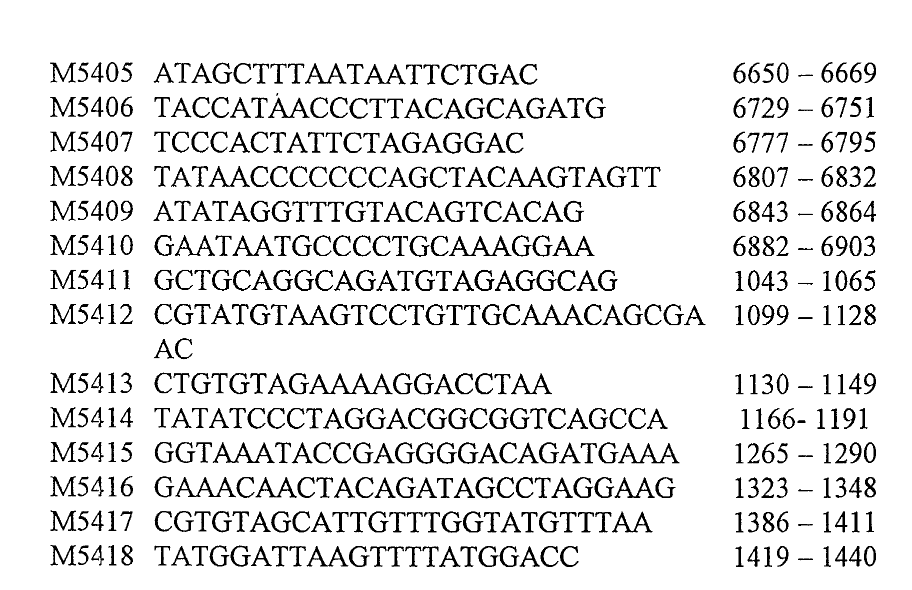 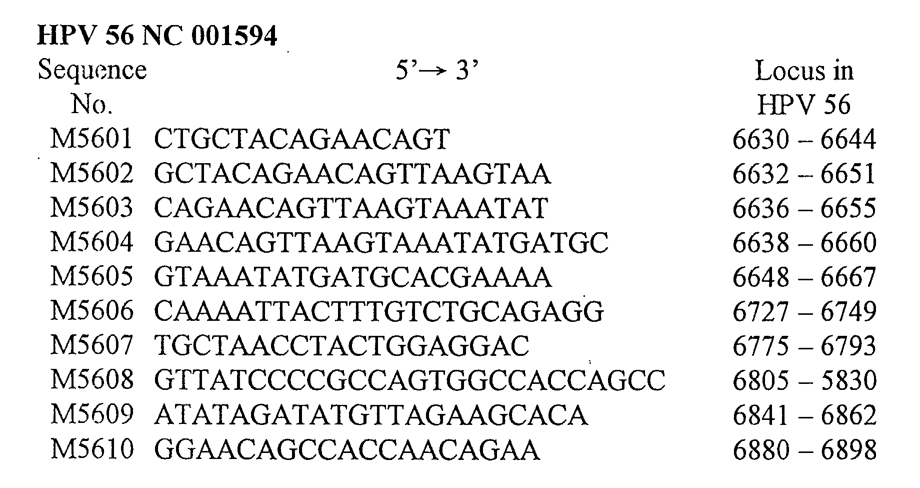 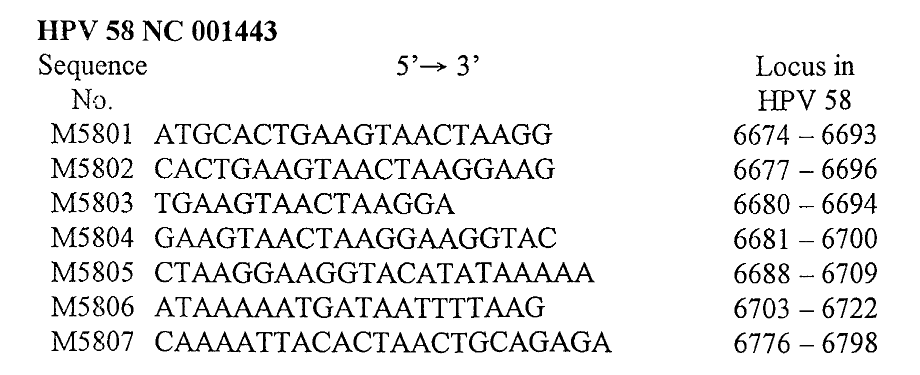 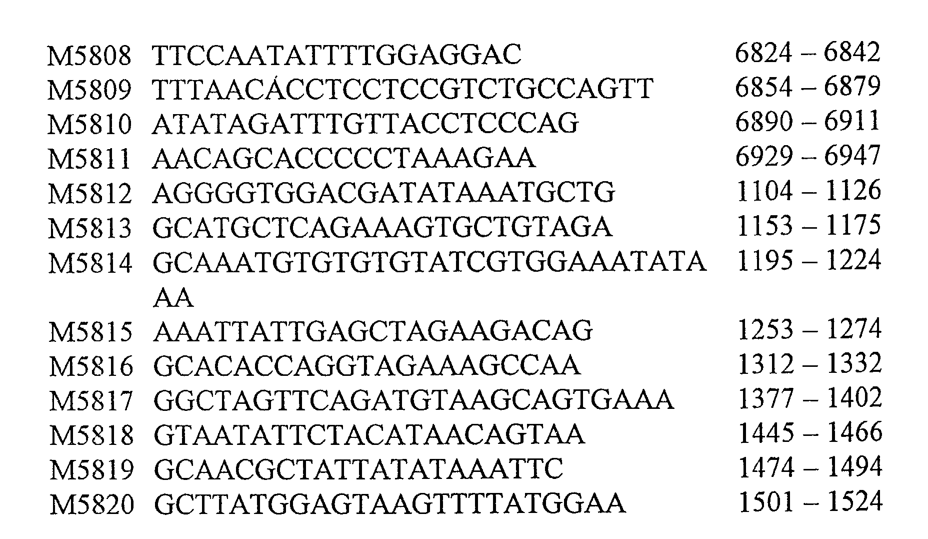 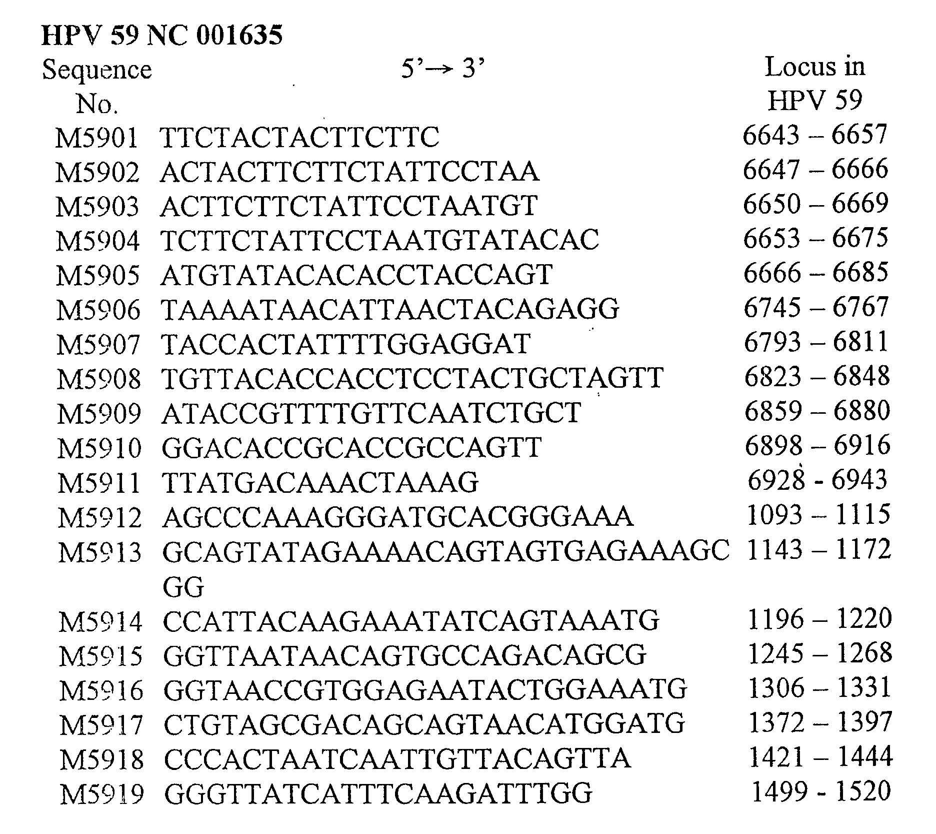 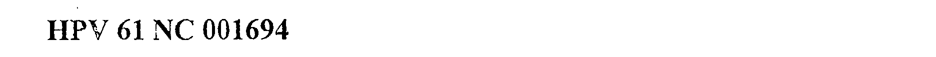 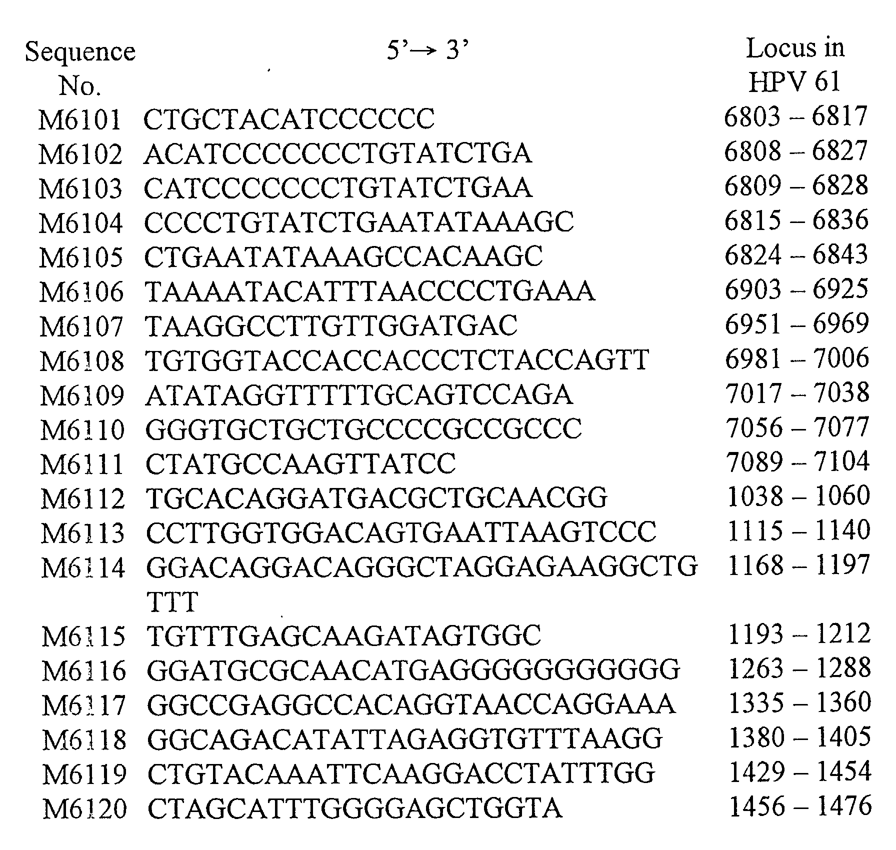 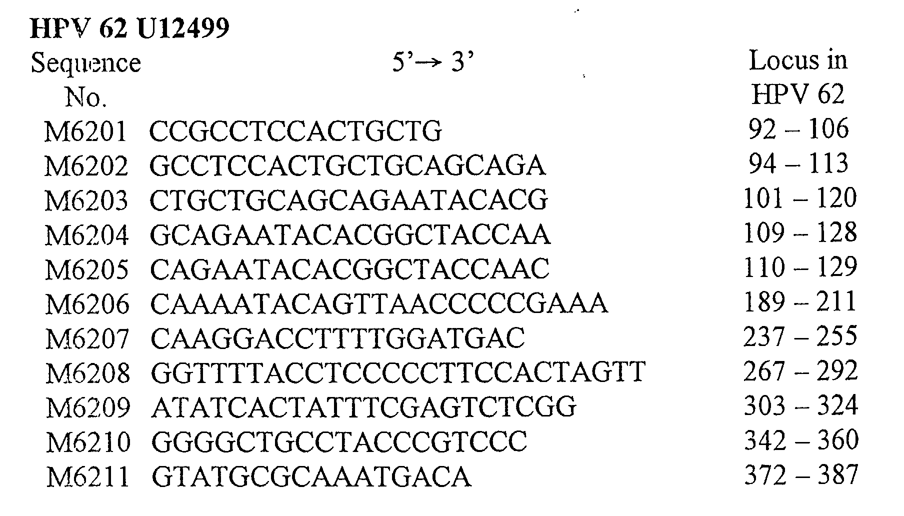 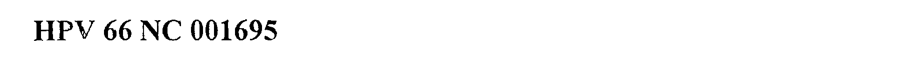 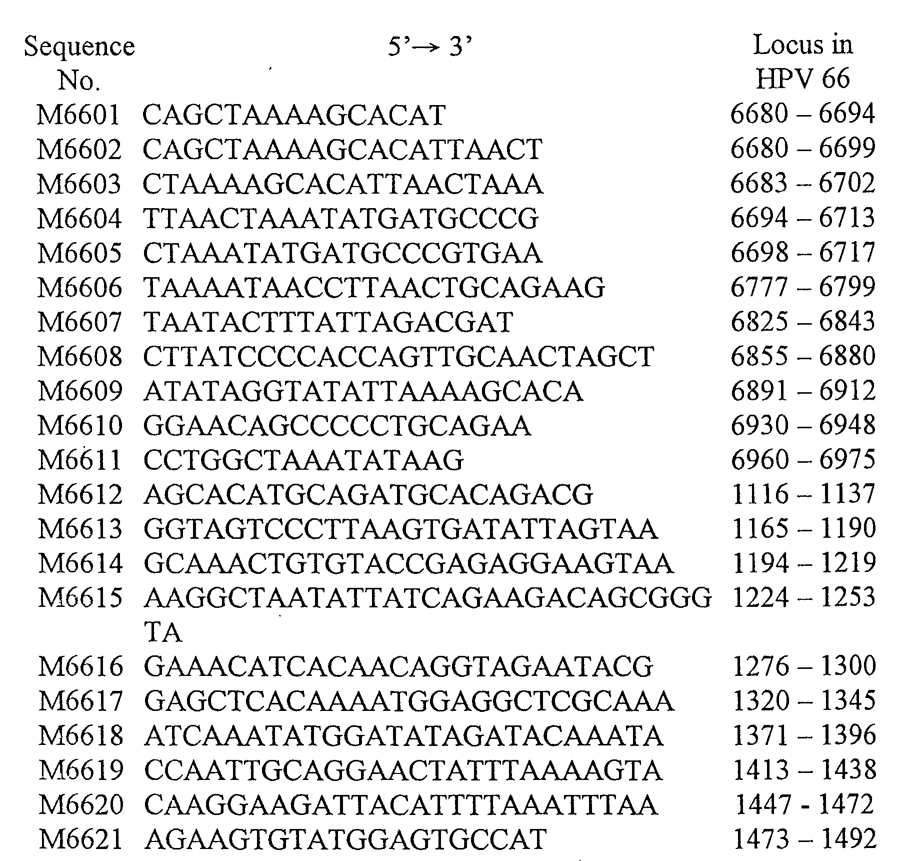 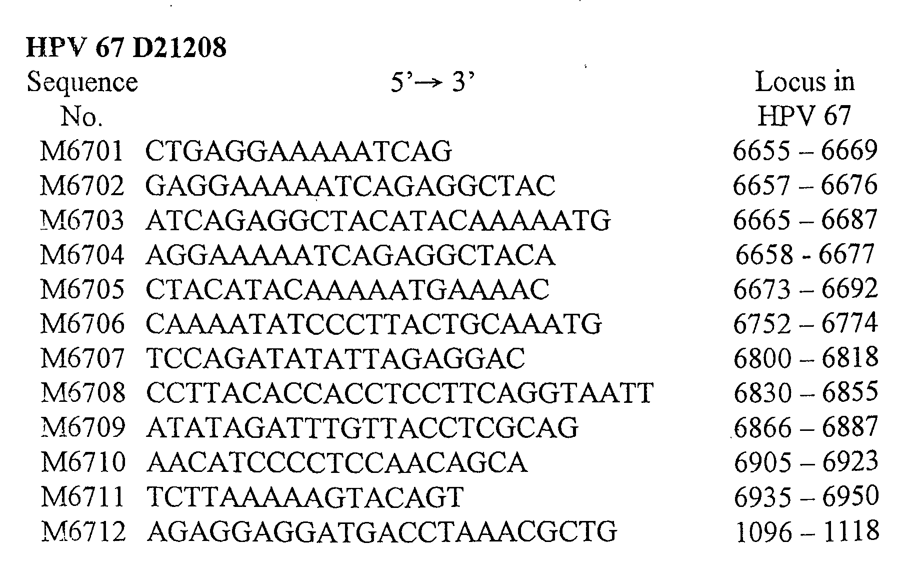 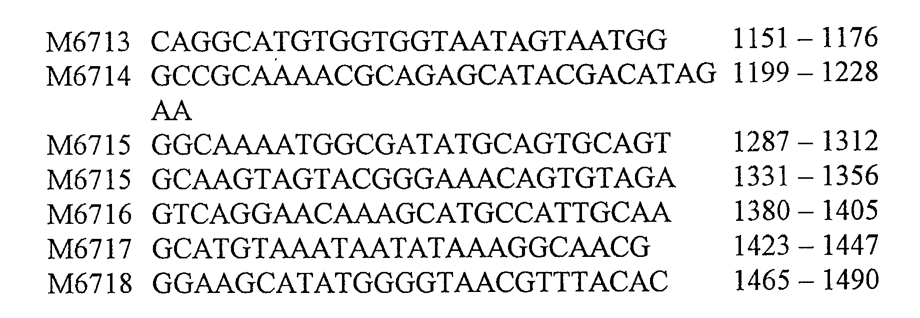 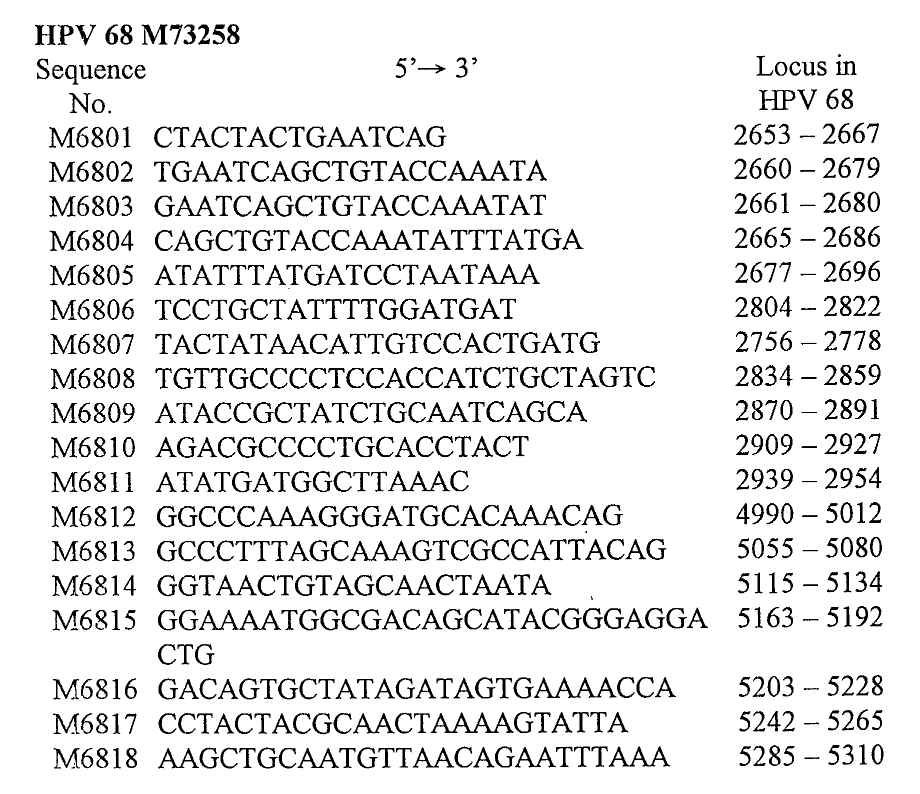 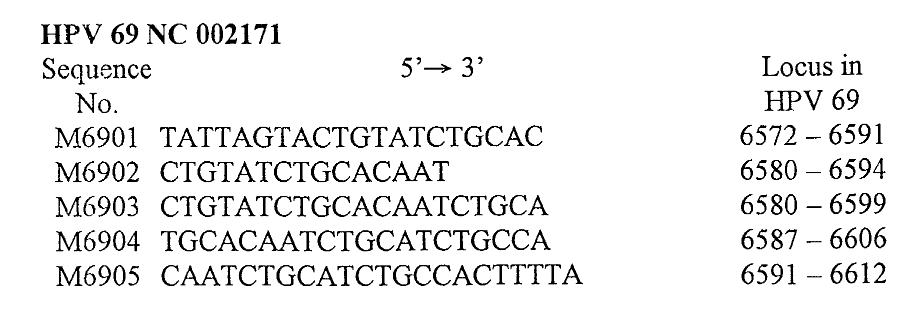 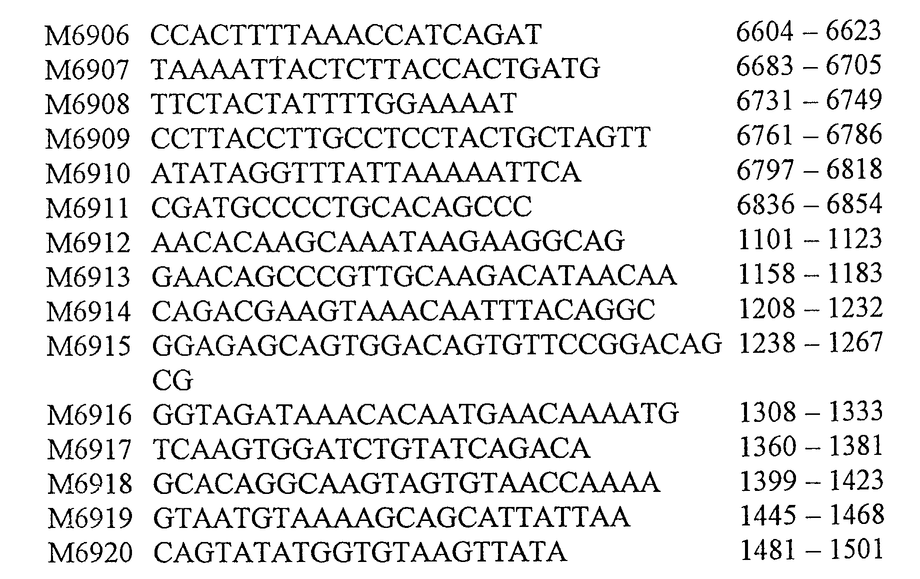 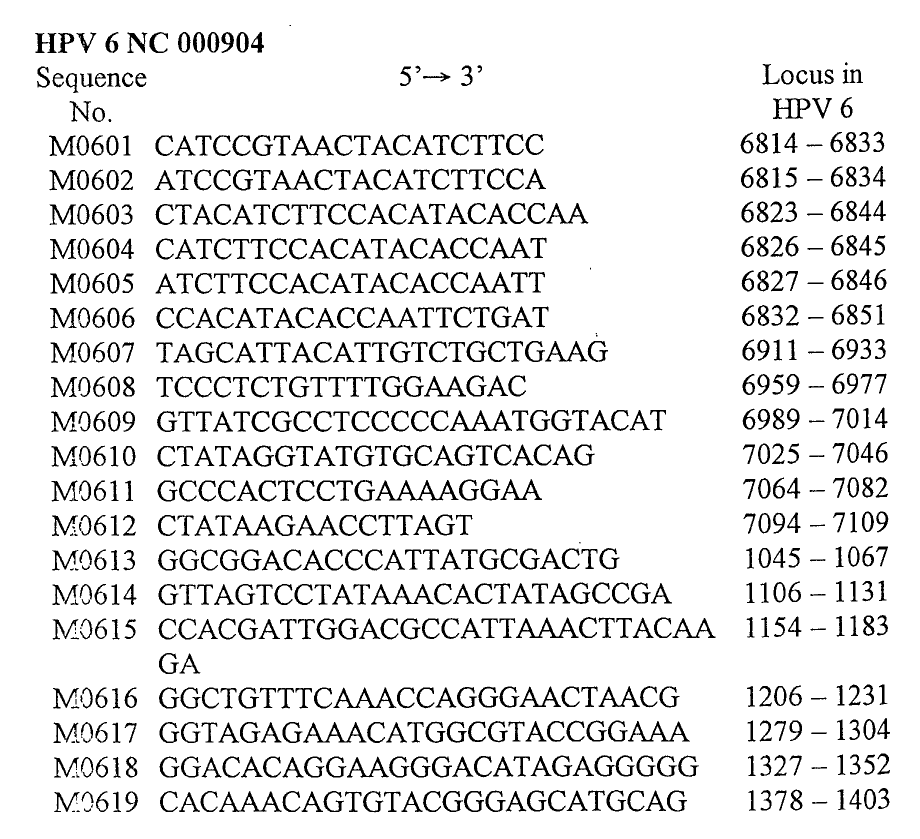 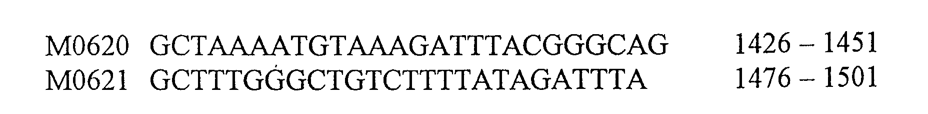 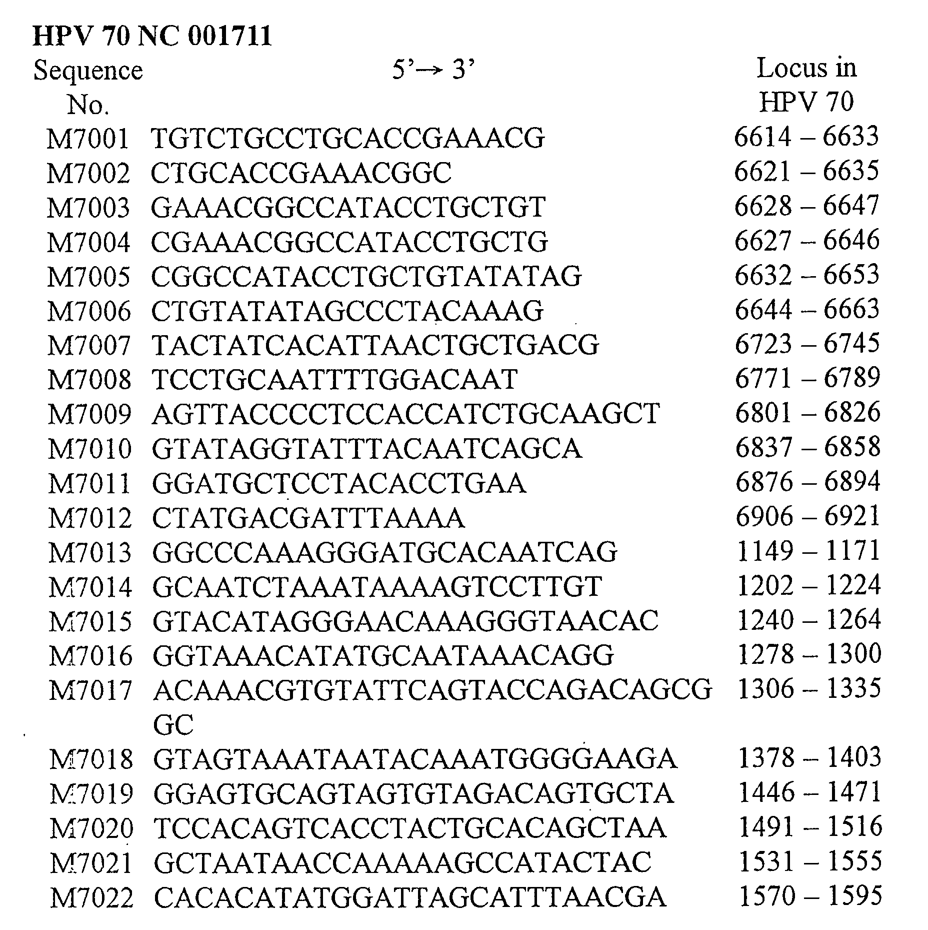 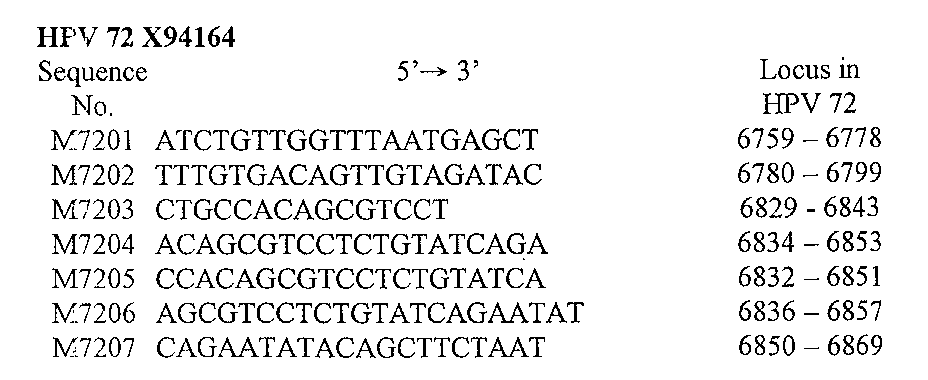 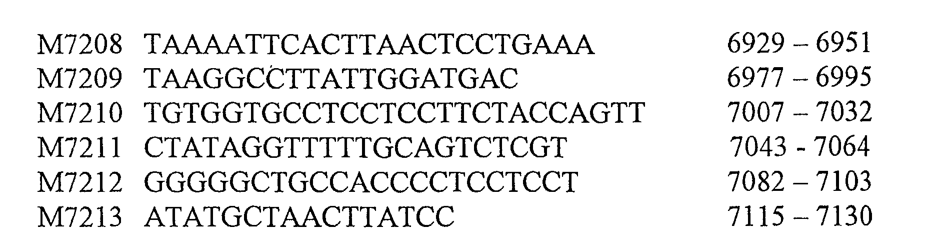 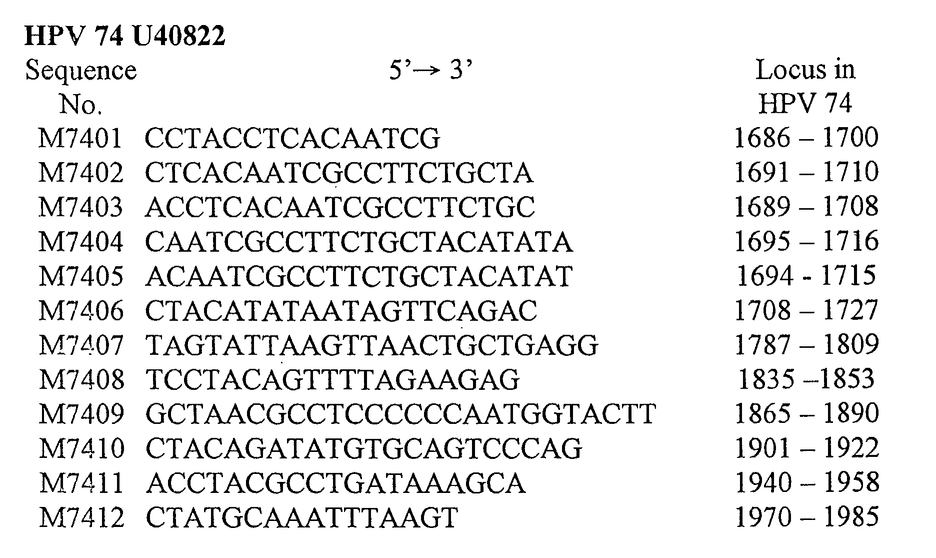 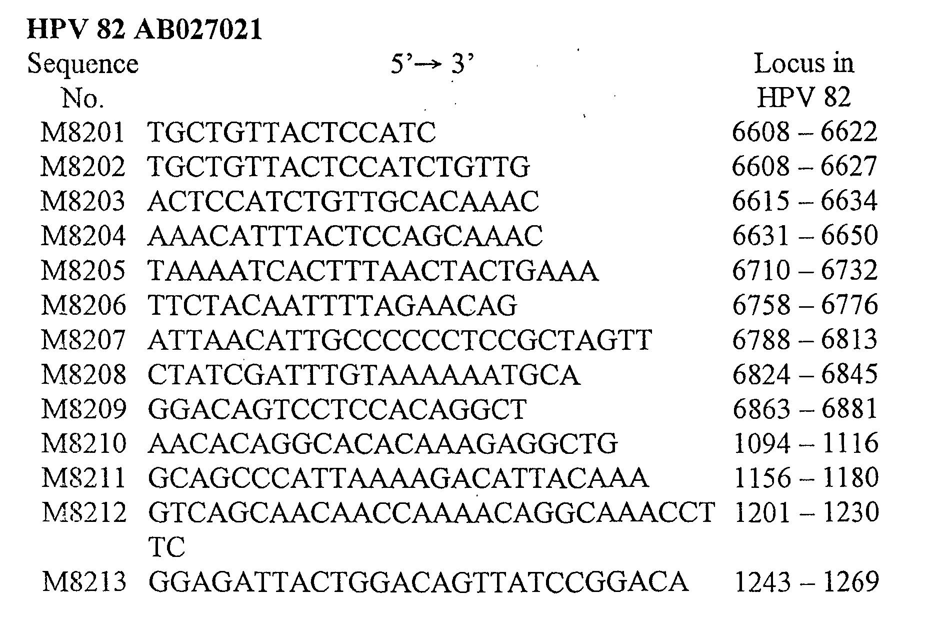 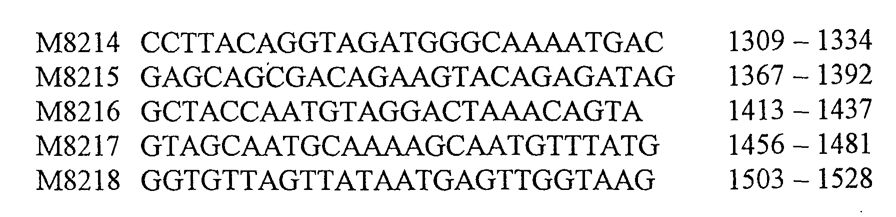 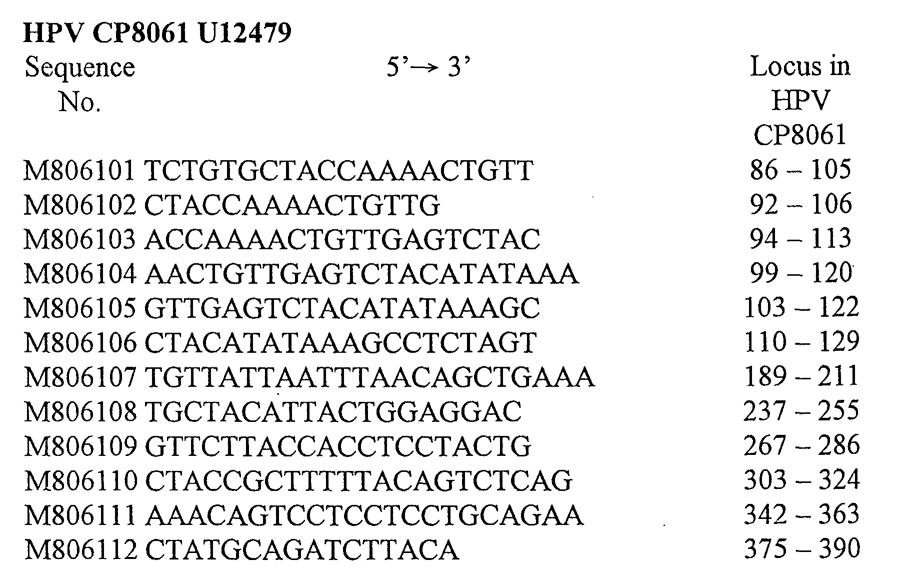 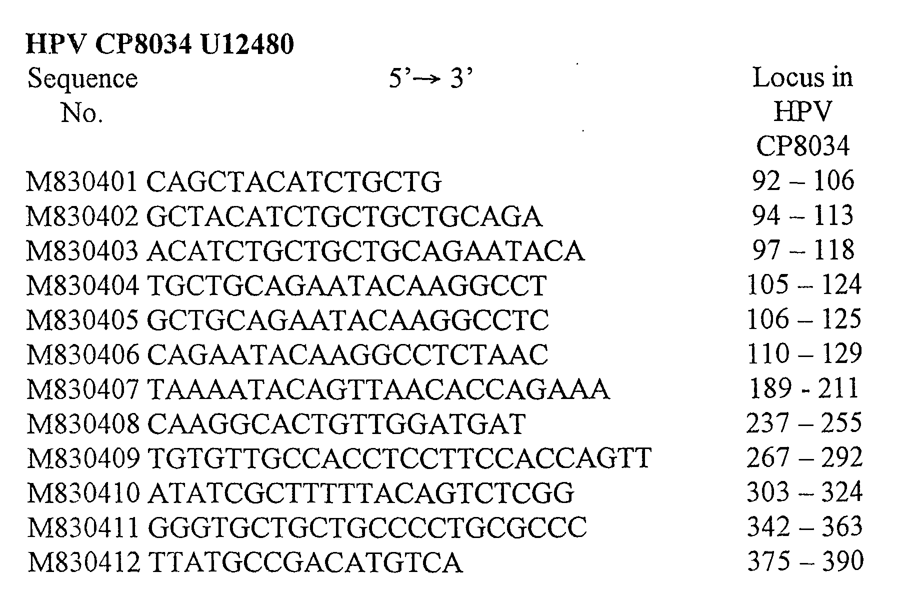 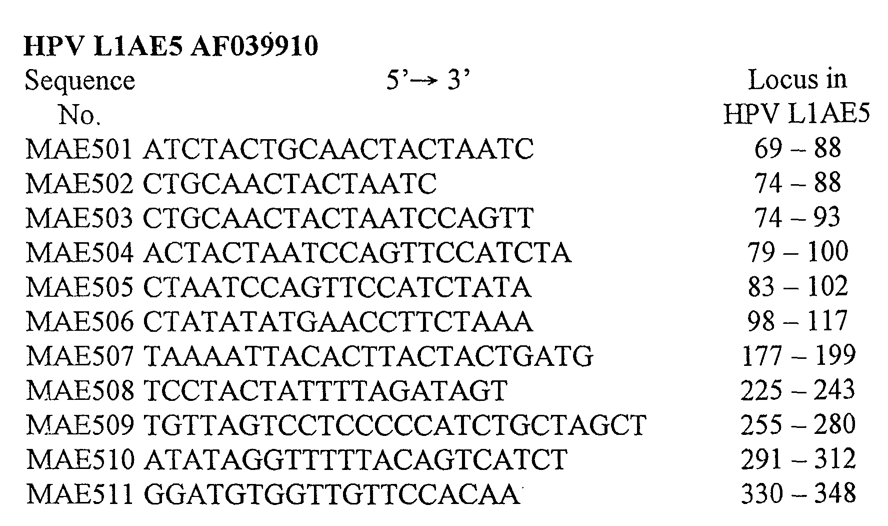 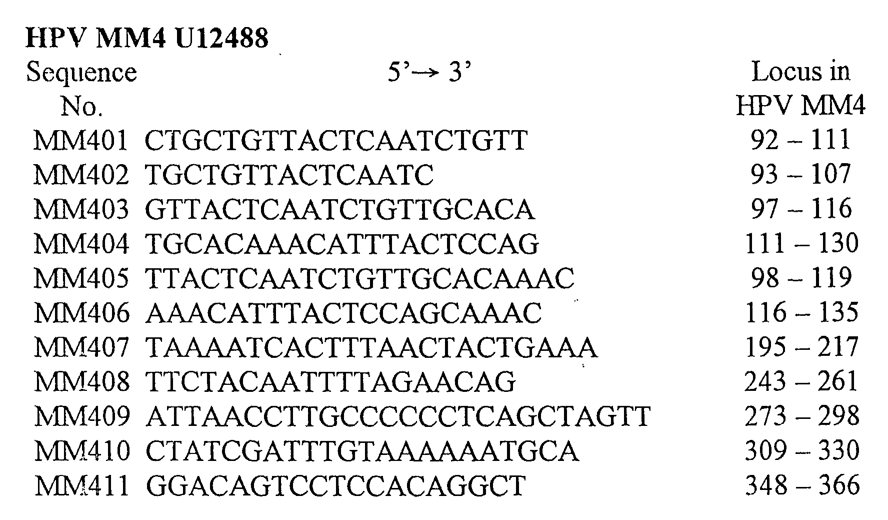 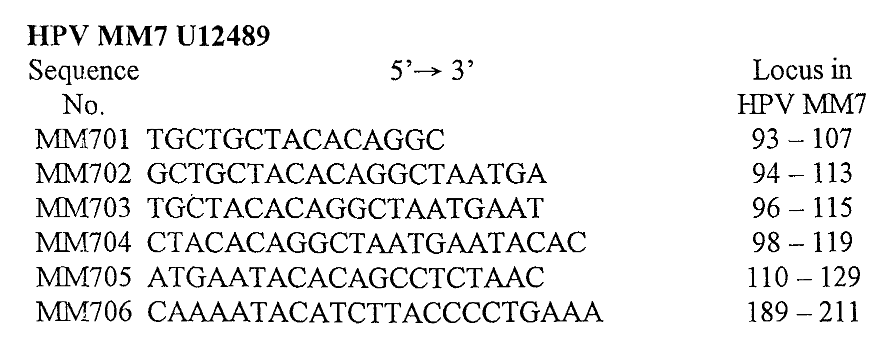 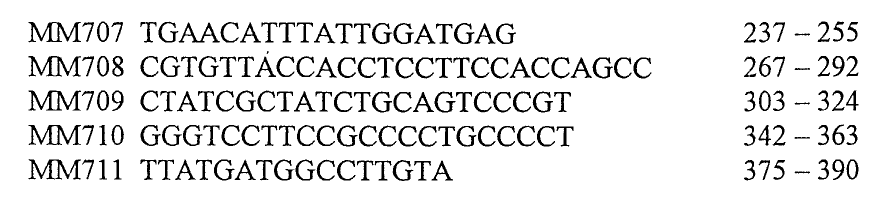 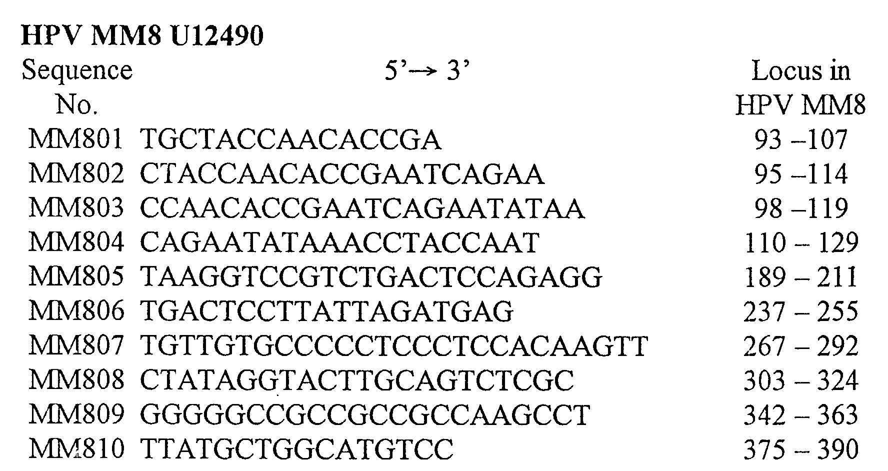 **characterized in that** the sequence of the first oligonucleotide is selected from one of the sequence group corresponding to the first subtype of human papilloma virus and complementary sequences thereof, and the sequence of the second oligonucleotide is selected from one of the sequence group corresponding to the second subtype of human papilloma virus and complementary sequences thereof.

5. The detector (10, 20, 30) according to claim 4, **characterized in that** the detector (10, 20, 30) is an oligonucleotide chip.

6. A method for simultaneously detecting and identifying subtypes of human papilloma viruses contained in a sample, comprising steps of:
providing a first oligonucleotide and a second oligonucleotide for respectively hybridizing with a first and a second subtypes of human papilloma viruses;
hybridizing deoxyribonucleic acid (DNA) contained in the sample with the first and second oligonucleotides; and
removing nonhybridized DNA,
thereby the subtypes of human papilloma viruses contained in the sample are detected and identified.

7. The method according to claim 6, **characterized in that** the DNA contained in the sample is the product of polymerase chain reaction (PCR).

8. The method according to claim 6, **characterized in that** the DNA contained in the sample has signaling substances labeled thereon.

9. The method according to claim 8, **characterized in that** the signaling substance is biotin.

10. The method according to claim 9 **characterized by** comprising a step of performing a reaction of biotin and avidin-alkalinephosphatase.

11. The method according to claim 8, **characterized in that** the signaling substances are fluorescent substances.

12. The method according to claim 11, **characterized in that** the fluorescent substance is Cyanine 5.

13. A method for detecting a subtype of human papilloma viruses contained in a sample, comprising steps of:
providing an oligonucleotide complementary to a sequence specific to the subtype of human papilloma viruses;
hybridizing the oligonucleotide with deoxyribonucleic acid (DNA) contained in the sample;
removing non-hybridized DNA contained in the sample; and
detecting hybridized DNA to show whether the subtype of human papilloma viruses contained in the sample,
**characterized in that** the sequence specific to the subtype of human papilloma viruses is selected from following:
| HPV subtype | Accession number/bp | loci /bp |
|---|---|---|
| HPV 11 | NC 001525/7931 | 6727 - 7135/409 |
| | | 1044 - 1509/466 |
| HPV 16 | NC 001526/7904 | 6602 - 7013/412 |
| | | 1089 - 1538/450 |
| HPV 18 | NC 001357/7857 | 6578 - 6992/415 |
| | | 1135 - 1609/475 |
| HPV 26 | NC 001583/7855 | 6553 - 6967/415 |
| | | 1093 - 1522/430 |
| HPV 31 | NC 001527/7912 | 6520 - 6931/412 |
| | | 1083 - 1476/394 |
| HPV 32 | NC 001586/7961 | 6837 - 7245/409 |
| | | 1032 - 1536/505 |
| HPV 33 | NC 001528/7909 | 6559 - 6967/409 |
| | | 1100 - 1532/433 |
| HPV 35 | NC 001529/7851 | 6542 - 6953/412 |
| | | 1089 - 1497/409 |
| HPV 37 | NC 001687/7421 | 6711 - 7125/415 |
| HPV 39 | NC 001535/7833 | 6605 - 7019/415 |
| | | 1149 - 1593/445 |
| HPV 42 | NC 001534/7917 | 6802-7210/409 |
| | | 1085-1485/401 |
| HPV 43 | U12504/455 | 21-435/415 |
| HPV 44 | NC 001689/7833 | 6647 - 7061/415 |
| | | 1038 - 1491/454 |
| HPV 45 | NC 001590/7858 | 6582 - 6996/415 |
| | | 1136 - 1567/432 |
| HPV 51 | NC 001533/7808 | 6486 - 6897/412 |
| | | 1092 - 1506/415 |
| HPV 52 | NC 001592/7942 | 6623 - 7031/409 |
| | | 1085 - 1526/442 |
| HPV 53 | NC 001593/7856 | 6614 - 7022/409 |
| HPV 54 | NC 001676/7759 | 6561 - 6972/412 |
| | | 1013 - 1484/472 |
| HPV 56 | NC 001594/7844 | 6559 - 6967/409 |
| HPV 58 | NC 001443/7824 | 6608 - 7016/409 |
| | | 1104 - 1536/433 |
| HPV 59 | NC 001635/7896 | 6571 - 6985/415 |
| | | 1093 - 1528/436 |
| HPV 61 | NC 001694/7989 | 6732 - 7146/415 |
| | | 1005 - 1515/511 |
| HPV 62 | U12499/449 | 21 - 429/409 |
| HPV 66 | NC 001695/7824 | 6609 - 7017/409 |
| | | 1023 - 1545/523 |
| HPV 67 | D21208/7801 | 6584 - 6992/409 |
| | | 1096 - 1504/409 |
| HPV 68 | M73258/6042 | 2582 - 2996/415 |
| | | 4990 - 5350/361 |
| HPV 69 | NC 002171/7700 | 6509 - 6923/415 |
| | | 1101 - 1518/418 |
| HPV 6 | NC 000904/8012 | 6743 - 7151/409 |
| | | 1045 - 1510/466 |
| HPV 70 | NC 001711/7905 | 6549 - 6963/415 |
| | | 1149 - 1608/460 |
| HPV 72 | X94164/7988 | 6758 - 7172/415 |
| HPV 74 | U40822/3891 | 1613 - 2027/415 |
| HPV 82 | AB027021/7871 | 6536 - 6950/415 |
| | | 1094 - 1532/439 |
| HPV CP8061 | U12479/452 | 21 - 432/412 |
| HPV CP8304 | U12480/452 | 21 - 432/412 |
| HPV L1AE5 | AF039910/364 | 11 - 360/350 |
| HPV MM4 | U12488/455 | 21 - 435/415 |
| HPV MM7 | U12489/452 | 21 - 432/412 |
| HPV MM8 | U12490/452 | 21 - 432/412 |


SEQUENCE LISTING

<110> King Car Food Industrial Co., Ltd.

<120> Method and Detector for Identifying Subtypes of Human
      Papilloma Viruses

<130> P13603

<140> EP 01 123 379.8
<141> 2002-01-16

<160> 649

<170> PatentIn Ver. 2.1

<210> 1
<211> 15
<212> DNA
<213> Artificial Sequence

<220>
<223> Description of Artificial Sequence:
      Oligonucleotide for Identifying HPV 11

<400> 1
atctgtgtct aaatc                                                  15


<210> 2
<211> 20
<212> DNA
<213> Artificial Sequence

<220>
<223> Description of Artificial Sequence:
      Oligonucleotide for Identifying HPV 11

<400> 2
tctgtgtcta aatctgctac                                             20


<210> 3
<211> 26
<212> DNA
<213> Artificial Sequence

<220>
<223> Description of Artificial Sequence:
      Oligonucleotide for Identifying HPV 11

<400> 3
atctgtgtct aaatctgcta cataca                                      26


<210> 4
<211> 20
<212> DNA
<213> Artificial Sequence

<220>
<223> Description of Artificial Sequence:
      Oligonucleotide for Identifying HPV 11

<400> 4
tgcatctgtg tctaaatctg                                                    20


<210> 5
<211> 20
<212> DNA
<213> Artificial Sequence

<220>
<223> Description of Artificial Sequence:
      Oligonucleotide for Identifying HPV 11

<400> 5
aaatctgcta catacactaa                                                    20


<210> 6
<211> 21
<212> DNA
<213> Artificial Sequence

<220>
<223> Description of Artificial Sequence:
      Oligonucleotide for Identifying HPV 11

<400> 6
ctaaatctgc tacatacact a                                                  21


<210> 7
<211> 20
<212> DNA
<213> Artificial Sequence

<220>
<223> Description of Artificial Sequence:
      Oligonucleotide for Identifying HPV 11

<400> 7
ctacatacac taattcagat                                                    20


<210> 8
<211> 23
<212> DNA
<213> Artificial Sequence

<220>
<223> Description of Artificial Sequence:
      Oligonucleotide for Identifying HPV 11

<400> 8
tagcattaca ttatctgcag aag                                                23


<210> 9
<211> 19
<212> DNA
<213> Artificial Sequence

<220>

<223> Description of Artificial Sequence:
      Oligonucleotide for Identifying HPV 11

<400> 9
tccttctgtt ttggaggac                                                    19


<210> 10
<211> 26
<212> DNA
<213> Artificial Sequence

<220>
<223> Description of Artificial Sequence:
      Oligonucleotide for Identifying HPV 11

<400> 10
tttatcgcct ccaccaaatg gtacac                                            26


<210> 11
<211> 25
<212> DNA
<213> Artificial Sequence

<220>
<223> Description of Artificial Sequence:
      Oligonucleotide for Identifying HPV 11

<400> 11
ttatagatat gtacagtcac aggcc                                             25


<210> 12
<211> 23
<212> DNA
<213> Artificial Sequence

<220>
<223> Description of Artificial Sequence:
      Oligonucleotide for Identifying HPV 11

<400> 12
acccacacct gaaaaagaaa aac                                               23


<210> 13
<211> 23
<212> DNA
<213> Artificial Sequence

<220>
<223> Description of Artificial Sequence:
      Oligonucleotide for Identifying HPV 11

<400> 13
ggcggatgct cattatgcga ctg                                               23


<210> 14
<211> 26
<212> DNA
<213> Artificial Sequence

<220>
<223> Description of Artificial Sequence:
      Oligonucleotide for Identifying HPV 11

<400> 14
atatgtaagt cctataagca atgtag                                          26


<210> 15
<211> 26
<212> DNA
<213> Artificial Sequence

<220>
<223> Description of Artificial Sequence:
      Oligonucleotide for Identifying HPV 11

<400> 15
ctaatgcagt agaaagtgag ataagt                                          26


<210> 16
<211> 26
<212> DNA
<213> Artificial Sequence

<220>
<223> Description of Artificial Sequence:
      Oligonucleotide for Identifying HPV 11

<400> 16
cacggttaga cgccattaaa cttaca                                          26


<210> 17
<211> 26
<212> DNA
<213> Artificial Sequence

<220>
<223> Description of Artificial Sequence:
      Oligonucleotide for Identifying HPV 11

<400> 17
cacagccaaa aaaggtaaag cgacgg                                          26


<210> 18
<211> 26
<212> DNA
<213> Artificial Sequence

<220>
<223> Description of Artificial Sequence:
      Oligonucleotide for Identifying HPV 11

<400> 18
gcaacgcagg tagagaaaca tggcga                                          26


<210> 19
<211> 30

<212> DNA
<213> Artificial Sequence

<220>
<223> Description of Artificial Sequence:
      Oligonucleotide for Identifying HPV 11

<400> 19
gaaaatgggg gagatggtca ggaaagggac                                        30


<210> 20
<211> 26
<212> DNA
<213> Artificial Sequence

<220>
<223> Description of Artificial Sequence:
      Oligonucleotide for Identifying HPV 11

<400> 20
gacacaggga gggacataga gggtga                                            26


<210> 21
<211> 26
<212> DNA
<213> Artificial Sequence

<220>
<223> Description of Artificial Sequence:
      Oligonucleotide for Identifying HPV 11

<400> 21
acatagagag gcggaagcag tagacg                                            26


<210> 22
<211> 26
<212> DNA
<213> Artificial Sequence

<220>
<223> Description of Artificial Sequence:
      Oligonucleotide for Identifying HPV 11

<400> 22
acagcacccg agagcatgca gacaca                                            26


<210> 23
<211> 26
<212> DNA
<213> Artificial Sequence

<220>
<223> Description of Artificial Sequence:
      Oligonucleotide for Identifying HPV 11

<400> 23
tcaggaatat tagaattact aaaatg                                            26

<210> 24
<211> 19
<212> DNA
<213> Artificial Sequence

<220>
<223> Description of Artificial Sequence:
      Oligonucleotide for Identifying HPV 11

<400> 24
gctgtcattt gttgattta                                                    19


<210> 25
<211> 20
<212> DNA
<213> Artificial Sequence

<220>
<223> Description of Artificial Sequence:
      Oligonucleotide for Identifying HPV 16

<400> 25
tatgtcatta tgtgctgcca                                                   20


<210> 26
<211> 20
<212> DNA
<213> Artificial Sequence

<220>
<223> Description of Artificial Sequence:
      Oligonucleotide for Identifying HPV 16

<400> 26
gtgctgccat atctacttca                                                   20


<210> 27
<211> 15
<212> DNA
<213> Artificial Sequence

<220>
<223> Description of Artificial Sequence:
      Oligonucleotide for Identifying HPV 16

<400> 27
tgccatatct acttc                                                        15


<210> 28
<211> 20
<212> DNA
<213> Artificial Sequence

<220>
<223> Description of Artificial Sequence:
      Oligonucleotide for Identifying HPV 16

<400> 28
tatctacttc agaaactaca                                                   20

<210> 29
<211> 22
<212> DNA
<213> Artificial Sequence

<220>
<223> Description of Artificial Sequence:
      Oligonucleotide for Identifying HPV 16

<400> 29
ctacttcaga aactacatat aa                                                22


<210> 30
<211> 20
<212> DNA
<213> Artificial Sequence

<220>
<223> Description of Artificial Sequence:
      Oligonucleotide for Identifying HPV 16

<400> 30
ataaaaatac taactttaag                                                   20


<210> 31
<211> 23
<212> DNA
<213> Artificial Sequence

<220>
<223> Description of Artificial Sequence:
      Oligonucleotide for Identifying HPV 16

<400> 31
caaaataacc ttaactgcag acg                                               23


<210> 32
<211> 19
<212> DNA
<213> Artificial Sequence

<220>
<223> Description of Artificial Sequence:
      Oligonucleotide for Identifying HPV 16

<400> 32
ttccactatt ttggaggac                                                    19


<210> 33
<211> 26
<212> DNA
<213> Artificial Sequence

<220>
<223> Description of Artificial Sequence:
      Oligonucleotide for Identifying HPV 16

<400> 33
tctacaacct cccccaggag gcacac                                          26


<210> 34
<211> 19
<212> DNA
<213> Artificial Sequence

<220>
<223> Description of Artificial Sequence:
      Oligonucleotide for Identifying HPV 16

<400> 34
ttataggttt gtaacccag                                                  19


<210> 35
<211> 19
<212> DNA
<213> Artificial Sequence

<220>
<223> Description of Artificial Sequence:
      Oligonucleotide for Identifying HPV 16

<400> 35
acatacacct ccagcacct                                                  19


<210> 36
<211> 16
<212> DNA
<213> Artificial Sequence

<220>
<223> Description of Artificial Sequence:
      Oligonucleotide for Identifying HPV 16

<400> 36
ccttaaaaaa tacact                                                     16


<210> 37
<211> 23
<212> DNA
<213> Artificial Sequence

<220>
<223> Description of Artificial Sequence:
      Oligonucleotide for Identifying HPV 16

<400> 37
agcaaaacaa catagagatg cag                                             23


<210> 38
<211> 26
<212> DNA
<213> Artificial Sequence

<220>
<223> Description of Artificial Sequence:

Oligonucleotide for Identifying HPV 16

<400> 38
acttagtgat attagtggat gtgtag                                          26


<210> 39
<211> 30
<212> DNA
<213> Artificial Sequence

<220>
<223> Description of Artificial Sequence:
      Oligonucleotide for Identifying HPV 16

<400> 39
tagtcctaga ttaaaagcta tatgtataga                                      30


<210> 40
<211> 26
<212> DNA
<213> Artificial Sequence

<220>
<223> Description of Artificial Sequence:
      Oligonucleotide for Identifying HPV 16

<400> 40
gaaaaacaaa gtagagctgc aaaaag                                          26


<210> 41
<211> 26
<212> DNA
<213> Artificial Sequence

<220>
<223> Description of Artificial Sequence:
      Oligonucleotide for Identifying HPV 16

<400> 41
ctcagcagat gttacaggta gaaggg                                          26


<210> 42
<211> 26
<212> DNA
<213> Artificial Sequence

<220>
<223> Description of Artificial Sequence:
      Oligonucleotide for Identifying HPV 16

<400> 42
ccatgagact gaaacaccat gtagtc                                          26


<210> 43
<211> 26
<212> DNA
<213> Artificial Sequence

<220>
<223> Description of Artificial Sequence:
      Oligonucleotide for Identifying HPV 16

<400> 43
gggggtggtt gcagtcagta cagtag                                          26


<210> 44
<211> 26
<212> DNA
<213> Artificial Sequence

<220>
<223> Description of Artificial Sequence:
      Oligonucleotide for Identifying HPV 16

<400> 44
gtgggggaga gggtgttagt gaaaga                                          26


<210> 45
<211> 26
<212> DNA
<213> Artificial Sequence

<220>
<223> Description of Artificial Sequence:
      Oligonucleotide for Identifying HPV 16

<400> 45
ctatatgcca aacaccactt acaaat                                          26


<210> 46
<211> 26
<212> DNA
<213> Artificial Sequence

<220>
<223> Description of Artificial Sequence:
      Oligonucleotide for Identifying HPV 16

<400> 46
gttatacggg gtgagttttt cagaat                                          26


<210> 47
<211> 15
<212> DNA
<213> Artificial Sequence

<220>
<223> Description of Artificial Sequence:
      Oligonucleotide for Identifying HPV 18

<400> 47
ttctacacag tctcc                                                      15


<210> 48
<211> 20
<212> DNA

<213> Artificial Sequence

<220>
<223> Description of Artificial Sequence:
      Oligonucleotide for Identifying HPV 18

<400> 48
cagtctcctg tacctgggca                                                    20


<210> 49
<211> 20
<212> DNA
<213> Artificial Sequence

<220>
<223> Description of Artificial Sequence:
      Oligonucleotide for Identifying HPV 18

<400> 49
agtctcctgt acctgggcaa                                                    20


<210> 50
<211> 23
<212> DNA
<213> Artificial Sequence

<220>
<223> Description of Artificial Sequence:
      Oligonucleotide for Identifying HPV 18

<400> 50
tctcctgtac ctgggcaata tga                                                23


<210> 51
<211> 20
<212> DNA
<213> Artificial Sequence

<220>
<223> Description of Artificial Sequence:
      Oligonucleotide for Identifying HPV 18

<400> 51
ctgtacctgg gcaatatgat                                                    20


<210> 52
<211> 20
<212> DNA
<213> Artificial Sequence

<220>
<223> Description of Artificial Sequence:
      Oligonucleotide for Identifying HPV 18

<400> 52
atgatgctac caaatttaag                                                    20


<210> 53

<211> 23
<212> DNA
<213> Artificial Sequence

<220>
<223> Description of Artificial Sequence:
      Oligonucleotide for Identifying HPV 18

<400> 53
tactattact ttaactgcag atg                                              23


<210> 54
<211> 19
<212> DNA
<213> Artificial Sequence

<220>
<223> Description of Artificial Sequence:
      Oligonucleotide for Identifying HPV 18

<400> 54
tagcagtatt ttagaggat                                                   19


<210> 55
<211> 26
<212> DNA
<213> Artificial Sequence

<220>
<223> Description of Artificial Sequence:
      Oligonucleotide for Identifying HPV 18

<400> 55
tgttcccccc cccccaacta ctagtt                                           26


<210> 56
<211> 22
<212> DNA
<213> Artificial Sequence

<220>
<223> Description of Artificial Sequence:
      Oligonucleotide for Identifying HPV 18

<400> 56
atatcgtttt gtacaatctg tt                                               22


<210> 57
<211> 19
<212> DNA
<213> Artificial Sequence

<220>
<223> Description of Artificial Sequence:
      Oligonucleotide for Identifying HPV 18

<400> 57
ggatgctgca ccggctgaa                                                   19

<210> 58
<211> 16
<212> DNA
<213> Artificial Sequence

<220>
<223> Description of Artificial Sequence:
      Oligonucleotide for Identifying HPV 18

<400> 58
ctatgataag ttaaag                                                    16


<210> 59
<211> 23
<212> DNA
<213> Artificial Sequence

<220>
<223> Description of Artificial Sequence:
      Oligonucleotide for Identifying HPV 18

<400> 59
ggtccacaat gatgcacaag tgt                                            23


<210> 60
<211> 26
<212> DNA
<213> Artificial Sequence

<220>
<223> Description of Artificial Sequence:
      Oligonucleotide for Identifying HPV 18

<400> 60
cacagaaaac agtccattag gggagc                                         26


<210> 61
<211> 26
<212> DNA
<213> Artificial Sequence

<220>
<223> Description of Artificial Sequence:
      Oligonucleotide for Identifying HPV 18

<400> 61
gctggaggtg gatacagagt taagtc                                         26


<210> 62
<211> 30
<212> DNA
<213> Artificial Sequence

<220>
<223> Description of Artificial Sequence:
      Oligonucleotide for Identifying HPV 18

<400> 62

agtgggcaga aaaaggcaaa aaggcggctg                                    30

<210> 63
<211> 26
<212> DNA
<213> Artificial Sequence

<220>
<223> Description of Artificial Sequence:
      Oligonucleotide for Identifying HPV 18

<400> 63
cacagattca ggtaactaca aatggc                                          26

<210> 64
<211> 26
<212> DNA
<213> Artificial Sequence

<220>
<223> Description of Artificial Sequence:
      Oligonucleotide for Identifying HPV 18

<400> 64
caatgtatgt agtggcggca gtacgg                                          26

<210> 65
<211> 26
<212> DNA
<213> Artificial Sequence

<220>
<223> Description of Artificial Sequence:
      Oligonucleotide for Identifying HPV 18

<400> 65
gacaacgggg gcacagaggg caacaa                                          26

<210> 66
<211> 26
<212> DNA
<213> Artificial Sequence

<220>
<223> Description of Artificial Sequence:
      Oligonucleotide for Identifying HPV 18

<400> 66
gtagacggta caagtgacaa tagcaa                                          26

<210> 67
<211> 26
<212> DNA
<213> Artificial Sequence

<220>
<223> Description of Artificial Sequence:
      Oligonucleotide for Identifying HPV 18

<400> 67
ccacaatgta ccatagcaca attaaa                                          26

<210> 68
<211> 26
<212> DNA
<213> Artificial Sequence

<220>
<223> Description of Artificial Sequence:
      Oligonucleotide for Identifying HPV 18

<400> 68
cacatatggg ctatcattta cagatt                                          26

<210> 69
<211> 20
<212> DNA
<213> Artificial Sequence

<220>
<223> Description of Artificial Sequence:
      Oligonucleotide for Identifying HPV 26

<400> 69
tagtacatta tctgcagcat                                                 20

<210> 70
<211> 15
<212> DNA
<213> Artificial Sequence

<220>
<223> Description of Artificial Sequence:
      Oligonucleotide for Identifying HPV 26

<400> 70
attatctgca gcatc                                                      15

<210> 71
<211> 20
<212> DNA
<213> Artificial Sequence

<220>
<223> Description of Artificial Sequence:
      Oligonucleotide for Identifying HPV 26

<400> 71
tgcagcatct gcatccactc                                                 20

<210> 72
<211> 24
<212> DNA
<213> Artificial Sequence

<220>

<223> Description of Artificial Sequence:
      Oligonucleotide for Identifying HPV 26

<400> 72
gcatctgcat ccactccatt taaa                                          24


<210> 73
<211> 20
<212> DNA
<213> Artificial Sequence

<220>
<223> Description of Artificial Sequence:
      Oligonucleotide for Identifying HPV 26

<400> 73
ctccatttaa accatctgat                                               20


<210> 74
<211> 23
<212> DNA
<213> Artificial Sequence

<220>
<223> Description of Artificial Sequence:
      Oligonucleotide for Identifying HPV 26

<400> 74
taaaataaca cttacaacag atg                                           23


<210> 75
<211> 19
<212> DNA
<213> Artificial Sequence

<220>
<223> Description of Artificial Sequence:
      Oligonucleotide for Identifying HPV 26

<400> 75
tgcctccata ttggaggat                                                19


<210> 76
<211> 26
<212> DNA
<213> Artificial Sequence

<220>
<223> Description of Artificial Sequence:
      Oligonucleotide for Identifying HPV 26

<400> 76
actaacctta cctcccactg ctagtt                                        26


<210> 77
<211> 22
<212> DNA
<213> Artificial Sequence

<220>
<223> Description of Artificial Sequence:
      Oligonucleotide for Identifying HPV 26

<400> 77
ctataggttt attaaaaact ct                                                22


<210> 78
<211> 19
<212> DNA
<213> Artificial Sequence

<220>
<223> Description of Artificial Sequence:
      Oligonucleotide for Identifying HPV 26

<400> 78
taacgcccct cctgtgcca                                                    19


<210> 79
<211> 23
<212> DNA
<213> Artificial Sequence

<220>
<223> Description of Artificial Sequence:
      Oligonucleotide for Identifying HPV 26

<400> 79
aaaacaggca aatacaaagg cag                                               23


<210> 80
<211> 26
<212> DNA
<213> Artificial Sequence

<220>
<223> Description of Artificial Sequence:
      Oligonucleotide for Identifying HPV 26

<400> 80
ctaggtagtc agaacagccc gttgca                                            26


<210> 81
<211> 30
<212> DNA
<213> Artificial Sequence

<220>
<223> Description of Artificial Sequence:
      Oligonucleotide for Identifying HPV 26

<400> 81
gcgacagtca gcagaataca caccaagtaa                                        30


<210> 82
<211> 26

<212> DNA
<213> Artificial Sequence

<220>
<223> Description of Artificial Sequence:
      Oligonucleotide for Identifying HPV 26

<400> 82
caaaaggaga gccgtggaca gtgtac                                          26


<210> 83
<211> 26
<212> DNA
<213> Artificial Sequence

<220>
<223> Description of Artificial Sequence:
      Oligonucleotide for Identifying HPV 26

<400> 83
ccgtacaggt agataaacaa tatgaa                                          26


<210> 84
<211> 25
<212> DNA
<213> Artificial Sequence

<220>
<223> Description of Artificial Sequence:
      Oligonucleotide for Identifying HPV 26

<400> 84
gcctagtgtg tgtagtcagg ggggg                                           25


<210> 85
<211> 26
<212> DNA
<213> Artificial Sequence

<220>
<223> Description of Artificial Sequence:
      Oligonucleotide for Identifying HPV 26

<400> 85
gcctcagtgg aagatatcga tgtaga                                          26


<210> 86
<211> 26
<212> DNA
<213> Artificial Sequence

<220>
<223> Description of Artificial Sequence:
      Oligonucleotide for Identifying HPV 26

<400> 86
cagtgttaca caaatatgtg aattat                                          26

<210> 87
<211> 26
<212> DNA
<213> Artificial Sequence

<220>
<223> Description of Artificial Sequence:
      Oligonucleotide for Identifying HPV 26

<400> 87
cagtatatgg tgtaagtttt gcagaa                                          26


<210> 88
<211> 15
<212> DNA
<213> Artificial Sequence

<220>
<223> Description of Artificial Sequence:
      Oligonucleotide for Identifying HPV 31

<400> 88
tgcaattgca aacag                                                      15


<210> 89
<211> 20
<212> DNA
<213> Artificial Sequence

<220>
<223> Description of Artificial Sequence:
      Oligonucleotide for Identifying HPV 31

<400> 89
gcaattgcaa acagtgatac                                                 20


<210> 90
<211> 20
<212> DNA
<213> Artificial Sequence

<220>
<223> Description of Artificial Sequence:
      Oligonucleotide for Identifying HPV 31

<400> 90
caattgcaaa cagtgatact                                                 20


<210> 91
<211> 23
<212> DNA
<213> Artificial Sequence

<220>
<223> Description of Artificial Sequence:
      Oligonucleotide for Identifying HPV 31

<400> 91
gcaaacagtg atactacatt taa                                             23

<210> 92
<211> 20
<212> DNA
<213> Artificial Sequence

<220>
<223> Description of Artificial Sequence:
      Oligonucleotide for Identifying HPV 31

<400> 92
ctacatttaa aagtagtaat                                                    20


<210> 93
<211> 23
<212> DNA
<213> Artificial Sequence

<220>
<223> Description of Artificial Sequence:
      Oligonucleotide for Identifying HPV 31

<400> 93
caaaataaca ttatctgcag aca                                                23


<210> 94
<211> 19
<212> DNA
<213> Artificial Sequence

<220>
<223> Description of Artificial Sequence:
      Oligonucleotide for Identifying HPV 31

<400> 94
tcctgctatt ttggaagat                                                     19


<210> 95
<211> 26
<212> DNA
<213> Artificial Sequence

<220>
<223> Description of Artificial Sequence:
      Oligonucleotide for Identifying HPV 31

<400> 95
attgaccaca cctccctcag gttctt                                             26


<210> 96
<211> 22
<212> DNA
<213> Artificial Sequence

<220>
<223> Description of Artificial Sequence:
      Oligonucleotide for Identifying HPV 31

<400> 96
ctataggttt gtcacctcac ag                                              22


<210> 97
<211> 19
<212> DNA
<213> Artificial Sequence

<220>
<223> Description of Artificial Sequence:
      Oligonucleotide for Identifying HPV 31

<400> 97
aactgccccc caaaagccc                                                  19


<210> 98
<211> 23
<212> DNA
<213> Artificial Sequence

<220>
<223> Description of Artificial Sequence:
      Oligonucleotide for Identifying HPV 31

<400> 98
agcggaggaa catgcagagg ctg                                             23


<210> 99
<211> 26
<212> DNA
<213> Artificial Sequence

<220>
<223> Description of Artificial Sequence:
      Oligonucleotide for Identifying HPV 31

<400> 99
tttaagtgat attagtagtt gtgtgg                                          26


<210> 100
<211> 30
<212> DNA
<213> Artificial Sequence

<220>
<223> Description of Artificial Sequence:
      Oligonucleotide for Identifying HPV 31

<400> 100
agtccacggt taaaagctat atgcatagaa                                      30


<210> 101
<211> 26
<212> DNA
<213> Artificial Sequence

<220>
<223> Description of Artificial Sequence:

Oligonucleotide for Identifying HPV 31

<400> 101
gactctttga acttccagac agcggg                                          26


<210> 102
<211> 26
<212> DNA
<213> Artificial Sequence

<220>
<223> Description of Artificial Sequence:
      Oligonucleotide for Identifying HPV 31

<400> 102
gcagcagatg gtacaggtag aggagc                                          26


<210> 103
<211> 26
<212> DNA
<213> Artificial Sequence

<220>
<223> Description of Artificial Sequence:
      Oligonucleotide for Identifying HPV 31

<400> 103
aatggtagtg acgggacaca tagtga                                          26


<210> 104
<211> 26
<212> DNA
<213> Artificial Sequence

<220>
<223> Description of Artificial Sequence:
      Oligonucleotide for Identifying HPV 31

<400> 104
ccaacacgta atatattgca agtgtt                                          26


<210> 105
<211> 26
<212> DNA
<213> Artificial Sequence

<220>
<223> Description of Artificial Sequence:
      Oligonucleotide for Identifying HPV 31

<400> 105
gcaatggtaa agctgctatg ttaggt                                          26


<210> 106
<211> 25
<212> DNA
<213> Artificial Sequence

<220>
<223> Description of Artificial Sequence:
      Oligonucleotide for Identifying HPV 31

<400> 106
ttatatggtg taagttttat ggaac                                          25


<210> 107
<211> 20
<212> DNA
<213> Artificial Sequence

<220>
<223> Description of Artificial Sequence:
      Oligonucleotide for Identifying HPV 32

<400> 107
tgctactgta acaactgaag                                                20


<210> 108
<211> 20
<212> DNA
<213> Artificial Sequence

<220>
<223> Description of Artificial Sequence:
      Oligonucleotide for Identifying HPV 32

<400> 108
gctactgtaa caactgaaga                                                20


<210> 109
<211> 15
<212> DNA
<213> Artificial Sequence

<220>
<223> Description of Artificial Sequence:
      Oligonucleotide for Identifying HPV 32

<400> 109
tactgtaaca actga                                                     15


<210> 110
<211> 20
<212> DNA
<213> Artificial Sequence

<220>
<223> Description of Artificial Sequence:
      Oligonucleotide for Identifying HPV 32

<400> 110
actgtaacaa ctgaagacac                                                20


<210> 111
<211> 22
<212> DNA

<213> Artificial Sequence

<220>
<223> Description of Artificial Sequence:
      Oligonucleotide for Identifying HPV 32

<400> 111
caactgaaga cacatacaag tc                                                22


<210> 112
<211> 23
<212> DNA
<213> Artificial Sequence

<220>
<223> Description of Artificial Sequence:
      Oligonucleotide for Identifying HPV 32

<400> 112
caaaattaca ttatctgtag agg                                               23


<210> 113
<211> 19
<212> DNA
<213> Artificial Sequence

<220>
<223> Description of Artificial Sequence:
      Oligonucleotide for Identifying HPV 32

<400> 113
tcctgacata ctagacgat                                                    19


<210> 114
<211> 26
<212> DNA
<213> Artificial Sequence

<220>
<223> Description of Artificial Sequence:
      Oligonucleotide for Identifying HPV 32

<400> 114
tgtagctcca ccgccctctg gtactt                                            26


<210> 115
<211> 22
<212> DNA
<213> Artificial Sequence

<220>
<223> Description of Artificial Sequence:
      Oligonucleotide for Identifying HPV 32

<400> 115
ttatagattt gtgcagtctc ag                                                22


<210> 116

<211> 19
<212> DNA
<213> Artificial Sequence

<220>
<223> Description of Artificial Sequence:
      Oligonucleotide for Identifying HPV 32

<400> 116
taaggtaaca gcacctgaa                                                    19


<210> 117
<211> 16
<212> DNA
<213> Artificial Sequence

<220>
<223> Description of Artificial Sequence:
      Oligonucleotide for Identifying HPV 32

<400> 117
tttttctgac tattca                                                       16


<210> 118
<211> 23
<212> DNA
<213> Artificial Sequence

<220>
<223> Description of Artificial Sequence:
      Oligonucleotide for Identifying HPV 32

<400> 118
agcacatgca gataaggagg cag                                               23


<210> 119
<211> 30
<212> DNA
<213> Artificial Sequence

<220>
<223> Description of Artificial Sequence:
      Oligonucleotide for Identifying HPV 32

<400> 119
ggcagtccat atgaaagtcc cgccagtgat                                        30


<210> 120
<211> 26
<212> DNA
<213> Artificial Sequence

<220>
<223> Description of Artificial Sequence:
      Oligonucleotide for Identifying HPV 32

<400> 120
gagctaagcc ctaggcttgg tggatt                                            26

<210> 121
<211> 26
<212> DNA
<213> Artificial Sequence

<220>
<223> Description of Artificial Sequence:
      Oligonucleotide for Identifying HPV 32

<400> 121
ggggccaaac gacgactatt tcaatc                                          26


<210> 122
<211> 26
<212> DNA
<213> Artificial Sequence

<220>
<223> Description of Artificial Sequence:
      Oligonucleotide for Identifying HPV 32

<400> 122
gcaggaacag gtagaaaatg gacatg                                          26


<210> 123
<211> 26
<212> DNA
<213> Artificial Sequence

<220>
<223> Description of Artificial Sequence:
      Oligonucleotide for Identifying HPV 32

<400> 123
gtgtacatgg ggtgcaggaa aatcag                                          26


<210> 124
<211> 26
<212> DNA
<213> Artificial Sequence

<220>
<223> Description of Artificial Sequence:
      Oligonucleotide for Identifying HPV 32

<400> 124
gcctacaaca agggtggtgg aattgc                                          26


<210> 125
<211> 26
<212> DNA
<213> Artificial Sequence

<220>
<223> Description of Artificial Sequence:
      Oligonucleotide for Identifying HPV 32

<400> 125

gcaagcaaca ttgttaggta agttta                                          26

<210> 126
<211> 26
<212> DNA
<213> Artificial Sequence

<220>
<223> Description of Artificial Sequence:
      Oligonucleotide for Identifying HPV 32

<400> 126
gctgtttgga ttgtcatttg gtgatt                                          26

<210> 127
<211> 20
<212> DNA
<213> Artificial Sequence

<220>
<223> Description of Artificial Sequence:
      Oligonucleotide for Identifying HPV 33

<400> 127
tatgcacaca agtaactagt                                                 20

<210> 128
<211> 15
<212> DNA
<213> Artificial Sequence

<220>
<223> Description of Artificial Sequence:
      Oligonucleotide for Identifying HPV 33

<400> 128
cacacaagta actag                                                      15

<210> 129
<211> 20
<212> DNA
<213> Artificial Sequence

<220>
<223> Description of Artificial Sequence:
      Oligonucleotide for Identifying HPV 33

<400> 129
acaagtaact agtgacagta                                                 20

<210> 130
<211> 23
<212> DNA
<213> Artificial Sequence

<220>
<223> Description of Artificial Sequence:
      Oligonucleotide for Identifying HPV 33

<400> 130
gtaactagtg acagtacata taa                                              23


<210> 131
<211> 20
<212> DNA
<213> Artificial Sequence

<220>
<223> Description of Artificial Sequence:
      Oligonucleotide for Identifying HPV 33

<400> 131
gtacatataa aaatgaaaat                                                  20


<210> 132
<211> 23
<212> DNA
<213> Artificial Sequence

<220>
<223> Description of Artificial Sequence:
      Oligonucleotide for Identifying HPV 33

<400> 132
caaagttacc ttaactgcag aag                                              23


<210> 133
<211> 19
<212> DNA
<213> Artificial Sequence

<220>
<223> Description of Artificial Sequence:
      Oligonucleotide for Identifying HPV 33

<400> 133
tccagatatt ttagaagat                                                   19


<210> 134
<211> 26
<212> DNA
<213> Artificial Sequence

<220>
<223> Description of Artificial Sequence:
      Oligonucleotide for Identifying HPV 33

<400> 134
tttaacacct cctccatctg ctagtt                                           26


<210> 135
<211> 22
<212> DNA
<213> Artificial Sequence

<220>

<223> Description of Artificial Sequence:
      Oligonucleotide for Identifying HPV 33

<400> 135
ctataggttt gttacctctc ag                                              22


<210> 136
<211> 19
<212> DNA
<213> Artificial Sequence

<220>
<223> Description of Artificial Sequence:
      Oligonucleotide for Identifying HPV 33

<400> 136
aacagtacct ccaaaggaa                                                  19


<210> 137
<211> 16
<212> DNA
<213> Artificial Sequence

<220>
<223> Description of Artificial Sequence:
      Oligonucleotide for Identifying HPV 33

<400> 137
cttaggtaaa tataca                                                     16


<210> 138
<211> 23
<212> DNA
<213> Artificial Sequence

<220>
<223> Description of Artificial Sequence:
      Oligonucleotide for Identifying HPV 33

<400> 138
aggggaggat gatttaaatg ctg                                             23


<210> 139
<211> 26
<212> DNA
<213> Artificial Sequence

<220>
<223> Description of Artificial Sequence:
      Oligonucleotide for Identifying HPV 33

<400> 139
gcatgttcac aaagtgctgc ggagga                                          26


<210> 140
<211> 26
<212> DNA
<213> Artificial Sequence

<220>
<223> Description of Artificial Sequence:
      Oligonucleotide for Identifying HPV 33

<400> 140
gatcgtgctg caaacccgtg tagaac                                          26


<210> 141
<211> 30
<212> DNA
<213> Artificial Sequence

<220>
<223> Description of Artificial Sequence:
      Oligonucleotide for Identifying HPV 33

<400> 141
gaatgcacat acagaaaacg aaaaatagat                                      30


<210> 142
<211> 26
<212> DNA
<213> Artificial Sequence

<220>
<223> Description of Artificial Sequence:
      Oligonucleotide for Identifying HPV 33

<400> 142
aaatagatga gctagaagac agcgga                                          26


<210> 143
<211> 26
<212> DNA
<213> Artificial Sequence

<220>
<223> Description of Artificial Sequence:
      Oligonucleotide for Identifying HPV 33

<400> 143
ggtacaacag gtagaaagtc aaaatg                                          26


<210> 144
<211> 26
<212> DNA
<213> Artificial Sequence

<220>
<223> Description of Artificial Sequence:
      Oligonucleotide for Identifying HPV 33

<400> 144
gacttagaat ctagtggggt ggggga                                          26


<210> 145
<211> 26

<212> DNA
<213> Artificial Sequence

<220>
<223> Description of Artificial Sequence:
      Oligonucleotide for Identifying HPV 33

<400> 145
ctgtgagaca aatgtagata gctgtg                                          26


<210> 146
<211> 26
<212> DNA
<213> Artificial Sequence

<220>
<223> Description of Artificial Sequence:
      Oligonucleotide for Identifying HPV 33

<400> 146
cgttgcagga aattagtaat gttcta                                          26


<210> 147
<211> 23
<212> DNA
<213> Artificial Sequence

<220>
<223> Description of Artificial Sequence:
      Oligonucleotide for Identifying HPV 33

<400> 147
gaggcctatg gaataagttt tat                                             23


<210> 148
<211> 20
<212> DNA
<213> Artificial Sequence

<220>
<223> Description of Artificial Sequence:
      Oligonucleotide for Identifying HPV 35

<400> 148
tctgctgtgt cttctagtga                                                 20


<210> 149
<211> 15
<212> DNA
<213> Artificial Sequence

<220>
<223> Description of Artificial Sequence:
      Oligonucleotide for Identifying HPV 35

<400> 149
tgctgtgtct tctag                                                      15

<210> 150
<211> 20
<212> DNA
<213> Artificial Sequence

<220>
<223> Description of Artificial Sequence:
      Oligonucleotide for Identifying HPV 35

<400> 150
gtgtcttcta gtgacagtac                                                    20


<210> 151
<211> 23
<212> DNA
<213> Artificial Sequence

<220>
<223> Description of Artificial Sequence:
      Oligonucleotide for Identifying HPV 35

<400> 151
cttctagtga cagtacatat aaa                                                23


<210> 152
<211> 20
<212> DNA
<213> Artificial Sequence

<220>
<223> Description of Artificial Sequence:
      Oligonucleotide for Identifying HPV 35

<400> 152
gtacatataa aaatgacaat                                                    20


<210> 153
<211> 23
<212> DNA
<213> Artificial Sequence

<220>
<223> Description of Artificial Sequence:
      Oligonucleotide for Identifying HPV 35

<400> 153
taaaataaca ctaacagcag atg                                                23


<210> 154
<211> 19
<212> DNA
<213> Artificial Sequence

<220>
<223> Description of Artificial Sequence:
      Oligonucleotide for Identifying HPV 35

<400> 154
cccgtccatt ttagaggat                                                     19

<210> 155
<211> 26
<212> DNA
<213> Artificial Sequence

<220>
<223> Description of Artificial Sequence:
      Oligonucleotide for Identifying HPV 35

<400> 155
ccttacacca ccgccttctg gtacct                                          26


<210> 156
<211> 22
<212> DNA
<213> Artificial Sequence

<220>
<223> Description of Artificial Sequence:
      Oligonucleotide for Identifying HPV 35

<400> 156
atatcgctat gtaacatcac ag                                              22


<210> 157
<211> 19
<212> DNA
<213> Artificial Sequence

<220>
<223> Description of Artificial Sequence:
      Oligonucleotide for Identifying HPV 35

<400> 157
acccagtgca ccaaaacct                                                  19


<210> 158
<211> 23
<212> DNA
<213> Artificial Sequence

<220>
<223> Description of Artificial Sequence:
      Oligonucleotide for Identifying HPV 35

<400> 158
ggagcaaaca cacaaagagg ctg                                             23


<210> 159
<211> 30
<212> DNA
<213> Artificial Sequence

<220>
<223> Description of Artificial Sequence:
      Oligonucleotide for Identifying HPV 35

<400> 159
gcagcgtgag cttatgtgtt aataataaca                                        30


<210> 160
<211> 24
<212> DNA
<213> Artificial Sequence

<220>
<223> Description of Artificial Sequence:
      Oligonucleotide for Identifying HPV 35

<400> 160
gtccacgttt aaaagctatt tgca                                              24


<210> 161
<211> 26
<212> DNA
<213> Artificial Sequence

<220>
<223> Description of Artificial Sequence:
      Oligonucleotide for Identifying HPV 35

<400> 161
cgattatttg aactaccaga cagcgg                                            26


<210> 162
<211> 26
<212> DNA
<213> Artificial Sequence

<220>
<223> Description of Artificial Sequence:
      Oligonucleotide for Identifying HPV 35

<400> 162
gagatacaac aggtagaggg gcatga                                            26


<210> 163
<211> 26
<212> DNA
<213> Artificial Sequence

<220>
<223> Description of Artificial Sequence:
      Oligonucleotide for Identifying HPV 35

<400> 163
gggcagtggg gatagtataa cctcta                                            26


<210> 164
<211> 26
<212> DNA
<213> Artificial Sequence

<220>
<223> Description of Artificial Sequence:

Oligonucleotide for Identifying HPV 35

<400> 164
gacatgatga gactccaacg cgagac                                          26


<210> 165
<211> 26
<212> DNA
<213> Artificial Sequence

<220>
<223> Description of Artificial Sequence:
      Oligonucleotide for Identifying HPV 35

<400> 165
ctaaaatgta gtaatgcaaa cgcagc                                          26


<210> 166
<211> 26
<212> DNA
<213> Artificial Sequence

<220>
<223> Description of Artificial Sequence:
      Oligonucleotide for Identifying HPV 35

<400> 166
gctatgttgg ctaaatttaa agaact                                          26


<210> 167
<211> 15
<212> DNA
<213> Artificial Sequence

<220>
<223> Description of Artificial Sequence:
      Oligonucleotide for Identifying HPV 37

<400> 167
tgtctactga caatg                                                      15


<210> 168
<211> 20
<212> DNA
<213> Artificial Sequence

<220>
<223> Description of Artificial Sequence:
      Oligonucleotide for Identifying HPV 37

<400> 168
tgtctactga caatggcgaa                                                 20


<210> 169
<211> 20
<212> DNA
<213> Artificial Sequence

<220>
<223> Description of Artificial Sequence:
      Oligonucleotide for Identifying HPV 37

<400> 169
tgacaatggc gaagttacag                                                    20


<210> 170
<211> 20
<212> DNA
<213> Artificial Sequence

<220>
<223> Description of Artificial Sequence:
      Oligonucleotide for Identifying HPV 37

<400> 170
gacaatggcg aagttacaga                                                    20


<210> 171
<211> 20
<212> DNA
<213> Artificial Sequence

<220>
<223> Description of Artificial Sequence:
      Oligonucleotide for Identifying HPV 37

<400> 171
aatggcgaag ttacagaata                                                    20


<210> 172
<211> 20
<212> DNA
<213> Artificial Sequence

<220>
<223> Description of Artificial Sequence:
      Oligonucleotide for Identifying HPV 37

<400> 172
cagaatataa ttctcaaaca                                                    20


<210> 173
<211> 23
<212> DNA
<213> Artificial Sequence

<220>
<223> Description of Artificial Sequence:
      Oligonucleotide for Identifying HPV 37

<400> 173
taaagttcct ttaaaggctg agg                                                23


<210> 174
<211> 19
<212> DNA

<213> Artificial Sequence

<220>
<223> Description of Artificial Sequence:
      Oligonucleotide for Identifying HPV 37

<400> 174
ttctggtata ttggaagag                                                    19


<210> 175
<211> 26
<212> DNA
<213> Artificial Sequence

<220>
<223> Description of Artificial Sequence:
      Oligonucleotide for Identifying HPV 37

<400> 175
atttgtacct actccagata attcag                                            26


<210> 176
<211> 22
<212> DNA
<213> Artificial Sequence

<220>
<223> Description of Artificial Sequence:
      Oligonucleotide for Identifying HPV 37

<400> 176
ttataggtac attaattcaa ag                                                22


<210> 177
<211> 19
<212> DNA
<213> Artificial Sequence

<220>
<223> Description of Artificial Sequence:
      Oligonucleotide for Identifying HPV 37

<400> 177
tgcagttgtt gaaaaagaa                                                    19


<210> 178
<211> 16
<212> DNA
<213> Artificial Sequence

<220>
<223> Description of Artificial Sequence:
      Oligonucleotide for Identifying HPV 37

<400> 178
ctttgcaaaa tataca                                                       16


<210> 179

<211> 15
<212> DNA
<213> Artificial Sequence

<220>
<223> Description of Artificial Sequence:
      Oligonucleotide for Identifying HPV 39

<400> 179
ctctatagag tcttc                                                        15


<210> 180
<211> 20
<212> DNA
<213> Artificial Sequence

<220>
<223> Description of Artificial Sequence:
      Oligonucleotide for Identifying HPV 39

<400> 180
   tagagtcttc cataccttct                                                  20


<210> 181
<211> 20
<212> DNA
<213> Artificial Sequence

<220>
<223> Description of Artificial Sequence:
      Oligonucleotide for Identifying HPV 39

<400> 181
atagagtctt ccataccttc                                                   20


<210> 182
<211> 23
<212> DNA
<213> Artificial Sequence

<220>
<223> Description of Artificial Sequence:
      Oligonucleotide for Identifying HPV 39

<400> 182
gtcttccata ccttctacat atg                                               23


<210> 183
<211> 20
<212> DNA
<213> Artificial Sequence

<220>
<223> Description of Artificial Sequence:
      Oligonucleotide for Identifying HPV 39

<400> 183
ctacatatga tccttctaag                                                   20

<210> 184
<211> 23
<212> DNA
<213> Artificial Sequence

<220>
<223> Description of Artificial Sequence:
      Oligonucleotide for Identifying HPV 39

<400> 184
tactgtcaca ttaacaactg atg                                              23


<210> 185
<211> 18
<212> DNA
<213> Artificial Sequence

<220>
<223> Description of Artificial Sequence:
      Oligonucleotide for Identifying HPV 39

<400> 185
ttcctctata ttggacaa                                                    18


<210> 186
<211> 26
<212> DNA
<213> Artificial Sequence

<220>
<223> Description of Artificial Sequence:
      Oligonucleotide for Identifying HPV 39

<400> 186
tgtagctcct ccaccatctg ccagtt                                           26


<210> 187
<211> 22
<212> DNA
<213> Artificial Sequence

<220>
<223> Description of Artificial Sequence:
      Oligonucleotide for Identifying HPV 39

<400> 187
ttacagatac ctacagtctg ca                                               22


<210> 188
<211> 19
<212> DNA
<213> Artificial Sequence

<220>
<223> Description of Artificial Sequence:
      Oligonucleotide for Identifying HPV 39

<400> 188

ggatgctcca gcacctgaa                                                    19

<210> 189
<211> 16
<212> DNA
<213> Artificial Sequence

<220>
<223> Description of Artificial Sequence:
      Oligonucleotide for Identifying HPV 39

<400> 189
atatgacggt ctaaag                                                       16

<210> 190
<211> 23
<212> DNA
<213> Artificial Sequence

<220>
<223> Description of Artificial Sequence:
      Oligonucleotide for Identifying HPV 39

<400> 190
ggcccaaagg gatgcacaag cag                                               23

<210> 191
<211> 30
<212> DNA
<213> Artificial Sequence

<220>
<223> Description of Artificial Sequence:
      Oligonucleotide for Identifying HPV 39

<400> 191
cagacagcag tggcgacact agaccgtatg                                        30

<210> 192
<211> 25
<212> DNA
<213> Artificial Sequence

<220>
<223> Description of Artificial Sequence:
      Oligonucleotide for Identifying HPV 39

<400> 192
gtaggcagga ataccagggg aacac                                             25

<210> 193
<211> 26
<212> DNA
<213> Artificial Sequence

<220>
<223> Description of Artificial Sequence:
      Oligonucleotide for Identifying HPV 39

<400> 193
gcagtacgca ggcaacacaa acggtg                                          26


<210> 194
<211> 26
<212> DNA
<213> Artificial Sequence

<220>
<223> Description of Artificial Sequence:
      Oligonucleotide for Identifying HPV 39

<400> 194
ggaggaggta actgtagcaa ctaata                                          26


<210> 195
<211> 26
<212> DNA
<213> Artificial Sequence

<220>
<223> Description of Artificial Sequence:
      Oligonucleotide for Identifying HPV 39

<400> 195
catggcggca gtgtacggga ggagtg                                          26


<210> 196
<211> 26
<212> DNA
<213> Artificial Sequence

<220>
<223> Description of Artificial Sequence:
      Oligonucleotide for Identifying HPV 39

<400> 196
ggatagtgct atagatagtg aaaacc                                          26


<210> 197
<211> 26
<212> DNA
<213> Artificial Sequence

<220>
<223> Description of Artificial Sequence:
      Oligonucleotide for Identifying HPV 39

<400> 197
ctccaactgc acaaattaaa ttattg                                          26


<210> 198
<211> 26
<212> DNA
<213> Artificial Sequence

<220>

<223> Description of Artificial Sequence:
      Oligonucleotide for Identifying HPV 39

<400> 198
ccaataacaa aaaggctgca atgcta                                          26


<210> 199
<211> 15
<212> DNA
<213> Artificial Sequence

<220>
<223> Description of Artificial Sequence:
      Oligonucleotide for Identifying HPV 44

<400> 199
tgccactaca cagtc                                                      15


<210> 200
<211> 20
<212> DNA
<213> Artificial Sequence

<220>
<223> Description of Artificial Sequence:
      Oligonucleotide for Identifying HPV 44

<400> 200
ctacacagtc ccctccgtct                                                 20


<210> 201
<211> 20
<212> DNA
<213> Artificial Sequence

<220>
<223> Description of Artificial Sequence:
      Oligonucleotide for Identifying HPV 44

<400> 201
tgccactaca cagtcccctc                                                 20


<210> 202
<211> 22
<212> DNA
<213> Artificial Sequence

<220>
<223> Description of Artificial Sequence:
      Oligonucleotide for Identifying HPV 44

<400> 202
cagtcccctc cgtctacata ta                                              22


<210> 203
<211> 20
<212> DNA
<213> Artificial Sequence

<220>
<223> Description of Artificial Sequence:
      Oligonucleotide for Identifying HPV 44

<400> 203
ctacatatac tagtgaacaa                                                    20


<210> 204
<211> 23
<212> DNA
<213> Artificial Sequence

<220>
<223> Description of Artificial Sequence:
      Oligonucleotide for Identifying HPV 44

<400> 204
tagtattacc ttaacggcgg agg                                                23


<210> 205
<211> 19
<212> DNA
<213> Artificial Sequence

<220>
<223> Description of Artificial Sequence:
      Oligonucleotide for Identifying HPV 44

<400> 205
tgctggtatt ttagaacag                                                     19


<210> 206
<211> 26
<212> DNA
<213> Artificial Sequence

<220>
<223> Description of Artificial Sequence:
      Oligonucleotide for Identifying HPV 44

<400> 206
gttgtcgccg cccccaaatg gtacct                                             26


<210> 207
<211> 22
<212> DNA
<213> Artificial Sequence

<220>
<223> Description of Artificial Sequence:
      Oligonucleotide for Identifying HPV 44

<400> 207
atacagatat gtgcagtccc ag                                                 22


<210> 208
<211> 19

<212> DNA
<213> Artificial Sequence

<220>
<223> Description of Artificial Sequence:
      Oligonucleotide for Identifying HPV 44

<400> 208
gccaccccct gaaaaggca                                                    19


<210> 209
<211> 16
<212> DNA
<213> Artificial Sequence

<220>
<223> Description of Artificial Sequence:
      Oligonucleotide for Identifying HPV 44

<400> 209
ctatgcaaaa ttaagt                                                       16


<210> 210
<211> 23
<212> DNA
<213> Artificial Sequence

<220>
<223> Description of Artificial Sequence:
      Oligonucleotide for Identifying HPV 44

<400> 210
ggcggatgct cattatgcgg ctg                                               23


<210> 211
<211> 30
<212> DNA
<213> Artificial Sequence

<220>
<223> Description of Artificial Sequence:
      Oligonucleotide for Identifying HPV 44

<400> 211
ggtagtccat atgttagtcc tttaagtaat                                        30


<210> 212
<211> 26
<212> DNA
<213> Artificial Sequence

<220>
<223> Description of Artificial Sequence:
      Oligonucleotide for Identifying HPV 44

<400> 212
cacggctgga cgctataaca ttaagt                                            26

<210> 213
<211> 26
<212> DNA
<213> Artificial Sequence

<220>
<223> Description of Artificial Sequence:
      Oligonucleotide for Identifying HPV 44

<400> 213
gacggctgtt tgacagacca gaatta                                          26


<210> 214
<211> 26
<212> DNA
<213> Artificial Sequence

<220>
<223> Description of Artificial Sequence:
      Oligonucleotide for Identifying HPV 44

<400> 214
gctgaaacgc aggtagagag aaatgg                                          26


<210> 215
<211> 26
<212> DNA
<213> Artificial Sequence

<220>
<223> Description of Artificial Sequence:
      Oligonucleotide for Identifying HPV 44

<400> 215
gggaggtgga caaggaaggg acacag                                          26


<210> 216
<211> 26
<212> DNA
<213> Artificial Sequence

<220>
<223> Description of Artificial Sequence:
      Oligonucleotide for Identifying HPV 44

<400> 216
ggaagtgcag acacatagca acacac                                          26


<210> 217
<211> 26
<212> DNA
<213> Artificial Sequence

<220>
<223> Description of Artificial Sequence:
      Oligonucleotide for Identifying HPV 44

<400> 217
ggtactagaa ctattgaaat gtaaga                                          26

<210> 218
<211> 26
<212> DNA
<213> Artificial Sequence

<220>
<223> Description of Artificial Sequence:
      Oligonucleotide for Identifying HPV 44

<400> 218
gcttggtaag tttaaggatt gctatg                                          26


<210> 219
<211> 20
<212> DNA
<213> Artificial Sequence

<220>
<223> Description of Artificial Sequence:
      Oligonucleotide for Identifying HPV 45

<400> 219
tgcctctaca caaaatcctg                                                 20


<210> 220
<211> 15
<212> DNA
<213> Artificial Sequence

<220>
<223> Description of Artificial Sequence:
      Oligonucleotide for Identifying HPV 45

<400> 220
ctctacacaa aatcc                                                      15


<210> 221
<211> 20
<212> DNA
<213> Artificial Sequence

<220>
<223> Description of Artificial Sequence:
      Oligonucleotide for Identifying HPV 45

<400> 221
acaaaatcct gtgccaagta                                                 20


<210> 222
<211> 20
<212> DNA
<213> Artificial Sequence

<220>
<223> Description of Artificial Sequence:
      Oligonucleotide for Identifying HPV 45

<400> 222
caaaatcctg tgccaagtac                                                    20

<210> 223
<211> 22
<212> DNA
<213> Artificial Sequence

<220>
<223> Description of Artificial Sequence:
      Oligonucleotide for Identifying HPV 45

<400> 223
aatcctgtgc caagtacata tg                                                 22

<210> 224
<211> 20
<212> DNA
<213> Artificial Sequence

<220>
<223> Description of Artificial Sequence:
      Oligonucleotide for Identifying HPV 45

<400> 224
gtacatatga ccctactaag                                                    20

<210> 225
<211> 23
<212> DNA
<213> Artificial Sequence

<220>
<223> Description of Artificial Sequence:
      Oligonucleotide for Identifying HPV 45

<400> 225
cactattact ttaactgcag agg                                                23

<210> 226
<211> 19
<212> DNA
<213> Artificial Sequence

<220>
<223> Description of Artificial Sequence:
      Oligonucleotide for Identifying HPV 45

<400> 226
tagtagtata ttagaaaat                                                     19

<210> 227
<211> 26
<212> DNA
<213> Artificial Sequence

<220>
<223> Description of Artificial Sequence:

Oligonucleotide for Identifying HPV 45

<400> 227
tgtccctcca ccacctacta caagtt                                          26


<210> 228
<211> 22
<212> DNA
<213> Artificial Sequence

<220>
<223> Description of Artificial Sequence:
      Oligonucleotide for Identifying HPV 45

<400> 228
atatcgtttt gtgcaatcag tt                                              22


<210> 229
<211> 19
<212> DNA
<213> Artificial Sequence

<220>
<223> Description of Artificial Sequence:
      Oligonucleotide for Identifying HPV 45

<400> 229
ggatactaca cctccagaa                                                  19


<210> 230
<211> 23
<212> DNA
<213> Artificial Sequence

<220>
<223> Description of Artificial Sequence:
      Oligonucleotide for Identifying HPV 45

<400> 230
agttcagaat gatgcacagg tgt                                             23


<210> 231
<211> 30
<212> DNA
<213> Artificial Sequence

<220>
<223> Description of Artificial Sequence:
      Oligonucleotide for Identifying HPV 45

<400> 231
gcagctaagt gtggatacgg atctaagtcc                                      30


<210> 232
<211> 26
<212> DNA
<213> Artificial Sequence

<220>
<223> Description of Artificial Sequence:
      Oligonucleotide for Identifying HPV 45

<400> 232
caagaaattt cattaaatag tgggca                                          26


<210> 233
<211> 26
<212> DNA
<213> Artificial Sequence

<220>
<223> Description of Artificial Sequence:
      Oligonucleotide for Identifying HPV 45

<400> 233
acggttgttt acaatatcag atagtg                                          26


<210> 234
<211> 26
<212> DNA
<213> Artificial Sequence

<220>
<223> Description of Artificial Sequence:
      Oligonucleotide for Identifying HPV 45

<400> 234
catagtacac aaagtagtgg tgggga                                          26


<210> 235
<211> 26
<212> DNA
<213> Artificial Sequence

<220>
<223> Description of Artificial Sequence:
      Oligonucleotide for Identifying HPV 45

<400> 235
gacaatgcag aaaatgtaga tccgca                                          26


<210> 236
<211> 26
<212> DNA
<213> Artificial Sequence

<220>
<223> Description of Artificial Sequence:
      Oligonucleotide for Identifying HPV 45

<400> 236
ggagctatta caagcaagta acaaaa                                          26


<210> 237
<211> 25
<212> DNA

<213> Artificial Sequence

<220>
<223> Description of Artificial Sequence:
      Oligonucleotide for Identifying HPV 45

<400> 237
gcaatgctgg cagtatttaa agaca                                          25


<210> 238
<211> 26
<212> DNA
<213> Artificial Sequence

<220>
<223> Description of Artificial Sequence:
      Oligonucleotide for Identifying HPV 45

<400> 238
atatgggctg tcatttacgg atttgg                                         26


<210> 239
<211> 20
<212> DNA
<213> Artificial Sequence

<220>
<223> Description of Artificial Sequence:
      Oligonucleotide for Identifying HPV 51

<400> 239
cactgccact gctgcggttt                                                20


<210> 240
<211> 15
<212> DNA
<213> Artificial Sequence

<220>
<223> Description of Artificial Sequence:
      Oligonucleotide for Identifying HPV 51

<400> 240
tgccactgct gcggt                                                     15


<210> 241
<211> 20
<212> DNA
<213> Artificial Sequence

<220>
<223> Description of Artificial Sequence:
      Oligonucleotide for Identifying HPV 51

<400> 241
cactgctgcg gtttccccaa                                                20


<210> 242

<211> 20
<212> DNA
<213> Artificial Sequence

<220>
<223> Description of Artificial Sequence:
      Oligonucleotide for Identifying HPV 51

<400> 242
ccactgctgc ggtttcccca                                                    20


<210> 243
<211> 22
<212> DNA
<213> Artificial Sequence

<220>
<223> Description of Artificial Sequence:
      Oligonucleotide for Identifying HPV 51

<400> 243
ctgcggtttc cccaacattt ac                                                 22


<210> 244
<211> 20
<212> DNA
<213> Artificial Sequence

<220>
<223> Description of Artificial Sequence:
      Oligonucleotide for Identifying HPV 51

<400> 244
caacatttac tccaagtaac                                                    20


<210> 245
<211> 23
<212> DNA
<213> Artificial Sequence

<220>
<223> Description of Artificial Sequence:
      Oligonucleotide for Identifying HPV 51

<400> 245
taaaattact ttaactacag agg                                                23


<210> 246
<211> 19
<212> DNA
<213> Artificial Sequence

<220>
<223> Description of Artificial Sequence:
      Oligonucleotide for Identifying HPV 51

<400> 246
tcctaccatt cttgaacag                                                     19

<210> 247
<211> 26
<212> DNA
<213> Artificial Sequence

<220>
<223> Description of Artificial Sequence:
      Oligonucleotide for Identifying HPV 51

<400> 247
attaacatta cctccgtctg ctagtt                                          26


<210> 248
<211> 22
<212> DNA
<213> Artificial Sequence

<220>
<223> Description of Artificial Sequence:
      Oligonucleotide for Identifying HPV 51

<400> 248
atataggttt gttagaaatg ca                                              22


<210> 249
<211> 19
<212> DNA
<213> Artificial Sequence

<220>
<223> Description of Artificial Sequence:
      Oligonucleotide for Identifying HPV 51

<400> 249
ggacacccct ccacaggct                                                  19


<210> 250
<211> 16
<212> DNA
<213> Artificial Sequence

<220>
<223> Description of Artificial Sequence:
      Oligonucleotide for Identifying HPV 51

<400> 250
tttggccaaa tataaa                                                     16


<210> 251
<211> 23
<212> DNA
<213> Artificial Sequence

<220>
<223> Description of Artificial Sequence:
      Oligonucleotide for Identifying HPV 51

<400> 251

attacaggca aacaaagagg ctg                                              23

<210> 252
<211> 30
<212> DNA
<213> Artificial Sequence

<220>
<223> Description of Artificial Sequence:
      Oligonucleotide for Identifying HPV 51

<400> 252
gcgaagcagc ccattaggag acattacaaa                                       30

<210> 253
<211> 26
<212> DNA
<213> Artificial Sequence

<220>
<223> Description of Artificial Sequence:
      Oligonucleotide for Identifying HPV 51

<400> 253
ccatagtcag gcaaacgagt cacaag                                           26

<210> 254
<211> 25
<212> DNA
<213> Artificial Sequence

<220>
<223> Description of Artificial Sequence:
      Oligonucleotide for Identifying HPV 51

<400> 254
agattactgg acagttatcc ggaca                                            25

<210> 255
<211> 26
<212> DNA
<213> Artificial Sequence

<220>
<223> Description of Artificial Sequence:
      Oligonucleotide for Identifying HPV 51

<400> 255
caggtagatg ggcaacatgg cggttc                                           26

<210> 256
<211> 26
<212> DNA
<213> Artificial Sequence

<220>
<223> Description of Artificial Sequence:
      Oligonucleotide for Identifying HPV 51

<400> 256
agctgtgcaa atgtagaact aaacag                                          26


<210> 257
<211> 26
<212> DNA
<213> Artificial Sequence

<220>
<223> Description of Artificial Sequence:
      Oligonucleotide for Identifying HPV 51

<400> 257
ggtattagtt ataatgagtt ggtacg                                          26


<210> 258
<211> 15
<212> DNA
<213> Artificial Sequence

<220>
<223> Description of Artificial Sequence:
      Oligonucleotide for Identifying HPV 52

<400> 258
tgaggttaaa aagga                                                      15


<210> 259
<211> 20
<212> DNA
<213> Artificial Sequence

<220>
<223> Description of Artificial Sequence:
      Oligonucleotide for Identifying HPV 52

<400> 259
tgaggttaaa aaggaaagca                                                 20


<210> 260
<211> 20
<212> DNA
<213> Artificial Sequence

<220>
<223> Description of Artificial Sequence:
      Oligonucleotide for Identifying HPV 52

<400> 260
gaggttaaaa aggaaagcac                                                 20


<210> 261
<211> 20
<212> DNA
<213> Artificial Sequence

<220>

<223> Description of Artificial Sequence:
      Oligonucleotide for Identifying HPV 52

<400> 261
ttaaaaagga aagcacatat                                                    20


<210> 262
<211> 22
<212> DNA
<213> Artificial Sequence

<220>
<223> Description of Artificial Sequence:
      Oligonucleotide for Identifying HPV 52

<400> 262
aaaggaaagc acatataaaa at                                                 22


<210> 263
<211> 20
<212> DNA
<213> Artificial Sequence

<220>
<223> Description of Artificial Sequence:
      Oligonucleotide for Identifying HPV 52

<400> 263
gcacatataa aaatgaaaat                                                    20


<210> 264
<211> 23
<212> DNA
<213> Artificial Sequence

<220>
<223> Description of Artificial Sequence:
      Oligonucleotide for Identifying HPV 52

<400> 264
caaaattaca ttaacagctg atg                                                23


<210> 265
<211> 19
<212> DNA
<213> Artificial Sequence

<220>
<223> Description of Artificial Sequence:
      Oligonucleotide for Identifying HPV 52

<400> 265
tgccactatt ttagaggac                                                     19


<210> 266
<211> 26
<212> DNA
<213> Artificial Sequence

<220>
<223> Description of Artificial Sequence:
      Oligonucleotide for Identifying HPV 52

<400> 266
ccttacccca ccaccgtctg catctt                                          26


<210> 267
<211> 22
<212> DNA
<213> Artificial Sequence

<220>
<223> Description of Artificial Sequence:
      Oligonucleotide for Identifying HPV 52

<400> 267
atacagattt gtcacttcta ct                                              22


<210> 268
<211> 19
<212> DNA
<213> Artificial Sequence

<220>
<223> Description of Artificial Sequence:
      Oligonucleotide for Identifying HPV 52

<400> 268
aaacacacca cctaaagga                                                  19


<210> 269
<211> 16
<212> DNA
<213> Artificial Sequence

<220>
<223> Description of Artificial Sequence:
      Oligonucleotide for Identifying HPV 52

<400> 269
tttaaaggac tatatg                                                     16


<210> 270
<211> 23
<212> DNA
<213> Artificial Sequence

<220>
<223> Description of Artificial Sequence:
      Oligonucleotide for Identifying HPV 52

<400> 270
aggggaggat gatttacatg ctg                                             23


<210> 271
<211> 30

<212> DNA
<213> Artificial Sequence

<220>
<223> Description of Artificial Sequence:
      Oligonucleotide for Identifying HPV 52

<400> 271
gcagtccgga aagtgctggg caagatggtg                                        30


<210> 272
<211> 26
<212> DNA
<213> Artificial Sequence

<220>
<223> Description of Artificial Sequence:
      Oligonucleotide for Identifying HPV 52

<400> 272
ggtagtccgc gtgcaaaaca catttg                                            26


<210> 273
<211> 26
<212> DNA
<213> Artificial Sequence

<220>
<223> Description of Artificial Sequence:
      Oligonucleotide for Identifying HPV 52

<400> 273
ccaaaacgca aaccatgtca cgtaga                                            26


<210> 274
<211> 22
<212> DNA
<213> Artificial Sequence

<220>
<223> Description of Artificial Sequence:
      Oligonucleotide for Identifying HPV 52

<400> 274
caaaatggcg actggcaaag ta                                                22


<210> 275
<211> 26
<212> DNA
<213> Artificial Sequence

<220>
<223> Description of Artificial Sequence:
      Oligonucleotide for Identifying HPV 52

<400> 275
gctagtaatt cagatgtaag ttgtac                                            26

<210> 276
<211> 26
<212> DNA
<213> Artificial Sequence

<220>
<223> Description of Artificial Sequence:
      Oligonucleotide for Identifying HPV 52

<400> 276
gaggaaaata gtaatagaac gctaaa                                          26


<210> 277
<211> 26
<212> DNA
<213> Artificial Sequence

<220>
<223> Description of Artificial Sequence:
      Oligonucleotide for Identifying HPV 52

<400> 277
gcgaaaatag cataaaaaca actgta                                          26


<210> 278
<211> 26
<212> DNA
<213> Artificial Sequence

<220>
<223> Description of Artificial Sequence:
      Oligonucleotide for Identifying HPV 52

<400> 278
agaaacatat ggtgttagct ttatgg                                          26


<210> 279
<211> 20
<212> DNA
<213> Artificial Sequence

<220>
<223> Description of Artificial Sequence:
      Oligonucleotide for Identifying HPV 53

<400> 279
tccgcaacca cacagtctat                                                 20


<210> 280
<211> 15
<212> DNA
<213> Artificial Sequence

<220>
<223> Description of Artificial Sequence:
      Oligonucleotide for Identifying HPV 53

<400> 280
ccgcaaccac acagt                                                      15

<210> 281
<211> 20
<212> DNA
<213> Artificial Sequence

<220>
<223> Description of Artificial Sequence:
      Oligonucleotide for Identifying HPV 53

<400> 281
ccgcaaccac acagtctatg                                                    20


<210> 282
<211> 22
<212> DNA
<213> Artificial Sequence

<220>
<223> Description of Artificial Sequence:
      Oligonucleotide for Identifying HPV 53

<400> 282
cacagtctat gtctacatat aa                                                 22


<210> 283
<211> 20
<212> DNA
<213> Artificial Sequence

<220>
<223> Description of Artificial Sequence:
      Oligonucleotide for Identifying HPV 53

<400> 283
ctacatataa ttcaaagcaa                                                    20


<210> 284
<211> 23
<212> DNA
<213> Artificial Sequence

<220>
<223> Description of Artificial Sequence:
      Oligonucleotide for Identifying HPV 53

<400> 284
taaaatatcc ctgtctgctg agg                                                23


<210> 285
<211> 19
<212> DNA
<213> Artificial Sequence

<220>
<223> Description of Artificial Sequence:
      Oligonucleotide for Identifying HPV 53

<400> 285
ttctacctta ctggaagac                                                    19


<210> 286
<211> 26
<212> DNA
<213> Artificial Sequence

<220>
<223> Description of Artificial Sequence:
      Oligonucleotide for Identifying HPV 53

<400> 286
tttgtcgcct cctgttgcca ctagct                                            26


<210> 287
<211> 22
<212> DNA
<213> Artificial Sequence

<220>
<223> Description of Artificial Sequence:
      Oligonucleotide for Identifying HPV 53

<400> 287
atacagatat gtgaaaagtg ca                                                22


<210> 288
<211> 19
<212> DNA
<213> Artificial Sequence

<220>
<223> Description of Artificial Sequence:
      Oligonucleotide for Identifying HPV 53

<400> 288
ggatcagccc cctcctgaa                                                    19


<210> 289
<211> 15
<212> DNA
<213> Artificial Sequence

<220>
<223> Description of Artificial Sequence:
      Oligonucleotide for Identifying HPV 54

<400> 289
tacagcatcc acgca                                                        15


<210> 290
<211> 20
<212> DNA
<213> Artificial Sequence

<220>
<223> Description of Artificial Sequence:

Oligonucleotide for Identifying HPV 54

<400> 290
cagcatccac gcaggatagc                                                    20


<210> 291
<211> 20
<212> DNA
<213> Artificial Sequence

<220>
<223> Description of Artificial Sequence:
      Oligonucleotide for Identifying HPV 54

<400> 291
acgcaggata gctttaataa                                                    20


<210> 292
<211> 20
<212> DNA
<213> Artificial Sequence

<220>
<223> Description of Artificial Sequence:
      Oligonucleotide for Identifying HPV 54

<400> 292
cacgcaggat agctttaata                                                    20


<210> 293
<211> 20
<212> DNA
<213> Artificial Sequence

<220>
<223> Description of Artificial Sequence:
      Oligonucleotide for Identifying HPV 54

<400> 293
atagctttaa taattctgac                                                    20


<210> 294
<211> 23
<212> DNA
<213> Artificial Sequence

<220>
<223> Description of Artificial Sequence:
      Oligonucleotide for Identifying HPV 54

<400> 294
taccataacc cttacagcag atg                                                23


<210> 295
<211> 19
<212> DNA
<213> Artificial Sequence

<220>
<223> Description of Artificial Sequence:
      Oligonucleotide for Identifying HPV 54

<400> 295
tcccactatt ctagaggac                                                    19


<210> 296
<211> 26
<212> DNA
<213> Artificial Sequence

<220>
<223> Description of Artificial Sequence:
      Oligonucleotide for Identifying HPV 54

<400> 296
tataaccccc ccagctacaa gtagtt                                            26


<210> 297
<211> 22
<212> DNA
<213> Artificial Sequence

<220>
<223> Description of Artificial Sequence:
      Oligonucleotide for Identifying HPV 54

<400> 297
atataggttt gtacagtcac ag                                                22


<210> 298
<211> 22
<212> DNA
<213> Artificial Sequence

<220>
<223> Description of Artificial Sequence:
      Oligonucleotide for Identifying HPV 54

<400> 298
gaataatgcc cctgcaaagg aa                                                22


<210> 299
<211> 23
<212> DNA
<213> Artificial Sequence

<220>
<223> Description of Artificial Sequence:
      Oligonucleotide for Identifying HPV 54

<400> 299
gctgcaggca gatgtagagg cag                                               23


<210> 300
<211> 30
<212> DNA

<213> Artificial Sequence

<220>
<223> Description of Artificial Sequence:
      Oligonucleotide for Identifying HPV 54

<400> 300
cgtatgtaag tcctgttgca aacagcgaac                                        30


<210> 301
<211> 20
<212> DNA
<213> Artificial Sequence

<220>
<223> Description of Artificial Sequence:
      Oligonucleotide for Identifying HPV 54

<400> 301
ctgtgtagaa aaggacctaa                                                   20


<210> 302
<211> 26
<212> DNA
<213> Artificial Sequence

<220>
<223> Description of Artificial Sequence:
      Oligonucleotide for Identifying HPV 54

<400> 302
tatatcccta ggacggcggt cagcca                                            26


<210> 303
<211> 26
<212> DNA
<213> Artificial Sequence

<220>
<223> Description of Artificial Sequence:
      Oligonucleotide for Identifying HPV 54

<400> 303
ggtaaatacc gaggggacag atgaaa                                            26


<210> 304
<211> 26
<212> DNA
<213> Artificial Sequence

<220>
<223> Description of Artificial Sequence:
      Oligonucleotide for Identifying HPV 54

<400> 304
gaaacaacta cagatagcct aggaag                                            26


<210> 305

<211> 26
<212> DNA
<213> Artificial Sequence

<220>
<223> Description of Artificial Sequence:
      Oligonucleotide for Identifying HPV 54

<400> 305
cgtgtagcat tgtttggtat gtttaa                                          26


<210> 306
<211> 22
<212> DNA
<213> Artificial Sequence

<220>
<223> Description of Artificial Sequence:
      Oligonucleotide for Identifying HPV 54

<400> 306
tatggattaa gttttatgga cc                                              22


<210> 307
<211> 15
<212> DNA
<213> Artificial Sequence

<220>
<223> Description of Artificial Sequence:
      Oligonucleotide for Identifying HPV 56

<400> 307
ctgctacaga acagt                                                      15


<210> 308
<211> 20
<212> DNA
<213> Artificial Sequence

<220>
<223> Description of Artificial Sequence:
      Oligonucleotide for Identifying HPV 56

<400> 308
gctacagaac agttaagtaa                                                 20


<210> 309
<211> 20
<212> DNA
<213> Artificial Sequence

<220>
<223> Description of Artificial Sequence:
      Oligonucleotide for Identifying HPV 56

<400> 309
cagaacagtt aagtaaatat                                                 20

<210> 310
<211> 23
<212> DNA
<213> Artificial Sequence

<220>
<223> Description of Artificial Sequence:
      Oligonucleotide for Identifying HPV 56

<400> 310
gaacagttaa gtaaatatga tgc                                              23


<210> 311
<211> 20
<212> DNA
<213> Artificial Sequence

<220>
<223> Description of Artificial Sequence:
      Oligonucleotide for Identifying HPV 56

<400> 311
gtaaatatga tgcacgaaaa                                                  20


<210> 312
<211> 23
<212> DNA
<213> Artificial Sequence

<220>
<223> Description of Artificial Sequence:
      Oligonucleotide for Identifying HPV 56

<400> 312
caaaattact ttgtctgcag agg                                              23


<210> 313
<211> 19
<212> DNA
<213> Artificial Sequence

<220>
<223> Description of Artificial Sequence:
      Oligonucleotide for Identifying HPV 56

<400> 313
tgctaaccta ctggaggac                                                   19


<210> 314
<211> 26
<212> DNA
<213> Artificial Sequence

<220>
<223> Description of Artificial Sequence:
      Oligonucleotide for Identifying HPV 56

<400> 314

gttatccccg ccagtggcca ccagcc 26

<210> 315
<211> 22
<212> DNA
<213> Artificial Sequence

<220>
<223> Description of Artificial Sequence:
      Oligonucleotide for Identifying HPV 56

<400> 315
atatagatat gttagaagca ca 22

<210> 316
<211> 19
<212> DNA
<213> Artificial Sequence

<220>
<223> Description of Artificial Sequence:
      Oligonucleotide for Identifying HPV 56

<400> 316
ggaacagcca ccaacagaa 19

<210> 317
<211> 20
<212> DNA
<213> Artificial Sequence

<220>
<223> Description of Artificial Sequence:
      Oligonucleotide for Identifying HPV 58

<400> 317
atgcactgaa gtaactaagg 20

<210> 318
<211> 20
<212> DNA
<213> Artificial Sequence

<220>
<223> Description of Artificial Sequence:
      Oligonucleotide for Identifying HPV 58

<400> 318
cactgaagta actaaggaag 20

<210> 319
<211> 15
<212> DNA
<213> Artificial Sequence

<220>
<223> Description of Artificial Sequence:
      Oligonucleotide for Identifying HPV 58

<400> 319
tgaagtaact aagga                                                        15


<210> 320
<211> 20
<212> DNA
<213> Artificial Sequence

<220>
<223> Description of Artificial Sequence:
      Oligonucleotide for Identifying HPV 58

<400> 320
gaagtaacta aggaaggtac                                                   20


<210> 321
<211> 22
<212> DNA
<213> Artificial Sequence

<220>
<223> Description of Artificial Sequence:
      Oligonucleotide for Identifying HPV 58

<400> 321
ctaaggaagg tacatataaa aa                                                22


<210> 322
<211> 20
<212> DNA
<213> Artificial Sequence

<220>
<223> Description of Artificial Sequence:
      Oligonucleotide for Identifying HPV 58

<400> 322
ataaaaatga taattttaag                                                   20


<210> 323
<211> 23
<212> DNA
<213> Artificial Sequence

<220>
<223> Description of Artificial Sequence:
      Oligonucleotide for Identifying HPV 58

<400> 323
caaaattaca ctaactgcag aga                                               23


<210> 324
<211> 19
<212> DNA
<213> Artificial Sequence

<220>

<223> Description of Artificial Sequence:
      Oligonucleotide for Identifying HPV 58

<400> 324
ttccaatatt ttggaggac                                                    19


<210> 325
<211> 26
<212> DNA
<213> Artificial Sequence

<220>
<223> Description of Artificial Sequence:
      Oligonucleotide for Identifying HPV 58

<400> 325
tttaacacct cctccgtctg ccagtt                                            26


<210> 326
<211> 22
<212> DNA
<213> Artificial Sequence

<220>
<223> Description of Artificial Sequence:
      Oligonucleotide for Identifying HPV 58

<400> 326
atatagattt gttacctccc ag                                                22


<210> 327
<211> 19
<212> DNA
<213> Artificial Sequence

<220>
<223> Description of Artificial Sequence:
      Oligonucleotide for Identifying HPV 58

<400> 327
aacagcaccc cctaaagaa                                                    19


<210> 328
<211> 23
<212> DNA
<213> Artificial Sequence

<220>
<223> Description of Artificial Sequence:
      Oligonucleotide for Identifying HPV 58

<400> 328
aggggtggac gatataaatg ctg                                               23


<210> 329
<211> 23
<212> DNA
<213> Artificial Sequence

<220>
<223> Description of Artificial Sequence:
      Oligonucleotide for Identifying HPV 58

<400> 329
gcatgctcag aaagtgctgt aga                                              23


<210> 330
<211> 30
<212> DNA
<213> Artificial Sequence

<220>
<223> Description of Artificial Sequence:
      Oligonucleotide for Identifying HPV 58

<400> 330
gcaaatgtgt gtgtatcgtg gaaatataaa                                       30


<210> 331
<211> 22
<212> DNA
<213> Artificial Sequence

<220>
<223> Description of Artificial Sequence:
      Oligonucleotide for Identifying HPV 58

<400> 331
aaattattga gctagaagac ag                                               22


<210> 332
<211> 21
<212> DNA
<213> Artificial Sequence

<220>
<223> Description of Artificial Sequence:
      Oligonucleotide for Identifying HPV 58

<400> 332
gcacaccagg tagaaagcca a                                                21


<210> 333
<211> 26
<212> DNA
<213> Artificial Sequence

<220>
<223> Description of Artificial Sequence:
      Oligonucleotide for Identifying HPV 58

<400> 333
ggctagttca gatgtaagca gtgaaa                                           26


<210> 334
<211> 22

<212> DNA
<213> Artificial Sequence

<220>
<223> Description of Artificial Sequence:
      Oligonucleotide for Identifying HPV 58

<400> 334
gtaatattct acataacagt aa                                                22


<210> 335
<211> 21
<212> DNA
<213> Artificial Sequence

<220>
<223> Description of Artificial Sequence:
      Oligonucleotide for Identifying HPV 58

<400> 335
gcaacgctat tatataaatt c                                                 21


<210> 336
<211> 24
<212> DNA
<213> Artificial Sequence

<220>
<223> Description of Artificial Sequence:
      Oligonucleotide for Identifying HPV 58

<400> 336
gcttatggag taagttttat ggaa                                              24


<210> 337
<211> 15
<212> DNA
<213> Artificial Sequence

<220>
<223> Description of Artificial Sequence:
      Oligonucleotide for Identifying HPV 59

<400> 337
ttctactact tcttc                                                        15


<210> 338
<211> 20
<212> DNA
<213> Artificial Sequence

<220>
<223> Description of Artificial Sequence:
      Oligonucleotide for Identifying HPV 59

<400> 338
actacttctt ctattcctaa                                                   20

<210> 339
<211> 20
<212> DNA
<213> Artificial Sequence

<220>
<223> Description of Artificial Sequence:
      Oligonucleotide for Identifying HPV 59

<400> 339
acttcttcta ttcctaatgt                                                    20


<210> 340
<211> 23
<212> DNA
<213> Artificial Sequence

<220>
<223> Description of Artificial Sequence:
      Oligonucleotide for Identifying HPV 59

<400> 340
tcttctattc ctaatgtata cac                                                23


<210> 341
<211> 20
<212> DNA
<213> Artificial Sequence

<220>
<223> Description of Artificial Sequence:
      Oligonucleotide for Identifying HPV 59

<400> 341
atgtatacac acctaccagt                                                    20


<210> 342
<211> 23
<212> DNA
<213> Artificial Sequence

<220>
<223> Description of Artificial Sequence:
      Oligonucleotide for Identifying HPV 59

<400> 342
taaaataaca ttaactacag agg                                                23


<210> 343
<211> 19
<212> DNA
<213> Artificial Sequence

<220>
<223> Description of Artificial Sequence:
      Oligonucleotide for Identifying HPV 59

<400> 343
taccactatt ttggaggat                                                     19

<210> 344
<211> 26
<212> DNA
<213> Artificial Sequence

<220>
<223> Description of Artificial Sequence:
      Oligonucleotide for Identifying HPV 59

<400> 344
tgttacacca cctcctactg ctagtt                                          26


<210> 345
<211> 22
<212> DNA
<213> Artificial Sequence

<220>
<223> Description of Artificial Sequence:
      Oligonucleotide for Identifying HPV 59

<400> 345
ataccgtttt gttcaatctg ct                                              22


<210> 346
<211> 19
<212> DNA
<213> Artificial Sequence

<220>
<223> Description of Artificial Sequence:
      Oligonucleotide for Identifying HPV 59

<400> 346
ggacaccgca ccgccagtt                                                  19


<210> 347
<211> 16
<212> DNA
<213> Artificial Sequence

<220>
<223> Description of Artificial Sequence:
      Oligonucleotide for Identifying HPV 59

<400> 347
ttatgacaaa ctaaag                                                     16


<210> 348
<211> 23
<212> DNA
<213> Artificial Sequence

<220>
<223> Description of Artificial Sequence:
      Oligonucleotide for Identifying HPV 59

<400> 348
agcccaaagg gatgcacggg aaa                                              23


<210> 349
<211> 30
<212> DNA
<213> Artificial Sequence

<220>
<223> Description of Artificial Sequence:
      Oligonucleotide for Identifying HPV 59

<400> 349
gcagtataga aaacagtagt gagaaagcgg                                       30


<210> 350
<211> 25
<212> DNA
<213> Artificial Sequence

<220>
<223> Description of Artificial Sequence:
      Oligonucleotide for Identifying HPV 59

<400> 350
ccattacaag aaatatcagt aaatg                                            25


<210> 351
<211> 24
<212> DNA
<213> Artificial Sequence

<220>
<223> Description of Artificial Sequence:
      Oligonucleotide for Identifying HPV 59

<400> 351
ggttaataac agtgccagac agcg                                             24


<210> 352
<211> 26
<212> DNA
<213> Artificial Sequence

<220>
<223> Description of Artificial Sequence:
      Oligonucleotide for Identifying HPV 59

<400> 352
ggtaaccgtg gagaatactg gaaatg                                           26


<210> 353
<211> 26
<212> DNA
<213> Artificial Sequence

<220>
<223> Description of Artificial Sequence:

Oligonucleotide for Identifying HPV 59

<400> 353
ctgtagcgac agcagtaaca tggatg                                          26


<210> 354
<211> 24
<212> DNA
<213> Artificial Sequence

<220>
<223> Description of Artificial Sequence:
      Oligonucleotide for Identifying HPV 59

<400> 354
cccactaatc aattgttaca gtta                                            24


<210> 355
<211> 22
<212> DNA
<213> Artificial Sequence

<220>
<223> Description of Artificial Sequence:
      Oligonucleotide for Identifying HPV 59

<400> 355
gggttatcat ttcaagattt gg                                              22


<210> 356
<211> 15
<212> DNA
<213> Artificial Sequence

<220>
<223> Description of Artificial Sequence:
      Oligonucleotide for Identifying HPV 61

<400> 356
ctgctacatc ccccc                                                      15


<210> 357
<211> 20
<212> DNA
<213> Artificial Sequence

<220>
<223> Description of Artificial Sequence:
      Oligonucleotide for Identifying HPV 61

<400> 357
acatcccccc ctgtatctga                                                 20


<210> 358
<211> 20
<212> DNA
<213> Artificial Sequence

<220>
<223> Description of Artificial Sequence:
      Oligonucleotide for Identifying HPV 61

<400> 358
catccccccc tgtatctgaa                                                    20


<210> 359
<211> 22
<212> DNA
<213> Artificial Sequence

<220>
<223> Description of Artificial Sequence:
      Oligonucleotide for Identifying HPV 61

<400> 359
cccctgtatc tgaatataaa gc                                                 22


<210> 360
<211> 20
<212> DNA
<213> Artificial Sequence

<220>
<223> Description of Artificial Sequence:
      Oligonucleotide for Identifying HPV 61

<400> 360
ctgaatataa agccacaagc                                                    20


<210> 361
<211> 23
<212> DNA
<213> Artificial Sequence

<220>
<223> Description of Artificial Sequence:
      Oligonucleotide for Identifying HPV 61

<400> 361
taaaatacat ttaacccctg aaa                                                23


<210> 362
<211> 19
<212> DNA
<213> Artificial Sequence

<220>
<223> Description of Artificial Sequence:
      Oligonucleotide for Identifying HPV 61

<400> 362
taaggccttg ttggatgac                                                     19


<210> 363
<211> 26
<212> DNA

<213> Artificial Sequence

<220>
<223> Description of Artificial Sequence:
      Oligonucleotide for Identifying HPV 61

<400> 363
tgtggtacca ccaccctcta ccagtt                                          26


<210> 364
<211> 22
<212> DNA
<213> Artificial Sequence

<220>
<223> Description of Artificial Sequence:
      Oligonucleotide for Identifying HPV 61

<400> 364
atataggttt ttgcagtcca ga                                              22


<210> 365
<211> 22
<212> DNA
<213> Artificial Sequence

<220>
<223> Description of Artificial Sequence:
      Oligonucleotide for Identifying HPV 61

<400> 365
gggtgctgct gccccgccgc cc                                              22


<210> 366
<211> 16
<212> DNA
<213> Artificial Sequence

<220>
<223> Description of Artificial Sequence:
      Oligonucleotide for Identifying HPV 61

<400> 366
ctatgccaag ttatcc                                                     16


<210> 367
<211> 23
<212> DNA
<213> Artificial Sequence

<220>
<223> Description of Artificial Sequence:
      Oligonucleotide for Identifying HPV 61

<400> 367
tgcacaggat gacgctgcaa cgg                                             23


<210> 368

<211> 26
<212> DNA
<213> Artificial Sequence

<220>
<223> Description of Artificial Sequence:
      Oligonucleotide for Identifying HPV 61

<400> 368
ccttggtgga cagtgaatta agtccc                                          26


<210> 369
<211> 30
<212> DNA
<213> Artificial Sequence

<220>
<223> Description of Artificial Sequence:
      Oligonucleotide for Identifying HPV 61

<400> 369
ggacaggaca gggctaggag aaggctgttt                                      30


<210> 370
<211> 20
<212> DNA
<213> Artificial Sequence

<220>
<223> Description of Artificial Sequence:
      Oligonucleotide for Identifying HPV 61

<400> 370
tgtttgagca agatagtggc                                                 20


<210> 371
<211> 26
<212> DNA
<213> Artificial Sequence

<220>
<223> Description of Artificial Sequence:
      Oligonucleotide for Identifying HPV 61

<400> 371
ggatgcgcaa catgaggggg gggggg                                          26


<210> 372
<211> 26
<212> DNA
<213> Artificial Sequence

<220>
<223> Description of Artificial Sequence:
      Oligonucleotide for Identifying HPV 61

<400> 372
ggccgaggcc acaggtaacc aggaaa                                          26

<210> 373
<211> 26
<212> DNA
<213> Artificial Sequence

<220>
<223> Description of Artificial Sequence:
      Oligonucleotide for Identifying HPV 61

<400> 373
ggcagacata ttagaggtgt ttaagg                                          26


<210> 374
<211> 26
<212> DNA
<213> Artificial Sequence

<220>
<223> Description of Artificial Sequence:
      Oligonucleotide for Identifying HPV 61

<400> 374
ctgtacaaat tcaaggacct atttgg                                          26


<210> 375
<211> 21
<212> DNA
<213> Artificial Sequence

<220>
<223> Description of Artificial Sequence:
      Oligonucleotide for Identifying HPV 61

<400> 375
ctagcatttg gggagctggt a                                               21


<210> 376
<211> 15
<212> DNA
<213> Artificial Sequence

<220>
<223> Description of Artificial Sequence:
      Oligonucleotide for Identifying HPV 62

<400> 376
ccgcctccac tgctg                                                      15


<210> 377
<211> 20
<212> DNA
<213> Artificial Sequence

<220>
<223> Description of Artificial Sequence:
      Oligonucleotide for Identifying HPV 62

<400> 377

gcctccactg ctgcagcaga                                                    20

<210> 378
<211> 20
<212> DNA
<213> Artificial Sequence

<220>
<223> Description of Artificial Sequence:
      Oligonucleotide for Identifying HPV 62

<400> 378
ctgctgcagc agaatacacg                                                    20

<210> 379
<211> 20
<212> DNA
<213> Artificial Sequence

<220>
<223> Description of Artificial Sequence:
      Oligonucleotide for Identifying HPV 62

<400> 379
gcagaataca cggctaccaa                                                    20

<210> 380
<211> 20
<212> DNA
<213> Artificial Sequence

<220>
<223> Description of Artificial Sequence:
      Oligonucleotide for Identifying HPV 62

<400> 380
cagaatacac ggctaccaac                                                    20

<210> 381
<211> 23
<212> DNA
<213> Artificial Sequence

<220>
<223> Description of Artificial Sequence:
      Oligonucleotide for Identifying HPV 62

<400> 381
caaaatacag ttaacccccg aaa                                                23

<210> 382
<211> 19
<212> DNA
<213> Artificial Sequence

<220>
<223> Description of Artificial Sequence:
      Oligonucleotide for Identifying HPV 62

<400> 382
caaggacctt ttggatgac                                                    19


<210> 383
<211> 26
<212> DNA
<213> Artificial Sequence

<220>
<223> Description of Artificial Sequence:
      Oligonucleotide for Identifying HPV 62

<400> 383
ggttttacct ccccttcca ctagtt                                             26


<210> 384
<211> 22
<212> DNA
<213> Artificial Sequence

<220>
<223> Description of Artificial Sequence:
      Oligonucleotide for Identifying HPV 62

<400> 384
atatcactat ttcgagtctc gg                                                22


<210> 385
<211> 19
<212> DNA
<213> Artificial Sequence

<220>
<223> Description of Artificial Sequence:
      Oligonucleotide for Identifying HPV 62

<400> 385
ggggctgcct acccgtccc                                                    19


<210> 386
<211> 16
<212> DNA
<213> Artificial Sequence

<220>
<223> Description of Artificial Sequence:
      Oligonucleotide for Identifying HPV 62

<400> 386
gtatgcgcaa atgaca                                                       16


<210> 387
<211> 15
<212> DNA
<213> Artificial Sequence

<220>

<223> Description of Artificial Sequence:
      Oligonucleotide for Identifying HPV 66

<400> 387
cagctaaaag cacat                                                        15


<210> 388
<211> 20
<212> DNA
<213> Artificial Sequence

<220>
<223> Description of Artificial Sequence:
      Oligonucleotide for Identifying HPV 66

<400> 388
cagctaaaag cacattaact                                                   20


<210> 389
<211> 20
<212> DNA
<213> Artificial Sequence

<220>
<223> Description of Artificial Sequence:
      Oligonucleotide for Identifying HPV 66

<400> 389
ctaaaagcac attaactaaa                                                   20


<210> 390
<211> 20
<212> DNA
<213> Artificial Sequence

<220>
<223> Description of Artificial Sequence:
      Oligonucleotide for Identifying HPV 66

<400> 390
ttaactaaat atgatgcccg                                                   20


<210> 391
<211> 20
<212> DNA
<213> Artificial Sequence

<220>
<223> Description of Artificial Sequence:
      Oligonucleotide for Identifying HPV 66

<400> 391
ctaaatatga tgcccgtgaa                                                   20


<210> 392
<211> 23
<212> DNA
<213> Artificial Sequence

<220>
<223> Description of Artificial Sequence:
      Oligonucleotide for Identifying HPV 66

<400> 392
taaaataacc ttaactgcag aag                                              23


<210> 393
<211> 19
<212> DNA
<213> Artificial Sequence

<220>
<223> Description of Artificial Sequence:
      Oligonucleotide for Identifying HPV 66

<400> 393
taatacttta ttagacgat                                                   19


<210> 394
<211> 26
<212> DNA
<213> Artificial Sequence

<220>
<223> Description of Artificial Sequence:
      Oligonucleotide for Identifying HPV 66

<400> 394
cttatcccca ccagttgcaa ctagct                                           26


<210> 395
<211> 22
<212> DNA
<213> Artificial Sequence

<220>
<223> Description of Artificial Sequence:
      Oligonucleotide for Identifying HPV 66

<400> 395
atataggtat attaaaagca ca                                               22


<210> 396
<211> 19
<212> DNA
<213> Artificial Sequence

<220>
<223> Description of Artificial Sequence:
      Oligonucleotide for Identifying HPV 66

<400> 396
ggaacagccc cctgcagaa                                                   19


<210> 397
<211> 16

<212> DNA
<213> Artificial Sequence

<220>
<223> Description of Artificial Sequence:
      Oligonucleotide for Identifying HPV 66

<400> 397
cctggctaaa tataag                                                    16


<210> 398
<211> 22
<212> DNA
<213> Artificial Sequence

<220>
<223> Description of Artificial Sequence:
      Oligonucleotide for Identifying HPV 66

<400> 398
agcacatgca gatgcacaga cg                                             22


<210> 399
<211> 26
<212> DNA
<213> Artificial Sequence

<220>
<223> Description of Artificial Sequence:
      Oligonucleotide for Identifying HPV 66

<400> 399
ggtagtccct taagtgatat tagtaa                                         26


<210> 400
<211> 26
<212> DNA
<213> Artificial Sequence

<220>
<223> Description of Artificial Sequence:
      Oligonucleotide for Identifying HPV 66

<400> 400
gcaaactgtg taccgagagg aagtaa                                         26


<210> 401
<211> 30
<212> DNA
<213> Artificial Sequence

<220>
<223> Description of Artificial Sequence:
      Oligonucleotide for Identifying HPV 66

<400> 401
aaggctaata ttatcagaag acagcgggta                                     30

<210> 402
<211> 25
<212> DNA
<213> Artificial Sequence

<220>
<223> Description of Artificial Sequence:
      Oligonucleotide for Identifying HPV 66

<400> 402
gaaacatcac aacaggtaga atacg                                          25


<210> 403
<211> 26
<212> DNA
<213> Artificial Sequence

<220>
<223> Description of Artificial Sequence:
      Oligonucleotide for Identifying HPV 66

<400> 403
gagctcacaa aatggaggct cgcaaa                                         26


<210> 404
<211> 26
<212> DNA
<213> Artificial Sequence

<220>
<223> Description of Artificial Sequence:
      Oligonucleotide for Identifying HPV 66

<400> 404
atcaaatatg gatatagata caaata                                         26


<210> 405
<211> 26
<212> DNA
<213> Artificial Sequence

<220>
<223> Description of Artificial Sequence:
      Oligonucleotide for Identifying HPV 66

<400> 405
ccaattgcag gaactattta aaagta                                         26


<210> 406
<211> 26
<212> DNA
<213> Artificial Sequence

<220>
<223> Description of Artificial Sequence:
      Oligonucleotide for Identifying HPV 66

<400> 406
caaggaagat tacattttaa atttaa                                         26

<210> 407
<211> 20
<212> DNA
<213> Artificial Sequence

<220>
<223> Description of Artificial Sequence:
      Oligonucleotide for Identifying HPV 66

<400> 407
agaagtgtat ggagtgccat                                                    20


<210> 408
<211> 15
<212> DNA
<213> Artificial Sequence

<220>
<223> Description of Artificial Sequence:
      Oligonucleotide for Identifying HPV 67

<400> 408
ctgaggaaaa atcag                                                         15


<210> 409
<211> 20
<212> DNA
<213> Artificial Sequence

<220>
<223> Description of Artificial Sequence:
      Oligonucleotide for Identifying HPV 67

<400> 409
gaggaaaaat cagaggctac                                                    20


<210> 410
<211> 23
<212> DNA
<213> Artificial Sequence

<220>
<223> Description of Artificial Sequence:
      Oligonucleotide for Identifying HPV 67

<400> 410
atcagaggct acatacaaaa atg                                                23


<210> 411
<211> 20
<212> DNA
<213> Artificial Sequence

<220>
<223> Description of Artificial Sequence:
      Oligonucleotide for Identifying HPV 67

<400> 411
aggaaaaatc agaggctaca                                                    20


<210> 412
<211> 20
<212> DNA
<213> Artificial Sequence

<220>
<223> Description of Artificial Sequence:
      Oligonucleotide for Identifying HPV 67

<400> 412
ctacatacaa aaatgaaaac                                                    20


<210> 413
<211> 23
<212> DNA
<213> Artificial Sequence

<220>
<223> Description of Artificial Sequence:
      Oligonucleotide for Identifying HPV 67

<400> 413
caaaatatcc cttactgcaa atg                                                23


<210> 414
<211> 19
<212> DNA
<213> Artificial Sequence

<220>
<223> Description of Artificial Sequence:
      Oligonucleotide for Identifying HPV 67

<400> 414
tccagatata ttagaggac                                                     19


<210> 415
<211> 26
<212> DNA
<213> Artificial Sequence

<220>
<223> Description of Artificial Sequence:
      Oligonucleotide for Identifying HPV 67

<400> 415
ccttacacca cctccttcag gtaatt                                             26


<210> 416
<211> 22
<212> DNA
<213> Artificial Sequence

<220>
<223> Description of Artificial Sequence:

Oligonucleotide for Identifying HPV 67

<400> 416
atatagattt gttacctcgc ag                                                22


<210> 417
<211> 19
<212> DNA
<213> Artificial Sequence

<220>
<223> Description of Artificial Sequence:
      Oligonucleotide for Identifying HPV 67

<400> 417
aacatcccct ccaacagca                                                    19


<210> 418
<211> 16
<212> DNA
<213> Artificial Sequence

<220>
<223> Description of Artificial Sequence:
      Oligonucleotide for Identifying HPV 67

<400> 418
tcttaaaaag tacagt                                                       16


<210> 419
<211> 23
<212> DNA
<213> Artificial Sequence

<220>
<223> Description of Artificial Sequence:
      Oligonucleotide for Identifying HPV 67

<400> 419
agaggaggat gacctaaacg ctg                                               23


<210> 420
<211> 26
<212> DNA
<213> Artificial Sequence

<220>
<223> Description of Artificial Sequence:
      Oligonucleotide for Identifying HPV 67

<400> 420
caggcatgtg gtggtaatag taatgg                                            26


<210> 421
<211> 30
<212> DNA
<213> Artificial Sequence

<220>
<223> Description of Artificial Sequence:
      Oligonucleotide for Identifying HPV 67

<400> 421
gccgcaaaac gcagagcata cgacatagaa                                    30


<210> 422
<211> 26
<212> DNA
<213> Artificial Sequence

<220>
<223> Description of Artificial Sequence:
      Oligonucleotide for Identifying HPV 67

<400> 422
ggcaaaatgg cgatatgcag tgcagt                                        26


<210> 423
<211> 26
<212> DNA
<213> Artificial Sequence

<220>
<223> Description of Artificial Sequence:
      Oligonucleotide for Identifying HPV 67

<400> 423
gcaagtagta cgggaaacag tgtaga                                        26


<210> 424
<211> 26
<212> DNA
<213> Artificial Sequence

<220>
<223> Description of Artificial Sequence:
      Oligonucleotide for Identifying HPV 67

<400> 424
gtcaggaaca aagcatgcca ttgcaa                                        26


<210> 425
<211> 25
<212> DNA
<213> Artificial Sequence

<220>
<223> Description of Artificial Sequence:
      Oligonucleotide for Identifying HPV 67

<400> 425
gcatgtaaat aatataaagg caacg                                         25


<210> 426
<211> 26
<212> DNA

<213> Artificial Sequence

<220>
<223> Description of Artificial Sequence:
      Oligonucleotide for Identifying HPV 67

<400> 426
ggaagcatat ggggtaacgt ttacac                                          26


<210> 427
<211> 15
<212> DNA
<213> Artificial Sequence

<220>
<223> Description of Artificial Sequence:
      Oligonucleotide for Identifying HPV 68

<400> 427
ctactactga atcag                                                      15


<210> 428
<211> 20
<212> DNA
<213> Artificial Sequence

<220>
<223> Description of Artificial Sequence:
      Oligonucleotide for Identifying HPV 68

<400> 428
tgaatcagct gtaccaaata                                                 20


<210> 429
<211> 20
<212> DNA
<213> Artificial Sequence

<220>
<223> Description of Artificial Sequence:
      Oligonucleotide for Identifying HPV 68

<400> 429
gaatcagctg taccaaatat                                                 20


<210> 430
<211> 22
<212> DNA
<213> Artificial Sequence

<220>
<223> Description of Artificial Sequence:
      Oligonucleotide for Identifying HPV 68

<400> 430
cagctgtacc aaatatttat ga                                              22


<210> 431

<211> 20
<212> DNA
<213> Artificial Sequence

<220>
<223> Description of Artificial Sequence:
      Oligonucleotide for Identifying HPV 68

<400> 431
atatttatga tcctaataaa                                                  20


<210> 432
<211> 19
<212> DNA
<213> Artificial Sequence

<220>
<223> Description of Artificial Sequence:
      Oligonucleotide for Identifying HPV 68

<400> 432
tcctgctatt ttggatgat                                                   19


<210> 433
<211> 23
<212> DNA
<213> Artificial Sequence

<220>
<223> Description of Artificial Sequence:
      Oligonucleotide for Identifying HPV 68

<400> 433
tactataaca ttgtccactg atg                                              23


<210> 434
<211> 26
<212> DNA
<213> Artificial Sequence

<220>
<223> Description of Artificial Sequence:
      Oligonucleotide for Identifying HPV 68

<400> 434
tgttgcccct ccaccatctg ctagtc                                           26


<210> 435
<211> 22
<212> DNA
<213> Artificial Sequence

<220>
<223> Description of Artificial Sequence:
      Oligonucleotide for Identifying HPV 68

<400> 435
ataccgctat ctgcaatcag ca                                               22

<210> 436
<211> 19
<212> DNA
<213> Artificial Sequence

<220>
<223> Description of Artificial Sequence:
      Oligonucleotide for Identifying HPV 68

<400> 436
agacgcccct gcacctact                                                    19


<210> 437
<211> 16
<212> DNA
<213> Artificial Sequence

<220>
<223> Description of Artificial Sequence:
      Oligonucleotide for Identifying HPV 68

<400> 437
atatgatggc ttaaac                                                       16


<210> 438
<211> 23
<212> DNA
<213> Artificial Sequence

<220>
<223> Description of Artificial Sequence:
      Oligonucleotide for Identifying HPV 68

<400> 438
ggcccaaagg gatgcacaaa cag                                               23


<210> 439
<211> 26
<212> DNA
<213> Artificial Sequence

<220>
<223> Description of Artificial Sequence:
      Oligonucleotide for Identifying HPV 68

<400> 439
gccctttagc aaagtcgcca ttacag                                            26


<210> 440
<211> 20
<212> DNA
<213> Artificial Sequence

<220>
<223> Description of Artificial Sequence:
      Oligonucleotide for Identifying HPV 68

<400> 440

ggtaactgta gcaactaata                                                    20

<210> 441
<211> 30
<212> DNA
<213> Artificial Sequence

<220>
<223> Description of Artificial Sequence:
      Oligonucleotide for Identifying HPV 68

<400> 441
ggaaaatggc gacagcatac gggaggactg                                          30

<210> 442
<211> 26
<212> DNA
<213> Artificial Sequence

<220>
<223> Description of Artificial Sequence:
      Oligonucleotide for Identifying HPV 68

<400> 442
gacagtgcta tagatagtga aaacca                                              26

<210> 443
<211> 24
<212> DNA
<213> Artificial Sequence

<220>
<223> Description of Artificial Sequence:
      Oligonucleotide for Identifying HPV 68

<400> 443
cctactacgc aactaaaagt atta                                                24

<210> 444
<211> 26
<212> DNA
<213> Artificial Sequence

<220>
<223> Description of Artificial Sequence:
      Oligonucleotide for Identifying HPV 68

<400> 444
aagctgcaat gttaacagaa tttaaa                                              26

<210> 445
<211> 20
<212> DNA
<213> Artificial Sequence

<220>
<223> Description of Artificial Sequence:
      Oligonucleotide for Identifying HPV 69

<400> 445
tattagtact gtatctgcac                                                    20


<210> 446
<211> 15
<212> DNA
<213> Artificial Sequence

<220>
<223> Description of Artificial Sequence:
      Oligonucleotide for Identifying HPV 69

<400> 446
ctgtatctgc acaat                                                         15


<210> 447
<211> 20
<212> DNA
<213> Artificial Sequence

<220>
<223> Description of Artificial Sequence:
      Oligonucleotide for Identifying HPV 69

<400> 447
ctgtatctgc acaatctgca                                                    20


<210> 448
<211> 20
<212> DNA
<213> Artificial Sequence

<220>
<223> Description of Artificial Sequence:
      Oligonucleotide for Identifying HPV 69

<400> 448
tgcacaatct gcatctgcca                                                    20


<210> 449
<211> 22
<212> DNA
<213> Artificial Sequence

<220>
<223> Description of Artificial Sequence:
      Oligonucleotide for Identifying HPV 69

<400> 449
caatctgcat ctgccacttt ta                                                 22


<210> 450
<211> 20
<212> DNA
<213> Artificial Sequence

<220>

<223> Description of Artificial Sequence:
      Oligonucleotide for Identifying HPV 69

<400> 450
ccacttttaa accatcagat                                                20


<210> 451
<211> 23
<212> DNA
<213> Artificial Sequence

<220>
<223> Description of Artificial Sequence:
      Oligonucleotide for Identifying HPV 69

<400> 451
taaaattact cttaccactg atg                                            23


<210> 452
<211> 19
<212> DNA
<213> Artificial Sequence

<220>
<223> Description of Artificial Sequence:
      Oligonucleotide for Identifying HPV 69

<400> 452
ttctactatt ttggaaaat                                                 19


<210> 453
<211> 26
<212> DNA
<213> Artificial Sequence

<220>
<223> Description of Artificial Sequence:
      Oligonucleotide for Identifying HPV 69

<400> 453
ccttaccttg cctcctactg ctagtt                                         26


<210> 454
<211> 22
<212> DNA
<213> Artificial Sequence

<220>
<223> Description of Artificial Sequence:
      Oligonucleotide for Identifying HPV 69

<400> 454
atataggttt attaaaaatt ca                                             22


<210> 455
<211> 19
<212> DNA
<213> Artificial Sequence

<220>
<223> Description of Artificial Sequence:
      Oligonucleotide for Identifying HPV 69

<400> 455
cgatgcccct gcacagccc                                                    19


<210> 456
<211> 23
<212> DNA
<213> Artificial Sequence

<220>
<223> Description of Artificial Sequence:
      Oligonucleotide for Identifying HPV 69

<400> 456
aacacaagca aataagaagg cag                                               23


<210> 457
<211> 26
<212> DNA
<213> Artificial Sequence

<220>
<223> Description of Artificial Sequence:
      Oligonucleotide for Identifying HPV 69

<400> 457
gaacagcccg ttgcaagaca taacaa                                            26


<210> 458
<211> 25
<212> DNA
<213> Artificial Sequence

<220>
<223> Description of Artificial Sequence:
      Oligonucleotide for Identifying HPV 69

<400> 458
cagacgaagt aaacaattta caggc                                             25


<210> 459
<211> 30
<212> DNA
<213> Artificial Sequence

<220>
<223> Description of Artificial Sequence:
      Oligonucleotide for Identifying HPV 69

<400> 459
ggagagcagt ggacagtgtt ccggacagcg                                        30


<210> 460
<211> 26

<212> DNA
<213> Artificial Sequence

<220>
<223> Description of Artificial Sequence:
      Oligonucleotide for Identifying HPV 69

<400> 460
ggtagataaa cacaatgaac aaaatg                                          26


<210> 461
<211> 22
<212> DNA
<213> Artificial Sequence

<220>
<223> Description of Artificial Sequence:
      Oligonucleotide for Identifying HPV 69

<400> 461
tcaagtggat ctgtatcaga ca                                              22


<210> 462
<211> 25
<212> DNA
<213> Artificial Sequence

<220>
<223> Description of Artificial Sequence:
      Oligonucleotide for Identifying HPV 69

<400> 462
gcacaggcaa gtagtgtaac caaaa                                           25


<210> 463
<211> 24
<212> DNA
<213> Artificial Sequence

<220>
<223> Description of Artificial Sequence:
      Oligonucleotide for Identifying HPV 69

<400> 463
gtaatgtaaa agcagcatta ttaa                                            24


<210> 464
<211> 21
<212> DNA
<213> Artificial Sequence

<220>
<223> Description of Artificial Sequence:
      Oligonucleotide for Identifying HPV 69

<400> 464
cagtatatgg tgtaagttat a                                               21

<210> 465
<211> 20
<212> DNA
<213> Artificial Sequence

<220>
<223> Description of Artificial Sequence:
      Oligonucleotide for Identifying HPV 6

<400> 465
catccgtaac tacatcttcc                                                    20


<210> 466
<211> 20
<212> DNA
<213> Artificial Sequence

<220>
<223> Description of Artificial Sequence:
      Oligonucleotide for Identifying HPV 6

<400> 466
atccgtaact acatcttcca                                                    20


<210> 467
<211> 22
<212> DNA
<213> Artificial Sequence

<220>
<223> Description of Artificial Sequence:
      Oligonucleotide for Identifying HPV 6

<400> 467
ctacatcttc cacatacacc aa                                                 22


<210> 468
<211> 20
<212> DNA
<213> Artificial Sequence

<220>
<223> Description of Artificial Sequence:
      Oligonucleotide for Identifying HPV 6

<400> 468
catcttccac atacaccaat                                                    20


<210> 469
<211> 20
<212> DNA
<213> Artificial Sequence

<220>
<223> Description of Artificial Sequence:
      Oligonucleotide for Identifying HPV 6

<400> 469
atcttccaca tacaccaatt                                                    20

<210> 470
<211> 20
<212> DNA
<213> Artificial Sequence

<220>
<223> Description of Artificial Sequence:
      Oligonucleotide for Identifying HPV 6

<400> 470
ccacatacac caattctgat                                                    20


<210> 471
<211> 23
<212> DNA
<213> Artificial Sequence

<220>
<223> Description of Artificial Sequence:
      Oligonucleotide for Identifying HPV 6

<400> 471
tagcattaca ttgtctgctg aag                                                23


<210> 472
<211> 19
<212> DNA
<213> Artificial Sequence

<220>
<223> Description of Artificial Sequence:
      Oligonucleotide for Identifying HPV 6

<400> 472
tccctctgtt ttggaagac                                                     19


<210> 473
<211> 26
<212> DNA
<213> Artificial Sequence

<220>
<223> Description of Artificial Sequence:
      Oligonucleotide for Identifying HPV 6

<400> 473
gttatcgcct cccccaaatg gtacat                                             26


<210> 474
<211> 22
<212> DNA
<213> Artificial Sequence

<220>
<223> Description of Artificial Sequence:
      Oligonucleotide for Identifying HPV 6

<400> 474
ctataggtat gtgcagtcac ag                                                22

<210> 475
<211> 19
<212> DNA
<213> Artificial Sequence

<220>
<223> Description of Artificial Sequence:
      Oligonucleotide for Identifying HPV 6

<400> 475
gcccactcct gaaaaggaa                                                    19

<210> 476
<211> 16
<212> DNA
<213> Artificial Sequence

<220>
<223> Description of Artificial Sequence:
      Oligonucleotide for Identifying HPV 6

<400> 476
ctataagaac cttagt                                                       16

<210> 477
<211> 23
<212> DNA
<213> Artificial Sequence

<220>
<223> Description of Artificial Sequence:
      Oligonucleotide for Identifying HPV 6

<400> 477
ggcggacacc cattatgcga ctg                                               23

<210> 478
<211> 26
<212> DNA
<213> Artificial Sequence

<220>
<223> Description of Artificial Sequence:
      Oligonucleotide for Identifying HPV 6

<400> 478
gttagtccta taaacactat agccga                                            26

<210> 479
<211> 30
<212> DNA
<213> Artificial Sequence

<220>
<223> Description of Artificial Sequence:

Oligonucleotide for Identifying HPV 6

<400> 479
ccacgattgg acgccattaa acttacaaga                                        30


<210> 480
<211> 26
<212> DNA
<213> Artificial Sequence

<220>
<223> Description of Artificial Sequence:
      Oligonucleotide for Identifying HPV 6

<400> 480
ggctgtttca aaccagggaa ctaacg                                            26


<210> 481
<211> 26
<212> DNA
<213> Artificial Sequence

<220>
<223> Description of Artificial Sequence:
      Oligonucleotide for Identifying HPV 6

<400> 481
ggtagagaaa catggcgtac cggaaa                                            26


<210> 482
<211> 26
<212> DNA
<213> Artificial Sequence

<220>
<223> Description of Artificial Sequence:
      Oligonucleotide for Identifying HPV 6

<400> 482
ggacacagga agggacatag aggggg                                            26


<210> 483
<211> 26
<212> DNA
<213> Artificial Sequence

<220>
<223> Description of Artificial Sequence:
      Oligonucleotide for Identifying HPV 6

<400> 483
cacaaacagt gtacgggagc atgcag                                            26


<210> 484
<211> 26
<212> DNA
<213> Artificial Sequence

<220>
<223> Description of Artificial Sequence:
      Oligonucleotide for Identifying HPV 6

<400> 484
gctaaaatgt aaagatttac gggcag                                          26


<210> 485
<211> 26
<212> DNA
<213> Artificial Sequence

<220>
<223> Description of Artificial Sequence:
      Oligonucleotide for Identifying HPV 6

<400> 485
gctttgggct gtcttttata gattta                                          26


<210> 486
<211> 20
<212> DNA
<213> Artificial Sequence

<220>
<223> Description of Artificial Sequence:
      Oligonucleotide for Identifying HPV  70

<400> 486
tgtctgcctg caccgaaacg                                                 20


<210> 487
<211> 15
<212> DNA
<213> Artificial Sequence

<220>
<223> Description of Artificial Sequence:
      Oligonucleotide for Identifying HPV  70

<400> 487
ctgcaccgaa acggc                                                      15


<210> 488
<211> 20
<212> DNA
<213> Artificial Sequence

<220>
<223> Description of Artificial Sequence:
      Oligonucleotide for Identifying HPV  70

<400> 488
gaaacggcca tacctgctgt                                                 20


<210> 489
<211> 20
<212> DNA

<213> Artificial Sequence

<220>
<223> Description of Artificial Sequence:
      Oligonucleotide for Identifying HPV  70

<400> 489
cgaaacggcc atacctgctg                                                    20


<210> 490
<211> 22
<212> DNA
<213> Artificial Sequence

<220>
<223> Description of Artificial Sequence:
      Oligonucleotide for Identifying HPV  70

<400> 490
cggccatacc tgctgtatat ag                                                 22


<210> 491
<211> 20
<212> DNA
<213> Artificial Sequence

<220>
<223> Description of Artificial Sequence:
      Oligonucleotide for Identifying HPV  70

<400> 491
ctgtatatag ccctacaaag                                                    20


<210> 492
<211> 23
<212> DNA
<213> Artificial Sequence

<220>
<223> Description of Artificial Sequence:
      Oligonucleotide for Identifying HPV  70

<400> 492
tactatcaca ttaactgctg acg                                                23


<210> 493
<211> 19
<212> DNA
<213> Artificial Sequence

<220>
<223> Description of Artificial Sequence:
      Oligonucleotide for Identifying HPV  70

<400> 493
tcctgcaatt ttggacaat                                                     19


<210> 494

<211> 26
<212> DNA
<213> Artificial Sequence

<220>
<223> Description of Artificial Sequence:
      Oligonucleotide for Identifying HPV  70

<400> 494
agttacccct ccaccatctg caagct                                          26


<210> 495
<211> 22
<212> DNA
<213> Artificial Sequence

<220>
<223> Description of Artificial Sequence:
      Oligonucleotide for Identifying HPV  70

<400> 495
gtataggtat ttacaatcag ca                                              22


<210> 496
<211> 19
<212> DNA
<213> Artificial Sequence

<220>
<223> Description of Artificial Sequence:
      Oligonucleotide for Identifying HPV  70

<400> 496
ggatgctcct acacctgaa                                                  19


<210> 497
<211> 16
<212> DNA
<213> Artificial Sequence

<220>
<223> Description of Artificial Sequence:
      Oligonucleotide for Identifying HPV  70

<400> 497
ctatgacgat ttaaaa                                                     16


<210> 498
<211> 23
<212> DNA
<213> Artificial Sequence

<220>
<223> Description of Artificial Sequence:
      Oligonucleotide for Identifying HPV  70

<400> 498
ggcccaaagg gatgcacaat cag                                             23

<210> 499
<211> 23
<212> DNA
<213> Artificial Sequence

<220>
<223> Description of Artificial Sequence:
      Oligonucleotide for Identifying HPV  70

<400> 499
gcaatctaaa taaaagtcct tgt                                              23


<210> 500
<211> 25
<212> DNA
<213> Artificial Sequence

<220>
<223> Description of Artificial Sequence:
      Oligonucleotide for Identifying HPV  70

<400> 500
gtacataggg aacaaagggt aacac                                            25


<210> 501
<211> 23
<212> DNA
<213> Artificial Sequence

<220>
<223> Description of Artificial Sequence:
      Oligonucleotide for Identifying HPV  70

<400> 501
ggtaaacata tgcaataaac agg                                              23


<210> 502
<211> 30
<212> DNA
<213> Artificial Sequence

<220>
<223> Description of Artificial Sequence:
      Oligonucleotide for Identifying HPV  70

<400> 502
acaaacgtgt attcagtacc agacagcggc                                       30


<210> 503
<211> 26
<212> DNA
<213> Artificial Sequence

<220>
<223> Description of Artificial Sequence:
      Oligonucleotide for Identifying HPV  70

<400> 503

gtagtaaata atacaaatgg ggaaga                                          26

<210> 504
<211> 26
<212> DNA
<213> Artificial Sequence

<220>
<223> Description of Artificial Sequence:
      Oligonucleotide for Identifying HPV  70

<400> 504
ggagtgcagt agtgtagaca gtgcta                                          26

<210> 505
<211> 26
<212> DNA
<213> Artificial Sequence

<220>
<223> Description of Artificial Sequence:
      Oligonucleotide for Identifying HPV  70

<400> 505
tccacagtca cctactgcac agctaa                                          26

<210> 506
<211> 25
<212> DNA
<213> Artificial Sequence

<220>
<223> Description of Artificial Sequence:
      Oligonucleotide for Identifying HPV  70

<400> 506
gctaataacc aaaaagccat actac                                           25

<210> 507
<211> 26
<212> DNA
<213> Artificial Sequence

<220>
<223> Description of Artificial Sequence:
      Oligonucleotide for Identifying HPV  70

<400> 507
cacacatatg gattagcatt taacga                                          26

<210> 508
<211> 20
<212> DNA
<213> Artificial Sequence

<220>
<223> Description of Artificial Sequence:
      Oligonucleotide for Identifying HPV 72

<400> 508
atctgttggt ttaatgagct                                                    20


<210> 509
<211> 20
<212> DNA
<213> Artificial Sequence

<220>
<223> Description of Artificial Sequence:
      Oligonucleotide for Identifying HPV 72

<400> 509
tttgtgacag ttgtagatac                                                    20


<210> 510
<211> 15
<212> DNA
<213> Artificial Sequence

<220>
<223> Description of Artificial Sequence:
      Oligonucleotide for Identifying HPV 72

<400> 510
ctgccacagc gtcct                                                         15


<210> 511
<211> 20
<212> DNA
<213> Artificial Sequence

<220>
<223> Description of Artificial Sequence:
      Oligonucleotide for Identifying HPV 72

<400> 511
acagcgtcct ctgtatcaga                                                    20


<210> 512
<211> 20
<212> DNA
<213> Artificial Sequence

<220>
<223> Description of Artificial Sequence:
      Oligonucleotide for Identifying HPV 72

<400> 512
ccacagcgtc ctctgtatca                                                    20


<210> 513
<211> 22
<212> DNA
<213> Artificial Sequence

<220>

<223> Description of Artificial Sequence:
      Oligonucleotide for Identifying HPV 72

<400> 513
agcgtcctct gtatcagaat at                                              22


<210> 514
<211> 20
<212> DNA
<213> Artificial Sequence

<220>
<223> Description of Artificial Sequence:
      Oligonucleotide for Identifying HPV 72

<400> 514
cagaatatac agcttctaat                                                 20


<210> 515
<211> 23
<212> DNA
<213> Artificial Sequence

<220>
<223> Description of Artificial Sequence:
      Oligonucleotide for Identifying HPV 72

<400> 515
taaaattcac ttaactcctg aaa                                             23


<210> 516
<211> 19
<212> DNA
<213> Artificial Sequence

<220>
<223> Description of Artificial Sequence:
      Oligonucleotide for Identifying HPV 72

<400> 516
taaggcctta ttggatgac                                                  19


<210> 517
<211> 26
<212> DNA
<213> Artificial Sequence

<220>
<223> Description of Artificial Sequence:
      Oligonucleotide for Identifying HPV 72

<400> 517
tgtggtgcct cctccttcta ccagtt                                          26


<210> 518
<211> 22
<212> DNA
<213> Artificial Sequence

<220>
<223> Description of Artificial Sequence:
      Oligonucleotide for Identifying HPV 72

<400> 518
ctataggttt ttgcagtctc gt                                                22


<210> 519
<211> 22
<212> DNA
<213> Artificial Sequence

<220>
<223> Description of Artificial Sequence:
      Oligonucleotide for Identifying HPV 72

<400> 519
gggggctgcc acccctcctc ct                                                22


<210> 520
<211> 16
<212> DNA
<213> Artificial Sequence

<220>
<223> Description of Artificial Sequence:
      Oligonucleotide for Identifying HPV 72

<400> 520
atatgctaac ttatcc                                                       16


<210> 521
<211> 15
<212> DNA
<213> Artificial Sequence

<220>
<223> Description of Artificial Sequence:
      Oligonucleotide for Identifying HPV 74

<400> 521
cctacctcac aatcg                                                        15


<210> 522
<211> 20
<212> DNA
<213> Artificial Sequence

<220>
<223> Description of Artificial Sequence:
      Oligonucleotide for Identifying HPV 74

<400> 522
ctcacaatcg ccttctgcta                                                   20


<210> 523
<211> 20

<212> DNA
<213> Artificial Sequence

<220>
<223> Description of Artificial Sequence:
      Oligonucleotide for Identifying HPV 74

<400> 523
acctcacaat cgccttctgc                                                    20


<210> 524
<211> 22
<212> DNA
<213> Artificial Sequence

<220>
<223> Description of Artificial Sequence:
      Oligonucleotide for Identifying HPV 74

<400> 524
caatcgcctt ctgctacata ta                                                 22


<210> 525
<211> 22
<212> DNA
<213> Artificial Sequence

<220>
<223> Description of Artificial Sequence:
      Oligonucleotide for Identifying HPV 74

<400> 525
acaatcgcct tctgctacat at                                                 22


<210> 526
<211> 20
<212> DNA
<213> Artificial Sequence

<220>
<223> Description of Artificial Sequence:
      Oligonucleotide for Identifying HPV 74

<400> 526
ctacatataa tagttcagac                                                    20


<210> 527
<211> 23
<212> DNA
<213> Artificial Sequence

<220>
<223> Description of Artificial Sequence:
      Oligonucleotide for Identifying HPV 74

<400> 527
tagtattaag ttaactgctg agg                                                23

<210> 528
<211> 19
<212> DNA
<213> Artificial Sequence

<220>
<223> Description of Artificial Sequence:
      Oligonucleotide for Identifying HPV 74

<400> 528
tcctacagtt ttagaagag                                                    19


<210> 529
<211> 26
<212> DNA
<213> Artificial Sequence

<220>
<223> Description of Artificial Sequence:
      Oligonucleotide for Identifying HPV 74

<400> 529
gctaacgcct ccccccaatg gtactt                                            26


<210> 530
<211> 22
<212> DNA
<213> Artificial Sequence

<220>
<223> Description of Artificial Sequence:
      Oligonucleotide for Identifying HPV 74

<400> 530
ctacagatat gtgcagtccc ag                                                22


<210> 531
<211> 19
<212> DNA
<213> Artificial Sequence

<220>
<223> Description of Artificial Sequence:
      Oligonucleotide for Identifying HPV 74

<400> 531
acctacgcct gataaagca                                                    19


<210> 532
<211> 16
<212> DNA
<213> Artificial Sequence

<220>
<223> Description of Artificial Sequence:
      Oligonucleotide for Identifying HPV 74

<400> 532
ctatgcaaat ttaagt                                                       16

<210> 533
<211> 15
<212> DNA
<213> Artificial Sequence

<220>
<223> Description of Artificial Sequence:
      Oligonucleotide for Identifying HPV 82

<400> 533
tgctgttact ccatc                                                    15


<210> 534
<211> 20
<212> DNA
<213> Artificial Sequence

<220>
<223> Description of Artificial Sequence:
      Oligonucleotide for Identifying HPV 82

<400> 534
tgctgttact ccatctgttg                                               20


<210> 535
<211> 20
<212> DNA
<213> Artificial Sequence

<220>
<223> Description of Artificial Sequence:
      Oligonucleotide for Identifying HPV 82

<400> 535
actccatctg ttgcacaaac                                               20


<210> 536
<211> 20
<212> DNA
<213> Artificial Sequence

<220>
<223> Description of Artificial Sequence:
      Oligonucleotide for Identifying HPV 82

<400> 536
aaacatttac tccagcaaac                                               20


<210> 537
<211> 23
<212> DNA
<213> Artificial Sequence

<220>
<223> Description of Artificial Sequence:
      Oligonucleotide for Identifying HPV 82

<400> 537
taaaatcact ttaactactg aaa                                              23

<210> 538
<211> 19
<212> DNA
<213> Artificial Sequence

<220>
<223> Description of Artificial Sequence:
      Oligonucleotide for Identifying HPV 82

<400> 538
ttctacaatt ttagaacag                                                   19

<210> 539
<211> 26
<212> DNA
<213> Artificial Sequence

<220>
<223> Description of Artificial Sequence:
      Oligonucleotide for Identifying HPV 82

<400> 539
attaacattg cccccctccg ctagtt                                           26

<210> 540
<211> 22
<212> DNA
<213> Artificial Sequence

<220>
<223> Description of Artificial Sequence:
      Oligonucleotide for Identifying HPV 82

<400> 540
ctatcgattt gtaaaaaatg ca                                               22

<210> 541
<211> 19
<212> DNA
<213> Artificial Sequence

<220>
<223> Description of Artificial Sequence:
      Oligonucleotide for Identifying HPV 82

<400> 541
ggacagtcct ccacaggct                                                   19

<210> 542
<211> 23
<212> DNA
<213> Artificial Sequence

<220>
<223> Description of Artificial Sequence:

Oligonucleotide for Identifying HPV 82

<400> 542
aacacaggca cacaaagagg ctg                                              23


<210> 543
<211> 25
<212> DNA
<213> Artificial Sequence

<220>
<223> Description of Artificial Sequence:
      Oligonucleotide for Identifying HPV 82

<400> 543
gcagcccatt aaaagacatt acaaa                                            25


<210> 544
<211> 30
<212> DNA
<213> Artificial Sequence

<220>
<223> Description of Artificial Sequence:
      Oligonucleotide for Identifying HPV 82

<400> 544
gtcagcaaca accaaaacag gcaaaccttc                                       30


<210> 545
<211> 27
<212> DNA
<213> Artificial Sequence

<220>
<223> Description of Artificial Sequence:
      Oligonucleotide for Identifying HPV 82

<400> 545
ggagattact ggacagttat ccggaca                                          27


<210> 546
<211> 26
<212> DNA
<213> Artificial Sequence

<220>
<223> Description of Artificial Sequence:
      Oligonucleotide for Identifying HPV 82

<400> 546
ccttacaggt agatgggcaa aatgac                                           26


<210> 547
<211> 26
<212> DNA
<213> Artificial Sequence

<220>
<223> Description of Artificial Sequence:
      Oligonucleotide for Identifying HPV 82

<400> 547
gagcagcgac agaagtacag agatag                                          26


<210> 548
<211> 25
<212> DNA
<213> Artificial Sequence

<220>
<223> Description of Artificial Sequence:
      Oligonucleotide for Identifying HPV 82

<400> 548
gctaccaatg taggactaaa cagta                                           25


<210> 549
<211> 26
<212> DNA
<213> Artificial Sequence

<220>
<223> Description of Artificial Sequence:
      Oligonucleotide for Identifying HPV 82

<400> 549
gtagcaatgc aaaagcaatg tttatg                                          26


<210> 550
<211> 26
<212> DNA
<213> Artificial Sequence

<220>
<223> Description of Artificial Sequence:
      Oligonucleotide for Identifying HPV 82

<400> 550
ggtgttagtt ataatgagtt ggtaag                                          26


<210> 551
<211> 20
<212> DNA
<213> Artificial Sequence

<220>
<223> Description of Artificial Sequence:
      Oligonucleotide for Identifying HPV CP8061

<400> 551
tctgtgctac caaaactgtt                                                 20


<210> 552
<211> 15
<212> DNA

<213> Artificial Sequence

<220>
<223> Description of Artificial Sequence:
      Oligonucleotide for Identifying HPV CP8061

<400> 552
ctaccaaaac tgttg                                                        15


<210> 553
<211> 20
<212> DNA
<213> Artificial Sequence

<220>
<223> Description of Artificial Sequence:
      Oligonucleotide for Identifying HPV CP8061

<400> 553
accaaaactg ttgagtctac                                                   20


<210> 554
<211> 22
<212> DNA
<213> Artificial Sequence

<220>
<223> Description of Artificial Sequence:
      Oligonucleotide for Identifying HPV CP8061

<400> 554
aactgttgag tctacatata aa                                                22


<210> 555
<211> 20
<212> DNA
<213> Artificial Sequence

<220>
<223> Description of Artificial Sequence:
      Oligonucleotide for Identifying HPV CP8061

<400> 555
gttgagtcta catataaagc                                                   20


<210> 556
<211> 20
<212> DNA
<213> Artificial Sequence

<220>
<223> Description of Artificial Sequence:
      Oligonucleotide for Identifying HPV CP8061

<400> 556
ctacatataa agcctctagt                                                   20


<210> 557

<211> 23
<212> DNA
<213> Artificial Sequence

<220>
<223> Description of Artificial Sequence:
      Oligonucleotide for Identifying HPV CP8061

<400> 557
tgttattaat ttaacagctg aaa                                              23


<210> 558
<211> 19
<212> DNA
<213> Artificial Sequence

<220>
<223> Description of Artificial Sequence:
      Oligonucleotide for Identifying HPV CP8061

<400> 558
tgctacatta ctggaggac                                                   19


<210> 559
<211> 20
<212> DNA
<213> Artificial Sequence

<220>
<223> Description of Artificial Sequence:
      Oligonucleotide for Identifying HPV CP8061

<400> 559
gttcttacca cctcctactg                                                  20


<210> 560
<211> 22
<212> DNA
<213> Artificial Sequence

<220>
<223> Description of Artificial Sequence:
      Oligonucleotide for Identifying HPV CP8061

<400> 560
ctaccgcttt ttacagtctc ag                                               22


<210> 561
<211> 22
<212> DNA
<213> Artificial Sequence

<220>
<223> Description of Artificial Sequence:
      Oligonucleotide for Identifying HPV CP8061

<400> 561
aaacagtcct cctcctgcag aa                                               22

<210> 562
<211> 16
<212> DNA
<213> Artificial Sequence

<220>
<223> Description of Artificial Sequence:
      Oligonucleotide for Identifying HPV CP8061

<400> 562
ctatgcagat cttaca                                                    16


<210> 563
<211> 15
<212> DNA
<213> Artificial Sequence

<220>
<223> Description of Artificial Sequence:
      Oligonucleotide for Identifying HPV CP8034

<400> 563
cagctacatc tgctg                                                     15


<210> 564
<211> 20
<212> DNA
<213> Artificial Sequence

<220>
<223> Description of Artificial Sequence:
      Oligonucleotide for Identifying HPV CP8034

<400> 564
gctacatctg ctgctgcaga                                                20


<210> 565
<211> 22
<212> DNA
<213> Artificial Sequence

<220>
<223> Description of Artificial Sequence:
      Oligonucleotide for Identifying HPV CP8034

<400> 565
acatctgctg ctgcagaata ca                                             22


<210> 566
<211> 20
<212> DNA
<213> Artificial Sequence

<220>
<223> Description of Artificial Sequence:
      Oligonucleotide for Identifying HPV CP8034

<400> 566

tgctgcagaa tacaaggcct                                                    20

<210> 567
<211> 20
<212> DNA
<213> Artificial Sequence

<220>
<223> Description of Artificial Sequence:
      Oligonucleotide for Identifying HPV CP8034

<400> 567
gctgcagaat acaaggcctc                                                    20

<210> 568
<211> 20
<212> DNA
<213> Artificial Sequence

<220>
<223> Description of Artificial Sequence:
      Oligonucleotide for Identifying HPV CP8034

<400> 568
cagaatacaa ggcctctaac                                                    20

<210> 569
<211> 23
<212> DNA
<213> Artificial Sequence

<220>
<223> Description of Artificial Sequence:
      Oligonucleotide for Identifying HPV CP8034

<400> 569
taaaatacag ttaacaccag aaa                                                23

<210> 570
<211> 19
<212> DNA
<213> Artificial Sequence

<220>
<223> Description of Artificial Sequence:
      Oligonucleotide for Identifying HPV CP8034

<400> 570
caaggcactg ttggatgat                                                     19

<210> 571
<211> 26
<212> DNA
<213> Artificial Sequence

<220>
<223> Description of Artificial Sequence:
      Oligonucleotide for Identifying HPV CP8034

<400> 571
tgtgttgcca cctccttcca ccagtt                                          26


<210> 572
<211> 22
<212> DNA
<213> Artificial Sequence

<220>
<223> Description of Artificial Sequence:
      Oligonucleotide for Identifying HPV CP8034

<400> 572
atatcgcttt ttacagtctc gg                                              22


<210> 573
<211> 22
<212> DNA
<213> Artificial Sequence

<220>
<223> Description of Artificial Sequence:
      Oligonucleotide for Identifying HPV CP8034

<400> 573
gggtgctgct gcccctgcgc cc                                              22


<210> 574
<211> 16
<212> DNA
<213> Artificial Sequence

<220>
<223> Description of Artificial Sequence:
      Oligonucleotide for Identifying HPV CP8034

<400> 574
ttatgccgac atgtca                                                     16


<210> 575
<211> 20
<212> DNA
<213> Artificial Sequence

<220>
<223> Description of Artificial Sequence:
      Oligonucleotide for Identifying HPV L1AE5

<400> 575
atctactgca actactaatc                                                 20


<210> 576
<211> 15
<212> DNA
<213> Artificial Sequence

<220>

<223> Description of Artificial Sequence:
      Oligonucleotide for Identifying HPV L1AE5

<400> 576
ctgcaactac taatc                                                    15


<210> 577
<211> 20
<212> DNA
<213> Artificial Sequence

<220>
<223> Description of Artificial Sequence:
      Oligonucleotide for Identifying HPV L1AE5

<400> 577
ctgcaactac taatccagtt                                               20


<210> 578
<211> 22
<212> DNA
<213> Artificial Sequence

<220>
<223> Description of Artificial Sequence:
      Oligonucleotide for Identifying HPV L1AE5

<400> 578
actactaatc cagttccatc ta                                            22


<210> 579
<211> 20
<212> DNA
<213> Artificial Sequence

<220>
<223> Description of Artificial Sequence:
      Oligonucleotide for Identifying HPV L1AE5

<400> 579
ctaatccagt tccatctata                                               20


<210> 580
<211> 20
<212> DNA
<213> Artificial Sequence

<220>
<223> Description of Artificial Sequence:
      Oligonucleotide for Identifying HPV L1AE5

<400> 580
ctatatatga accttctaaa                                               20


<210> 581
<211> 23
<212> DNA
<213> Artificial Sequence

<220>
<223> Description of Artificial Sequence:
      Oligonucleotide for Identifying HPV L1AE5

<400> 581
taaaattaca cttactactg atg                                              23


<210> 582
<211> 19
<212> DNA
<213> Artificial Sequence

<220>
<223> Description of Artificial Sequence:
      Oligonucleotide for Identifying HPV L1AE5

<400> 582
tcctactatt ttagatagt                                                   19


<210> 583
<211> 26
<212> DNA
<213> Artificial Sequence

<220>
<223> Description of Artificial Sequence:
      Oligonucleotide for Identifying HPV L1AE5

<400> 583
tgttagtcct cccccatctg ctagct                                           26


<210> 584
<211> 22
<212> DNA
<213> Artificial Sequence

<220>
<223> Description of Artificial Sequence:
      Oligonucleotide for Identifying HPV L1AE5

<400> 584
atataggttt ttacagtcat ct                                               22


<210> 585
<211> 19
<212> DNA
<213> Artificial Sequence

<220>
<223> Description of Artificial Sequence:
      Oligonucleotide for Identifying HPV L1AE5

<400> 585
ggatgtggtt gttccacaa                                                   19


<210> 586
<211> 20

<212> DNA
<213> Artificial Sequence

<220>
<223> Description of Artificial Sequence:
      Oligonucleotide for Identifying HPV MM4

<400> 586
ctgctgttac tcaatctgtt                                                  20


<210> 587
<211> 15
<212> DNA
<213> Artificial Sequence

<220>
<223> Description of Artificial Sequence:
      Oligonucleotide for Identifying HPV MM4

<400> 587
tgctgttact caatc                                                       15


<210> 588
<211> 20
<212> DNA
<213> Artificial Sequence

<220>
<223> Description of Artificial Sequence:
      Oligonucleotide for Identifying HPV MM4

<400> 588
gttactcaat ctgttgcaca                                                  20


<210> 589
<211> 20
<212> DNA
<213> Artificial Sequence

<220>
<223> Description of Artificial Sequence:
      Oligonucleotide for Identifying HPV MM4

<400> 589
tgcacaaaca tttactccag                                                  20


<210> 590
<211> 22
<212> DNA
<213> Artificial Sequence

<220>
<223> Description of Artificial Sequence:
      Oligonucleotide for Identifying HPV MM4

<400> 590
ttactcaatc tgttgcacaa ac                                               22

<210> 591
<211> 20
<212> DNA
<213> Artificial Sequence

<220>
<223> Description of Artificial Sequence:
      Oligonucleotide for Identifying HPV MM4

<400> 591
aaacatttac tccagcaaac                                                    20


<210> 592
<211> 23
<212> DNA
<213> Artificial Sequence

<220>
<223> Description of Artificial Sequence:
      Oligonucleotide for Identifying HPV MM4

<400> 592
taaaatcact ttaactactg aaa                                                23


<210> 593
<211> 19
<212> DNA
<213> Artificial Sequence

<220>
<223> Description of Artificial Sequence:
      Oligonucleotide for Identifying HPV MM4

<400> 593
ttctacaatt ttagaacag                                                     19


<210> 594
<211> 26
<212> DNA
<213> Artificial Sequence

<220>
<223> Description of Artificial Sequence:
      Oligonucleotide for Identifying HPV MM4

<400> 594
   attaaccttg cccccctcag ctagtt                                              26


<210> 595
<211> 22
<212> DNA
<213> Artificial Sequence

<220>
<223> Description of Artificial Sequence:
      Oligonucleotide for Identifying HPV MM4

<400> 595
ctatcgattt gtaaaaaatg ca                                                 22

<210> 596
<211> 19
<212> DNA
<213> Artificial Sequence

<220>
<223> Description of Artificial Sequence:
      Oligonucleotide for Identifying HPV MM4

<400> 596
ggacagtcct ccacaggct                                                    19


<210> 597
<211> 15
<212> DNA
<213> Artificial Sequence

<220>
<223> Description of Artificial Sequence:
      Oligonucleotide for Identifying HPV MM7

<400> 597
tgctgctaca caggc                                                        15


<210> 598
<211> 20
<212> DNA
<213> Artificial Sequence

<220>
<223> Description of Artificial Sequence:
      Oligonucleotide for Identifying HPV MM7

<400> 598
gctgctacac aggctaatga                                                   20


<210> 599
<211> 20
<212> DNA
<213> Artificial Sequence

<220>
<223> Description of Artificial Sequence:
      Oligonucleotide for Identifying HPV MM7

<400> 599
tgctacacag gctaatgaat                                                   20


<210> 600
<211> 22
<212> DNA
<213> Artificial Sequence

<220>
<223> Description of Artificial Sequence:
      Oligonucleotide for Identifying HPV MM7

<400> 600
ctacacaggc taatgaatac ac                                              22


<210> 601
<211> 20
<212> DNA
<213> Artificial Sequence

<220>
<223> Description of Artificial Sequence:
      Oligonucleotide for Identifying HPV MM7

<400> 601
atgaatacac agcctctaac                                                 20


<210> 602
<211> 23
<212> DNA
<213> Artificial Sequence

<220>
<223> Description of Artificial Sequence:
      Oligonucleotide for Identifying HPV MM7

<400> 602
caaaatacat cttacccctg aaa                                             23


<210> 603
<211> 19
<212> DNA
<213> Artificial Sequence

<220>
<223> Description of Artificial Sequence:
      Oligonucleotide for Identifying HPV MM7

<400> 603
tgaacattta ttggatgag                                                  19


<210> 604
<211> 26
<212> DNA
<213> Artificial Sequence

<220>
<223> Description of Artificial Sequence:
      Oligonucleotide for Identifying HPV MM7

<400> 604
cgtgttacca cctccttcca ccagcc                                          26


<210> 605
<211> 22
<212> DNA
<213> Artificial Sequence

<220>
<223> Description of Artificial Sequence:

Oligonucleotide for Identifying HPV MM7

<400> 605
ctatcgctat ctgcagtccc gt                                                22


<210> 606
<211> 22
<212> DNA
<213> Artificial Sequence

<220>
<223> Description of Artificial Sequence:
      Oligonucleotide for Identifying HPV MM7

<400> 606
gggtccttcc gcccctgccc ct                                                22


<210> 607
<211> 16
<212> DNA
<213> Artificial Sequence

<220>
<223> Description of Artificial Sequence:
      Oligonucleotide for Identifying HPV MM7

<400> 607
ttatgatggc cttgta                                                       16


<210> 608
<211> 15
<212> DNA
<213> Artificial Sequence

<220>
<223> Description of Artificial Sequence:
      Oligonucleotide for Identifying HPV MM8

<400> 608
tgctaccaac accga                                                        15


<210> 609
<211> 20
<212> DNA
<213> Artificial Sequence

<220>
<223> Description of Artificial Sequence:
      Oligonucleotide for Identifying HPV MM8

<400> 609
ctaccaacac cgaatcagaa                                                   20


<210> 610
<211> 22
<212> DNA
<213> Artificial Sequence

<220>
<223> Description of Artificial Sequence:
      Oligonucleotide for Identifying HPV MM8

<400> 610
ccaacaccga atcagaatat aa                                                22

<210> 611
<211> 20
<212> DNA
<213> Artificial Sequence

<220>
<223> Description of Artificial Sequence:
      Oligonucleotide for Identifying HPV MM8

<400> 611
cagaatataa acctaccaat                                                   20

<210> 612
<211> 23
<212> DNA
<213> Artificial Sequence

<220>
<223> Description of Artificial Sequence:
      Oligonucleotide for Identifying HPV MM8

<400> 612
taaggtccgt ctgactccag agg                                               23

<210> 613
<211> 19
<212> DNA
<213> Artificial Sequence

<220>
<223> Description of Artificial Sequence:
      Oligonucleotide for Identifying HPV MM8

<400> 613
tgactcctta ttagatgag                                                    19

<210> 614
<211> 26
<212> DNA
<213> Artificial Sequence

<220>
<223> Description of Artificial Sequence:
      Oligonucleotide for Identifying HPV MM8

<400> 614
tgttgtgccc cctccctcca caagtt                                            26

<210> 615
<211> 22
<212> DNA

<213> Artificial Sequence

<220>
<223> Description of Artificial Sequence:
      Oligonucleotide for Identifying HPV MM8

<400> 615
ctataggtac ttgcagtctc gc                                                22


<210> 616
<211> 22
<212> DNA
<213> Artificial Sequence

<220>
<223> Description of Artificial Sequence:
      Oligonucleotide for Identifying HPV MM8

<400> 616
gggggccgcc gccgccaagc ct                                                22


<210> 617
<211> 16
<212> DNA
<213> Artificial Sequence

<220>
<223> Description of Artificial Sequence:
      Oligonucleotide for Identifying HPV MM8

<400> 617
ttatgctggc atgtcc                                                       16


<210> 618
<211> 22
<212> DNA
<213> Artificial Sequence

<220>
<223> Description of Artificial Sequence:
      Oligonucleotide for Identifying HPV 42

<400> 618
tatatgttgg ggaaatcagc ta                                                22


<210> 619
<211> 20
<212> DNA
<213> Artificial Sequence

<220>
<223> Description of Artificial Sequence:
      Oligonucleotide for Identifying HPV 42

<400> 619
cactgcaaca tctggtgata                                                   20


<210> 620

<211> 30
<212> DNA
<213> Artificial Sequence

<220>
<223> Description of Artificial Sequence:
      Oligonucleotide for Identifying HPV 42

<400> 620
gcaacatctg gtgatacata tacagctgct                                        30


<210> 621
<211> 23
<212> DNA
<213> Artificial Sequence

<220>
<223> Description of Artificial Sequence:
      Oligonucleotide for Identifying HPV 42

<400> 621
cattaactgt tgaagttatg tca                                               23


<210> 622
<211> 26
<212> DNA
<213> Artificial Sequence

<220>
<223> Description of Artificial Sequence:
      Oligonucleotide for Identifying HPV 42

<400> 622
cctaacatat tagaggagtg gaatgt                                            26


<210> 623
<211> 20
<212> DNA
<213> Artificial Sequence

<220>
<223> Description of Artificial Sequence:
      Oligonucleotide for Identifying HPV 42

<400> 623
caccaccacc ttcaggaact                                                   20


<210> 624
<211> 24
<212> DNA
<213> Artificial Sequence

<220>
<223> Description of Artificial Sequence:
      Oligonucleotide for Identifying HPV 42

<400> 624
gttataggta tgtacaatca gaag                                              24

<210> 625
<211> 30
<212> DNA
<213> Artificial Sequence

<220>
<223> Description of Artificial Sequence:
      Oligonucleotide for Identifying HPV 42

<400> 625
gctaaggtaa caacgccaga aaaaaaggat                                        30


<210> 626
<211> 24
<212> DNA
<213> Artificial Sequence

<220>
<223> Description of Artificial Sequence:
      Oligonucleotide for Identifying HPV 42

<400> 626
cagacttttg gttttgggag gtaa                                              24


<210> 627
<211> 21
<212> DNA
<213> Artificial Sequence

<220>
<223> Description of Artificial Sequence:
      Oligonucleotide for Identifying HPV 42

<400> 627
gaaaagtttt ctactgattt a                                                 21


<210> 628
<211> 25
<212> DNA
<213> Artificial Sequence

<220>
<223> Description of Artificial Sequence:
      Oligonucleotide for Identifying HPV 42

<400> 628
gtcagtgatt cacagcacag catag                                             25


<210> 629
<211> 21
<212> DNA
<213> Artificial Sequence

<220>
<223> Description of Artificial Sequence:
      Oligonucleotide for Identifying HPV 42

<400> 629

gtcctaggct tggcggttta a                                              21

<210> 630
<211> 30
<212> DNA
<213> Artificial Sequence

<220>
<223> Description of Artificial Sequence:
      Oligonucleotide for Identifying HPV 42

<400> 630
cccaaggggc caaacgacga ttattccagt                                       30

<210> 631
<211> 26
<212> DNA
<213> Artificial Sequence

<220>
<223> Description of Artificial Sequence:
      Oligonucleotide for Identifying HPV 42

<400> 631
cagcagacac aggtagaaca cggaca                                           26

<210> 632
<211> 22
<212> DNA
<213> Artificial Sequence

<220>
<223> Description of Artificial Sequence:
      Oligonucleotide for Identifying HPV 42

<400> 632
gcagtgggta gtgaacttgg gg                                               22

<210> 633
<211> 26
<212> DNA
<213> Artificial Sequence

<220>
<223> Description of Artificial Sequence:
      Oligonucleotide for Identifying HPV 42

<400> 633
gaagaaggta gtactacaag tacgcc                                           26

<210> 634
<211> 25
<212> DNA
<213> Artificial Sequence

<220>
<223> Description of Artificial Sequence:
      Oligonucleotide for Identifying HPV 42

<400> 634
ggtagaatta cttaagtgta agaac                                          25


<210> 635
<211> 22
<212> DNA
<213> Artificial Sequence

<220>
<223> Description of Artificial Sequence:
      Oligonucleotide for Identifying HPV 42

<400> 635
gttaggtaag tttaaagaat tg                                             22


<210> 636
<211> 22
<212> DNA
<213> Artificial Sequence

<220>
<223> Description of Artificial Sequence:
      Oligonucleotide for Identifying HPV 42

<400> 636
gtcatttggc gatttagtaa ga                                             22


<210> 637
<211> 22
<212> DNA
<213> Artificial Sequence

<220>
<223> Description of Artificial Sequence:
      Oligonucleotide for Identifying HPV 43

<400> 637
catttgtttt gggaatcagt tg                                             22


<210> 638
<211> 20
<212> DNA
<213> Artificial Sequence

<220>
<223> Description of Artificial Sequence:
      Oligonucleotide for Identifying HPV 43

<400> 638
tgaccctact gtgcccagta                                                20


<210> 639
<211> 30
<212> DNA
<213> Artificial Sequence

<220>

<223> Description of Artificial Sequence:
      Oligonucleotide for Identifying HPV 43

<400> 639
actgtgccca gtacatatga caatgcaaag                                        30


<210> 640
<211> 23
<212> DNA
<213> Artificial Sequence

<220>
<223> Description of Artificial Sequence:
      Oligonucleotide for Identifying HPV 43

<400> 640
gtttatattt caattatgca taa                                               23


<210> 641
<211> 21
<212> DNA
<213> Artificial Sequence

<220>
<223> Description of Artificial Sequence:
      Oligonucleotide for Identifying HPV 43

<400> 641
ccagaggtta tgacatatat t                                                 21


<210> 642
<211> 23
<212> DNA
<213> Artificial Sequence

<220>
<223> Description of Artificial Sequence:
      Oligonucleotide for Identifying HPV 43

<400> 642
cccacattat tagaggactg gaa                                               23


<210> 643
<211> 21
<212> DNA
<213> Artificial Sequence

<220>
<223> Description of Artificial Sequence:
      Oligonucleotide for Identifying HPV 43

<400> 643
ccacctgcct ctgcttcttt g                                                 21


<210> 644
<211> 25
<212> DNA
<213> Artificial Sequence

<220>
<223> Description of Artificial Sequence:
      Oligonucleotide for Identifying HPV 43

<400> 644
cgctttttgt ctaacaaggc cattg                                          25


<210> 645
<211> 23
<212> DNA
<213> Artificial Sequence

<220>
<223> Description of Artificial Sequence:
      Oligonucleotide for Identifying HPV 43

<400> 645
ccaaaggaac gggaggatcc cta                                            23


<210> 646
<211> 25
<212> DNA
<213> Artificial Sequence

<220>
<223> Description of Artificial Sequence:
      Oligonucleotide for Identifying HPV 43

<400> 646
cttacagaaa agttttctgc acaac                                          25


<210> 647
<211> 21
<212> DNA
<213> Artificial Sequence

<220>
<223> Description of Artificial Sequence:
      Oligonucleotide Gap21-5

<400> 647
ccaccaactg cttagcaccc c                                              21


<210> 648
<211> 21
<212> DNA
<213> Artificial Sequence

<220>
<223> Description of Artificial Sequence:
      Oligonucleotide Gap21-3

<400> 648
tgcagcgtac tccccacatc a                                              21


<210> 649
<211> 20

<212> DNA
<213> Artificial Sequence

<220>
<223> Description of Artificial Sequence:GAP-DH sequence

<400> 649
gcccagactg tgggtggcag                                                    20